# EUROPEAN PATENT APPLICATION

(11) **EP 2 415 481 A2**
(43) Date of publication of application: **08.02.2012**
(21) Application number: 11185309.9
(22) Date of filing: 18.02.2005
(51) Int. Cl.: A61K 39/39, C12N 15/11

(54) **Immunostimulatory viral RNA oligonucleotides**

(30) Priority: 19.02.2004 US 545988 P
(62) Divisional of application: 05756145.8
(71) Applicant: Coley Pharmaceutical Group, Inc., New York, N.Y. 10017-5755 (US); Coley Pharmaceutical GmbH, 40225 Düsseldorf (DE)
(72) Inventor: Lipford, Grayson, B., Watertown, MA 02472 (US); Forsbach, Alexandra, 40764 Ratingen (DE)
(74) Representative: England, Peter Michael

(57) **Abstract**

An immunostimulatory composition comprising a 4-mer RNA motif provided as 5'-N₁-U-U-G-U-N₂-3', wherein N₁ and N₂ independently are RNA sequences 0-10 nucleotides long. The 4-mer motif is sufficient to confer new and altered immunostimulatory properties in new and existing oligonucleotides. Also provided are methods of stimulating an immune response comprising administering said immunostimulatory 4-mer motif composition.

## Description

### FIELD OF THE INVENTION

The invention relates to immunostimulatory nucleic acid compositions and methods of use therefor. More specifically, the invention relates to immunostimulatory viral RNA sequences, variants and conjugates thereof, and their use.

### BACKGROUND OF THE INVENTION

The immune response is conceptually divided into innate immunity and adaptive immunity. Innate immunity is believed to involve recognition of pathogen-associated molecular patterns (PAMPs) shared in common by certain classes of molecules expressed by infectious microorganisms or foreign macromolecules. PAMPs are believed to be recognized by pattern recognition receptors (PRRs) on certain immune cells. It has recently been reported that Toll-like receptors (TLRs) represent an important class of PRRs.

TLRs are a family of highly conserved polypeptides that play a critical role in innate immunity in mammals. Currently twelve family members, designated TLR1 - TLR12, have been identified. The various TLRs are structurally characterized by the presence of an extracellular domain having leucine-rich repeats, a transmembrane domain, and a ctyoplasmic signaling domain. The cytoplasmic domains of the various TLRs are characterized by a Toll-interleukin 1 receptor (TIR) domain. Medzhitov R et al. (1998) Mol Cell 2:253-8. Recognition of microbial invasion by TLRs triggers activation of a signaling cascade that is evolutionarily conserved in *Drosophila* and mammals. The TIR domain-containing adapter protein MyD88 has been reported to associate with TLRs and to recruit IL-1 receptor-associated kinase (IRAK) and tumor necrosis factor (TNF) receptor-associated factor 6 (TRAF6) to the TLRs: The TIR- and/or MyD88-dependent signaling pathway is believed to lead to activation of NF-kB transcription factors and c-Jun NH₂ terminal kinase (Jnk) mitogen-activated protein kinases (MAPKs), critical steps in immune activation and production of inflammatory cytokines. For a review, see Aderem A et al. (2000) Nature 406:782-87.

Ligands for a number of TLRs have been reported. Ligands for TLR2 include peptidoglycan and lipopeptides. Yoshimura A et al. (1999) J Immunol 163:1-5; Yoshimura A et al. (1999) J Immunol 163:1-5; Aliprantis AO et al. (1999) Science 285:736-9. Lipopolysaccharide (LPS) is a ligand for TLR4. Poltorak A et al. (1998) Science 282:2085-8; Hoshino K et al. (1999) J Immunol 162:3749-52*.* Bacterial flagellin is a ligand for TLR5. Hayashi F et al. (2001) Nature 410:1099-1103. Peptidoglycan has been reported to be a ligand not only for TLR2 but also for TLR6. Ozinsky A et al. (2000) Proc Natl Acad Sci USA 97:13766-71; Takeuchi O et al. (2001) Int Immunol 13:933-40.

In addition to the foregoing, natural ligands for certain TLRs have been reported to include certain types of nucleic acid molecules. Bacterial DNA (CpG DNA) has been reported to be a TLR9 ligand. Hemmi H et al. (2000) Nature 408:740-5; Bauer S et al. (2001) Proc Natl Acad Sci USA 98, 9237-42. More recently, it was reported that viral-derived double-stranded RNA (dsRNA) and poly I:C, a synthetic analog of dsRNA, are ligands of TLR3. Alexopoulou L et al. (2001) Nature 413:732-8.

Until recently, natural ligands for TLR7 and TLR8 were not known. It had previously been reported that certain low molecular weight synthetic compounds, the imidazoquinolones imiquimod (R-837) and resiquimod (R-848), are ligands of TLR7 and TLR8. Hemmi H et al. (2002) Nat Immunol 3:196-200; Jurk M et al. (2002) Nat Immunol 3:499*.* More recently, Lipford et al. discovered that certain G,U-containing oligoribonucleotides are immunostimulatory and act through TLR7 and TLR8. WO 03/086280. The immunostimulatory G,U-containing oligoribonucleotides described by Lipford et al. were believed to be derivable from RNA sources including ribosomal RNA, transfer RNA, messenger RNA, and viral RNA.

Certain of the immunostimulatory RNAs described by Lipford et al. include those with base sequences that include 5'-RURGY-3', wherein R represents purine, U represents uracil, G represents guanine, and Y represents pyrimidine. Certain of the immunostimulatory RNAs described by Lipford et al. include those with base sequences containing or provided by GUAGUGU, GUUGB, GUGUG, GUGUUUAC, GUAGGCAC, CUAGGCAC, CUCGGCAC, or GUUGUGGUUGUGGUUGUG (SEQ ID NO:1), wherein A represents adenine, C represents cytosine, and B represents U, G, or C. In some embodiments the immunostimulatory RNAs described by Lipford et al. are combined with the cationic lipid N-[1-(2, 3 dioleoyloxy)-propyl]-N,N,N-trimethylammonium methylsulfate (DOTAP).

### SUMMARY OF THE INVENTION

The invention is based in part on the surprising discovery by the inventors that certain short RNA sequences, which are relatively highly conserved and can be found at or in proximity to the 3' termini of single-stranded minus-sense RNA virus genomes, are immunostimulatory. These sequences are believed to include certain contact points which permit them to stimulate signaling via certain Toll-like receptors (TLRs) expressed on immune cells. The involved TLRs are believed to include at least one of TLR8 and TLR7. Although TLR3 may also act as a receptor for these nucleic acid molecules, an important feature of the immunostimulatory nucleic acids of the invention is the base sequence. Thus while sequence-nonspecific double-stranded RNA has been reported to be a ligand for TLR3, the instant invention discloses the immunostimulatory nature of sequence-specific single-stranded RNAs and related compositions. The immunostimulatory compositions of the invention have been found to act as strong inducers of a number of cytokines including type 1 interferons, interleukin-12 (IL-12), interleukin-6 (IL-6), and tumor necrosis factor alpha (TNF-α) in human peripheral blood mononuclear cells (PBMC) and mouse leukemic monocyte-macrophage (RAW 264) cells.

The invention is also based in part on the surprising discovery by the inventors of an immunostimulatory 4-mer RNA motif provided by the sequence 5'-C/U-U-G/U-U-3'. In addtion to being found in many of the 3' termini of single-stranded minus-sense RNA virus genomes just described, it has been discovered that this motif can be grafted into another oligonucleotide to confer new immunostimulatory properties upon the oligonucleotide. For example, the motif is sufficient to convert a non-imunostimulatory oligonucleotide into one that is capable of inducing a number of cytokines and other manifestations of immune activation. Further, the motif can be placed into a DNA context or into an RNA context.

The invention in general provides immunostimulatory compositions that are related to certain highly conserved nucleic acid sequences present in the 3' ends of the genomic RNA of single-stranded minus-sense RNA viruses, as well as methods for their use. The compositions are useful for stimulating an immune response in vitro or in vivo and may be used alone or in combination with an antigen or other agent for purposes of vaccination; treating certain conditions including allergy, asthma, infection, and cancer; or screening for other immunomodulatory compositions.

In one aspect the invention provides an immunostimulatory composition, including an isolated nucleic acid molecule 10 to 30 nucleotides long including a sequence provided by a 3' end of a single-stranded minus-sense RNA virus genome, wherein the nucleic acid molecule has a stabilized backbone. Where the single-stranded minus-sense RNA virus genome is a segmented genome, the sequence provided by a 3' end of the genome can be a sequence provided by a 3' end of any segment of the segmented genome. As described in greater detail below, a nucleic acid having a stabilized backbone refers to a nucleic acid molecule that is relatively stable against nuclease degradation compared to a nucleic acid having a phosphodiester backbone. In one embodiment the nucleic acid molecule is 10 to 20 nucleotides long. In one embodiment the nucleic acid molecule is 10 nucleotides long.

In one embodiment the nucleic acid molecule includes a sequence motif 5'-C/U-U-G/U-U-3', wherein U is uracil (U) oxyribonucleoside, C/U is cytosine (C) oxyribonucleoside or uracil (U) oxyribonucleoside, and G/U is guanine (G) oxyribonucleoside or uracil (U) oxyribonucleoside. In various specific embodiments according to this and other aspects of the invention the sequence motif is 5'-CUGU-3', 5'-UUGU-3', 5'-CUUU-3', or 5'-UUUU-3'. In one embodiment according to this and other aspects of the invention the nucleic acid molecule excludes the sequence 5'-GUUGU-3'.

In one embodiment the stabilized backbone includes at least one phosphorothioate internucleoside linkage. For example, in one embodiment the stabilized backbone is a phosphorothioate backbone, i.e., includes only phosphorothioate internucleoside linkages. In certain embodiments the stabilized backbone includes at least one pyrophosphate internucleoside linkage or the stabilized backbone is a pyrophosphate backbone, i.e., includes only pyrophosphate internucleoside linkages.

While in one embodiment the isolated nucleic acid molecule is RNA, in one embodiment the nucleic acid molecule includes at least one deoxyribonucleotide.

It is believed that the immunostimulatory compositions of the invention signal via at least one Toll-like receptor (TLR). In one embodiment the nucleic acid molecule is a TLR agonist. In one embodiment the nucleic acid molecule is an agonist of TLR8. In one embodiment the nucleic acid molecule is an agonist of TLR7. In one embodiment the nucleic acid molecule is an agonist of TLR3.

In certain embodiments the single-stranded minus-sense RNA virus belongs to the order *Mononegavirales* and can have a segmented or a non-segmented genome. In one embodiment the single-stranded minus-sense RNA virus is an orthomyxovirus. In one embodiment the single-stranded minus-sense RNA virus is a paramyxovirus. In one embodiment the single-stranded minus-sense RNA virus is a rhabdovirus. In yet another embodiment the single-stranded minus-sense RNA virus is a filovirus. In one embodiment the single-stranded minus-sense RNA virus is a bornavirus. In one embodiment the single-stranded minus-sense RNA virus is an influenza A virus. In one embodiment the single-stranded minus-sense RNA virus is an influenza B virus.

Immunostimulatory compositions of the invention optionally can be associated with another agent that may enhance or otherwise modify the immunostimulatory function of the nucleic acid. In one embodiment the nucleic acid molecule is associated with a cationic lipid.

Alternatively or in addition, immunostimulatory compositions of the invention optionally can include an antigen.

In one aspect the invention provides an immunostimulatory composition comprising an isolated nucleic acid molecule 4 to 30 nucleotides long comprising a sequence provided by a 3' end of a single-stranded minus-sense RNA virus genome, wherein the nucleic acid molecule has a stabilized backbone, and an antigen.

In one aspect the invention provides an immunostimulatory composition comprising an isolated oligoribonucleotide (ORN) 7-40 nucleotides long comprising

5'-N1-C/U-U-G/U-U-N₂-3'

wherein U is uracil (U) oxyribonucleoside, C/U is cytosine (C) oxyribonucleoside or uracil (U) oxyribonucleoside, G/U is guanine (G) oxyribonucleoside or uracil (U) oxyribonucleoside, N₁ and N₂ independently are RNA sequences 0-10 nucleotides long, and the oligoribonucleotide has a stabilized backbone.

In one aspect the invention provides an immunostimulatory composition comprising a chimeric DNA:RNA oligonucleotide 7-40 nucleotides long comprising

5'-dX₁-N₁-C/U-U-G/U-U-N₂-dX₂-3'

wherein U is uracil (U) oxyribonucleoside, C/U is cytosine (C) oxyribonucleoside or uracil (U) oxyribonucleoside, G/U is guanine (G) oxyribonucleoside or uracil (U) oxyribonucleoside, dX₁ and dX₂ independently are DNA sequences 0-6 nucleotides long wherein at least one of dX₁ and dX₂ is at least 1 nucleotide long, and N₁ and N₂ independently are RNA sequences 0-10 nucleotides long. In one embodiment according to this aspect of the invention N₁ and N₂ are both 0 nucleotides long. Also according to this aspect of the invention, in one embodiment dX₁ is 0 nucleotides long, and in one embodiment dX₂ is 0 nucleotides long. In various embodiments according to this aspect of the invention dX₁, dX₂, or both dX₁ and dX₂ can include a CpG motif. The CpG motif includes a DNA sequence including a central 5'-cytosine-guanosine-3' (CG) dinucleotide, wherein the C of the CG dinucleotide is unmethylated, and wherein the CG dinucleotide is flanked by a 5' dinucleotide preferably selected from guanosine-thymidine (GT), guanosine-guanosine (GG), guanosine-adenosine (GA), adenosine-thymidine (AT), and adenosine-adenosine (AA), and by a 3' dinucleotide preferably selected from thymidine-thymidine (TT) and cytosine-thymidine (CT).

In another aspect the invention provides a method for altering an immunostimulatory profile of a reference oligonucleotide having a reference immunostimulatory profile. The method according to this aspect of the invention includes the step of altering a reference oligonucleotide 3-40 nucleotides long to include an RNA motif 5'-C/U-U-G/U-U-3', wherein U is uracil (U) oxyribonucleoside, C/U is cytosine (C) oxyribonucleoside or uracil (U) oxyribonucleoside, G/U is guanine (G) oxyribonucleoside or uracil (U) oxyribonucleoside, wherein the the reference oligonucleotide does not include the immunostimulatory RNA motif 5'-C/U-U-G/U-U-3', wherein the altering results in an altered oligonucleotide having an altered immunostimulatory profile distinct from the reference immunostimulatory profile. An immunostimulatory profile of an oligonucleotide in one embodiment refers to the capacity of the oligonucleotide to stimulate signaling by one or more TLRs selected from TLR9, TLR8, TLR7, and TLR3. In one embodiment an immunostimulatory profile of an oligonucleotide refers to the capacity of the oligonucleotide to stimulate secretion of one or more cytokines, chemokines, or classes of immunoglobulin associated with an immune response. In one embodiment an immunostimulatory profile of an oligonucleotide refers to the capacity of the oligonucleotide to stimulate expression of one or more cell surface markers, including co-stimulatory molecules associated with immune activation, on a cell or population of cells of the immune system.

In one aspect the invention provides a method for altering an immunostimulatory profile of a CpG oligodeoxynucleotide (CpG ODN) having a reference immunostimulatory profile. The method according to this aspect of the invention includes the step of replacing at least one dC of the CpG ODN, at least one dT of the CpG ODN, or at least one dC of the CpG ODN and at least one dT of the CpG ODN with U, wherein U is uracil oxyribonucleoside, and wherein the replacing results in an altered oligonucleotide having an altered immunostimulatory profile distinct from the reference immunostimulatory profile. An altered oligonucleotide according to this aspect of the invention will always include at least one U. In various embodiments according to this aspect of the invention the altered oligonucleotide can be partly or completely RNA.

The invention in one aspect provides composition comprising an isolated immunostimulatory oligoribonucleotide, the sequence of which is provided as 5'-UUGUUGUUUGUUUUGUUGUUUUGUUGUU-3' (SEQ ID NO:286).

The invention in one aspect provides composition comprising an isolated immunostimulatory oligoribonucleotide, the sequence of which is provided as 5'-TUGTUGTTTTGTUGTTTTGTUGTT-3' (SEQ ID NO:287), wherein each T represents the ribonucleotide 5-methyluridine.

In another aspect the invention provides a method for stimulating an immune response. The method according to this aspect of the invention includes the step of contacting a cell of the immune system with an effective amount of a composition of the invention to stimulate an immune response. In one embodiment the immune response is a Th1-like immune response. In one embodiment the method involves contacting a cell of the immune system with an effective amount of a composition of the invention to stimulate expression of a type 1 interferon, e.g., an interferon alpha (IFN-α) or interferon beta (IFN-β). In one embodiment the method involves contacting a cell of the immune system with an effective amount of a composition of the invention to stimulate expression of IL-12.

In another aspect the invention provides a method for stimulating TLR signaling. The method according to this aspect of the invention includes the step of contacting a cell expressing a TLR with an effective amount of a composition of the invention to stimulate signaling by the TLR. In one embodiment the TLR is TLR9. In one embodiment the TLR is TLR8. In one embodiment the TLR is TLR7. In one embodiment the TLR is TLR3.

The invention also provides, in one aspect, a method for stimulating an immune response in a subject. The method according to this aspect of the invention includes the step of administering to a subject an effective amount of a composition of the invention to stimulate an immune response in the subject. In one embodiment the immune response in the subject is a Th1-like immune response. In one embodiment the method includes the step of administering to the subject an effective amount of a composition of the invention to stimulate expression of a type 1 interferon in the subject. In one embodiment the method includes the step of administering to the subject an effective amount of a composition of the invention to stimulate expression of IL-12 in the subject.

According to one aspect the invention provides a method for stimulating an antigen-specific immune response in a subject. The method according to this aspect of the invention includes the step of administering to a subject an effective amount of a composition of the invention and an antigen to stimulate an antigen-specific immune response in the subject. In one embodiment the antigen is an allergen and the antigen-specific immune response is an allergen-specific immune response in the subject. In one embodiment the antigen is a viral antigen and the antigen-specific immune response is a viral antigen-specific immune response in the subject. In one embodiment the antigen is a bacterial antigen and the antigen-specific immune response is a bacterial antigen-specific immune response in the subject. In one embodiment the antigen is a cancer antigen and the antigen-specific immune response is a cancer antigen-specific immune response in the subject.

The invention in one aspect provides a method for treating an allergic condition in a subject. The method according to this aspect of the invention includes the step of administering to a subject having or at risk of developing an allergic condition an effective amount of a composition of the invention to treat the allergic condition.

The invention in one aspect provides a method for treating asthma in a subject. The method according to this aspect of the invention includes the step of administering to a subject having or at risk of developing asthma an effective amount of a composition of the invention to treat the asthma.

The invention in one aspect provides a method for treating an infection in a subject. The method according to this aspect of the invention includes the step of administering to a subject having or at risk of developing an infection an effective amount of a composition of the invention to treat the infection. In one embodiment the infection is a viral infection. In one embodiment the infection is a bacterial infection.

The invention in one aspect provides a method for treating cancer in a subject. The method according to this aspect of the invention includes the step of administering to a subject having or at risk of developing cancer an effective amount of a composition of the invention to treat the cancer.

In one aspect the invention provides a method for screening for an antagonist of a TLR. The method according to this aspect of the invention includes the steps of contacting a reference cell expressing a TLR with an effective amount of a composition of the invention, in absence of a candidate antagonist of the TLR, to measure a reference amount of signaling by the TLR; contacting a test cell expressing the TLR with an effective amount of the composition, in presence of the candidate antagonist of the TLR, to measure a test amount of signaling by the TLR; and determining the candidate antagonist of the TLR is an antagonist of the TLR when the reference amount of signaling exceeds the test amount of signaling. In one embodiment the TLR is TLR9. In one embodiment the TLR is TLR8. In one embodiment the TLR is TLR7. In one embodiment the TLR is TLR3.

### BRIEF DESCRIPTION OF THE DRAWINGS

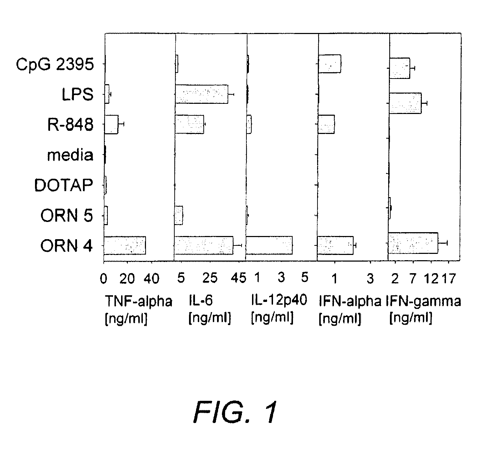

FIG. 1 is a bar graph depicting induction of various cytokines (TNF-α, IL-6, IL-12 p40, IFN-α, and IFN-y) in human PBMC following overnight stimulation with the CpG ODN 2395 (5'-TCGTCGTTTTCGGCGCGCGCCG-3', SEQ ID NO:343), lipoplysaccharide (LPS), resiquimod (R-848), media alone, cationic lipid alone (DOTAP), negative control oligoribonucleotide ORN 5 (5'-AGCGAAAGCAGGUCAAUUAU-3', SEQ ID NO:327), and viral-derived RNA oligonucleotide ORN 4 (5'-AUAAUUGACCUGCUUUCGCU-3', SEQ ID NO:5).

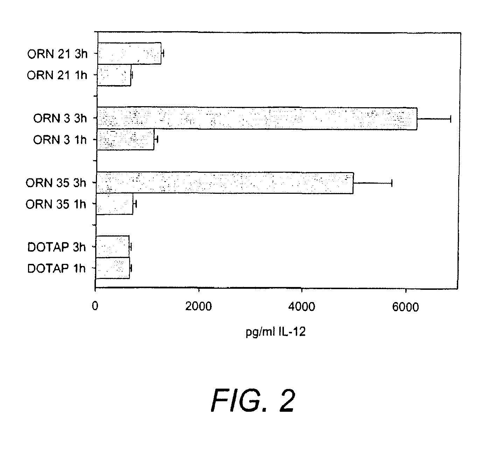

FIG.2 is a bar graph depicting induction of IL-12 in mice following injection with negative control ORN 21 (5'-GCCACCGAGCCGAAGGCACC-3', SEQ ID NO:337), viral-derived ORN 3 (5'-UGUUUUUUCUCUUGUUUGGU-3', SEQ ID NO:4), ORN 35 (5'-CCGUCUGUUGUGUGACUC-3', SEQ ID NO:344), or cationic lipid alone (DOTAP). Results are shown for samples obtained 1 hour and 3 hour following injection.

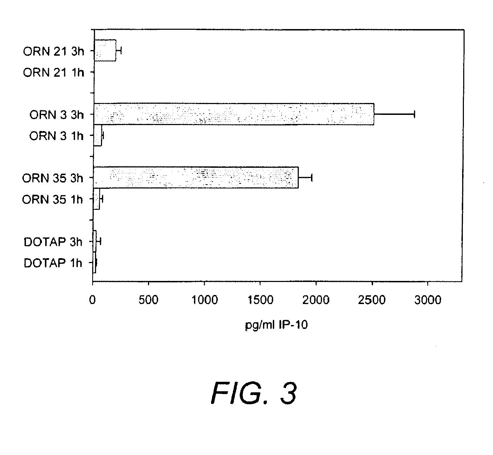

FIG.3 is a bar graph depicting induction of IP-10 in mice following injection with negative control ORN 21 (5'-GCCACCGAGCCGAAGGCACC-3', SEQ ID NO:337), viral-derived ORN 3 (5'-UGUUUUUUCUCUUGUUUGGU-3', SEQ ID NO:4), ORN 35 (5'-CCGUCUGUUGUGUGACUC-3', SEQ ID NO:344), or cationic lipid alone (DOTAP). Results are shown for samples obtained 1 hour and 3 hour following injection.

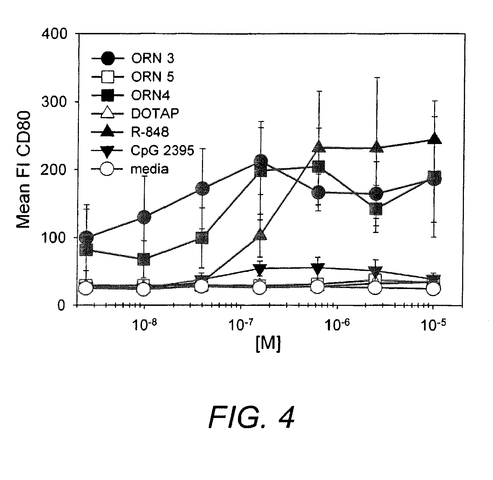

FIG. 4 is a graph depicting expression of CD80 on human CD14+ cells following overnight incubation with the indicated concentrations of viral-derived ORN 3 (5'-UGUUUUUUCUCUUGUUUGGU-3', SEQ ID NO:4), viral derived ORN 4 (5'-AUAAUUGACCUGCUUUCGCU-3', SEQ ID NO:5), negative control oligoribonucleotide ORN 5 (5'-AGCGAAAGCAGGUCAAUUAU-3', SEQ ID NO:327), DOTAP alone, media alone, R-848, CpG ODN 2395 (5'-TCGTCGTTTTCGGCGCGCGCCG-3', SEQ ID NO:343), or media alone.

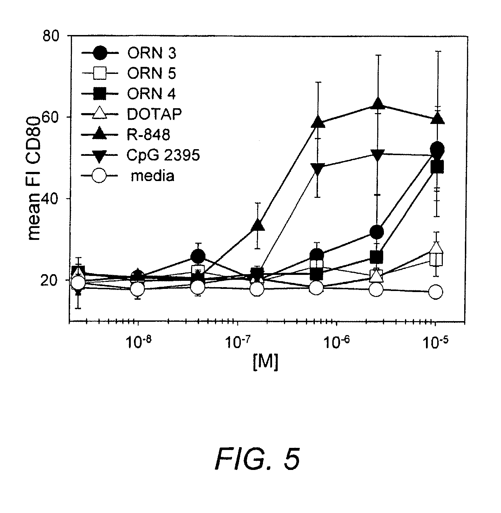

FIG. 5 is a graph depicting expression of CD80 on human CD19+ cells (B cells) following overnight incubation with the indicated concentrations of viral-derived ORN 3 (5'-UGUUUUUUCUCUUGUUUGGU-3', SEQ ID NO:4), viral derived ORN 4 (5'-AUAAUUGACCUGCUUUCGCU-3', SEQ ID NO:5), negative control oligoribonucleotide ORN 5 (5'-AGCGAAAGCAGGUCAAUUAU-3', SEQ ID NO:327), DOTAP alone, media alone, R-848, CpG ODN 2395 (5'-TCGTCGTTTTCGGCGCGCGCCG-3', SEQ ID NO:343), or media alone.

### DETAILED DESCRIPTION OF THE INVENTION

### Definitions

As used herein, the term "allergen" refers to an antigen capable of eliciting an allergic reaction or an allergic condition.

As used herein, the term "allergic condition" refers to an acquired hypersensitivity to a substance (allergen). Allergic conditions include eczema, allergic rhinitis or coryza, hay fever, bronchial asthma, urticaria (hives) and food allergies, and other atopic conditions.

As used herein, the term "antigen" refers to any molecule capable of being recognized by a T-cell antigen receptor or B-cell antigen receptor. The term broadly includes any type of molecule which is recognized by a host immune system as being foreign. Antigens generally include but are not limited to cells, cell extracts, proteins, polypeptides, peptides, polysaccharides, polysaccharide conjugates, peptide and non-peptide mimics of polysaccharides and other molecules, small molecules, lipids, glycolipids, polysaccharides, carbohydrates, viruses and viral extracts, and multicellular organisms such as parasites, and allergens. With respect to antigens that are proteins, polypeptides, or peptides, such antigens can include nucleic acid molecules encoding such antigens. Antigens more specifically include, but are not limited to, cancer antigens, which include cancer cells and molecules expressed in or on cancer cells; microbial antigens, which include microbes and molecules expressed in or on microbes; and allergens.

As used herein, the term "asthma" refers to a disorder of the respiratory system characterized by inflammation, narrowing of the airways, and increased reactivity of the airways to inhaled agents. Asthma is frequently, although not exclusively, associated with atopic or allergic symptoms.

As used herein with reference to a nucleic acid molecule, the term "backbone" refers to the polymeric sugar-phosphate backbone of naturally occurring nucleic acids, as well as to modified counterparts and mimics thereof, to which are covalently attached the nucleobases defining a base sequence of a particular nucleic acid molecule.

As used herein, the term "cancer" refers to a population of abnormal cells that proliferate without regulation by external signals. There are two types of cancers or neoplasms, benign and malignant. Nearly all benign cancers are encapsulated and are noninvasive. In contrast, malignant cancers are almost never encapsulated but invade adjacent tissue by infiltrative destructive growth. This infiltrative growth can be accompanied by cancer cells implanting at sites discontinuous with the original cancer.

As used herein, the term "cell of the immune system" refers to any bone marrow-derived cell that can participate in an innate or adaptive immune response. Cells of the immune system may include, without limitation, dendritic cells (DC), natural killer (NK) cells, monocytes, macrophages, granulocytes, B lymphocytes, plasma cells, T lymphocytes, and precursor cells thereof.

As used herein, the term "effective amount" refers to that amount of a substance that is necessary or sufficient to bring about a desired biologic effect. An effective amount can but need not be limited to an amount administered in a single administration.

As used herein, the term "immune response" refers to any aspect of an innate or adaptive immune response that reflects activation of an immune cell to proliferate, to perform an effector immune function, or to produce a gene product involved in an immune response. Gene products involved in an immune response can include secreted products (e.g., antibodies, cytokines, and chemokines) as well as intracellular and cell surface molecules characteristic of immune function (e.g., certain cluster of differentiation (CD) antigens, transcription factors, and gene transcripts). The term "immune response" can be applied to a single cell or to a population of cells.

As used herein, the term "infection" refers to an abnormal presence of an infectious microbe or infectious agent in a host. An infection with an infectious microbe specifically includes a bacterial, viral, fungal, or parasitic infection, and any combination thereof.

As used herein, the term "isolated" as used to describe a compound shall mean removed from the natural environment in which the compound occurs in nature. In one embodiment isolated means removed from non-nucleic acid molecules of a cell.

As used herein, the term "nucleic acid molecule" refers to any molecule containing multiple nucleotides (i.e., molecules comprising a sugar (e.g., ribose or deoxyribose) linked to a phosphate group and to an exchangeable organic base, which is either a substituted pyrimidine (e.g., cytosine (C), thymine (T) or uracil (U)) or a substituted purine (e.g., adenine (A) or guanine (G)). As described further below, bases include C, T, U, C, and G, as well as variants thereof. As used herein, the term refers to ribonucleotides (including oligoribonucleotides (ORN)) as well as deoxyribonucleotides (including oligodeoxynucleotides (ODN)). The term shall also include polynucleosides (i.e., a polynucleotide minus the phosphate) and any other organic base containing polymer. Nucleic acid molecules can be obtained from existing nucleic acid sources (e.g., genomic or cDNA), but are preferably synthetic (e.g., produced by oligonucleotide synthesis).

As used herein, the term "pharmaceutically acceptable carrier" refers to one or more compatible solid or liquid filler, diluents or encapsulating substances which are suitable for administration to a human or other vertebrate animal.

As used herein, the term "phosphorothioate backbone" refers to a stabilized sugar phosphate backbone of a nucleic acid molecule in which a non-bridging phosphate oxygen is replaced by sulfur at at least one internucleoside linkage. In one embodiment a non-bridging phosphate oxygen is replaced by sulfur at each and every internucleoside linkage.

As used herein, the term "single-stranded minus-sense RNA virus" refers to any virus belonging to the order *Mononegavirales* and having a vertebrate host. In one embodiment the single-stranded minus-sense RNA virus has a genomic RNA that has a 5' end and a 3' end, i.e., is not circular.

As used herein, the term "stabilized backbone" refers to a backbone of a nucleic acid molecule that is relatively stable against nuclease degradation compared to a phosphodiester backbone.

As used herein, the term "subject" refers to a human or non-human vertebrate. Non-human vertebrates include livestock animals, companion animals, and laboratory animals. Non-human subjects also specifically include non-human primates as well as rodents. Non-human subjects also specifically include, without limitation, chickens, horses, cows, pigs, goats, dogs, cats, guinea pigs, hamsters, mink, rabbits, and fish.

As used herein, the term "subject at risk of developing" a condition refers to a subject with a known or suspected exposure to an agent known to cause or to be associated with the condition or a known or suspected predisposition to develop the condition (e.g., a genetic marker for or a family history of the condition).

As used herein, the term "Th1-like immune response" refers to any adaptive immune response or aspect thereof that is characterized by production of a type 1 interferon, interferon gamma (IFN-γ), IFN-γ-inducible 10 kDa protein (IP-10), interleukin 12 (IL-12), IgG2a (in mice), IgG1 (in humans), or cell-mediated immunity, or any combination thereof. A Th1-like immune response includes but is not limited to a Th1 immune response.

As used herein, the term "Th2-like immune response" refers to any adaptive immune response or aspect thereof that is characterized by production of interleukin 4 (IL-4), IgE, IgG1 (in mice), IgG2 (in humans), or humoral immunity, or any combination thereof. A Th2-like immune response includes but is not limited to a Th2 immune response.

As used herein, the term "TLR signaling" refers to any aspect of intracellular signaling associated with signaling through a TLR.

As used herein, the terms "TLR agonist" and, equivalently, "agonist of TLR" refer to any agent that is capable of inducing signaling by a particular TLR. TLR signaling agonists specifically include, without limitation, immunostimulatory compositions of the invention.

As used herein, the term "treat" as used in reference to a disease or condition shall mean to intervene in such disease or condition so as to prevent or slow the development of, prevent, slow, or halt the progression of, or eliminate the disease or condition.

As used herein, the term "type 1 interferon" refers to any isoform of interferon alpha (IFN-α) or interferon beta (IFN-β).

### Specific Embodiments

The invention is related in part to the discovery by the inventors that certain nucleic acid sequences present in generally highly conserved regions of genomic RNA of certain

RNA viruses are highly immunostimulatory. More specifically, it has been discovered by the inventors that sequences found at the 3' termini of single-stranded minus-sense RNA virus genomic RNA molecules are immunostimulatory. Furthermore, it has now been discovered by the inventors that nucleic acid molecules of the invention, possessing the sequences just described, act as agonists for signaling by certain TLRs. Nucleic acid molecules of the invention are potent inducers of Th1-like immune responses and thus are useful for directing an immune response toward a Th1-like immune response. Such immune skewing is useful in situations in which it is desirable to reduce or redirect a Th2-like immune response, as well as in situations in which it is desirable to induce or augment a Th1-like immune response. Conditions for which it may be desirable to reduce or redirect a Th2-like immune response may include, without limitation, allergy and asthma. Conditions for which it may be desirable to induce or augment a Th1-like immune response may include, without limitation, vaccination, treatment of various infections, treatment of cancer, and potentiation of antibody-dependent cellular cytotoxicity (ADCC).

The immunostimulatory compositions of the invention include relatively short nucleic acid molecules having a sequence found in the genomic RNA of viruses belonging to the order *Mononegavirales.* Such viruses generally include viruses with segmented or nonsegmented genomes made up of single-stranded RNA molecules that are minus-sense (sometimes referred to as (-), negative strand, negative sense, or antisense). RNA-dependent RNA polymerase transcribes the genomic RNA to make complementary, positive-strand RNA molecules that in turn serve as templates for making more minus-sense genomic RNA as well as for encoding viral polypeptide gene products. Some viruses in this group have circular genomic RNA, and others have linear (non-circular) genomic RNA. Each non-circular genomic RNA molecule has a 5' end and a 3' end. These 5' and 3' ends have sequences that are highly conserved and often partially or exactly complementary. The conservation occurs both within families and across families, particularly within families. While these same 5' and 3' ends are thought to be critical for viral replication, they are generally non-coding, i.e., they are not translated into viral polypeptide gene product.

The order *Mononegavirales* specifically includes the viral families *Orthomyxoviridae, Paramyxoviridae, Filoviridae, Rhabdoviridae, Bornaviridae, Bunyaviridae,* and *Arenaviridae.* The family *Orthomyxoviridae* includes, without limitation, influenza A virus, influenza B virus, influenza C virus, Thogotovirus, Dhori virus, and infectious salmon anemia virus. The family *Paramyxoviridae* includes, without limitation, human parainfluenza virus, human respiratory syncytial virus (RSV), Sendai virus, Newcastle disease virus, mumps virus, rubeola (measles) virus, Hendra virus, avian pneumovirus, and canine distemper virus. The family *Filoviridae* includes, without limitation, Marburg virus and Ebola virus. The family *Rhabdoviridae* includes, without limitation, rabies virus, vesicular stomatitis virus (VSV), Mokola virus, Duvenhage virus, European bat virus, salmon infectious hematopoietic necrosis virus, viral hemorrhagic septicaemia virus, spring viremia of carp virus, and snakehead rhabdovirus. The family *Bornaviridae* includes, without limitation, Boma disease virus. The family *Bunyaviridae* includes, without limitation, Bunyamwera virus, Hantaan virus, California encephalitis virus, Rift Valley fever virus, and sandfly fever virus. The family *Arenaviridae* includes, without limitation, lymphocytic choreomeningitis virus (LCMV), Lassa fever virus, delta (hepatitis D) virus, and South American hemorrhagic fever virus.

Influenza type A viruses can infect people, birds, pigs, horses, seals, whales, and other animals, but wild birds are the natural hosts for these viruses. Influenza type A viruses are divided into subtypes based on two proteins on the surface of the virus. These proteins are called hemagglutinin (HA) and neuraminidase (NA). There are 15 different HA subtypes and 9 different NA subtypes. Subtypes of influenza A virus are named according to their HA and NA surface proteins, and many different combinations of HA and NA proteins are possible.. For example, an "H7N2 virus" designates an influenza A subtype that has an HA 7 protein and an NA 2 protein. Similarly an "H5N1" virus has an HA 5 protein and an NA 1 protein. Only some influenza A subtypes (i.e., H1N1, H1N2, and H3N2) are currently in general circulation among people. Other subtypes are found most commonly in other animal species. For example, H7N7 and H3N8 viruses cause illness in horses.

Humans can be infected with influenza types A, B, and C. However, the only subtypes of influenza A virus that normally infect people are influenza A subtypes H1N1, H1N2, and H3N2. Between 1957 and 1968, H2N2 viruses also circulated among people, but currently do not.

Of the various types of influenza viruses, only influenza A viruses infect birds. Wild birds are the natural host for all subtypes of influenza A virus. Typically wild birds do not get sick when they are infected with influenza virus. However, domestic poultry, such as turkeys and chickens, can get very sick and die from avian influenza, and some avian viruses also can cause serious disease and death in wild birds.

Examples of 3' terminal 20-mer sequences include the following, shown 5' to 3' reading left to right:

| | | |
|---|---|---|
| Para-RSV | 5'-UUGUACGCAUUUUUUCGCGU-3' | (SEQ ID NO:2) |
| Para-Measles | 5' -CUUACCCAACUUUGUUUGGU-3' | (SEQ ID NO:3) |
| Para-Sendai | 5'-UGUUUUUUCUCUUGUUUGGU-3' | (SEQ ID NO:4) |
| Ortho-Influenza | 5' -AUAAUUGACCUGCUUUCGCU-3' | (SEQ ID NO:5) |
| Rhabdo-Rabies | 5'-UUGAUCUGGUUGUUAAGCGU-3' | (SEQ ID NO:6) |
| Rhabdo-VSV | 5'-AAUGGUUUGUUUGUCUUCGU-3' | (SEQ ID NO:7) |

Generally, sequences that are most highly conserved appear to be located closest to the 3' terminus. That is, generally the 3' terminal dozen nucleotides are more highly conserved than 3' penultimate dozen nucleotides. It is also to be noted that some, but not all, of these representative sequences include a 5'-CpG-3' dinucleotide.

As will be described in greater detail below, it has been discovered according to the instant invention that many of the immunostimulatory single-stranded RNA sequences of the invention are characterized by the presence of the following 4-mer sequence motif, which may typically be found within the 3' terminal 15 or so nucleotides of single-stranded minus-sense RNA virus genomes or segments thereof:

5'-C/U-U-G/U-U-3'

wherein C/U indicates C or U and G/N indicates G or U. This 4-mer sequence motif thus includes the following four individual 4-mer sequences: CUGU, UUGU, CUUU, and UUUU. Without meaning to be bound to a particular theory or mechanism, it is the belief of the inventors that this 4-mer sequence motif includes contact points for interaction with receptors TLR8, TLR7, and/or TLR3. Also without meaning to be bound to a particular theory or mechanism, it is the belief of the inventors that position 1 of the motif is C or U, critically including a 2 oxygen on the base; position 2 of the motif critically is U; position 3 of the motif is a G or U, critically including a 4 oxygen or 6 oxygen, respectively, on the base; and position 4 of the motif critically is U. This 4-mer sequence motif is noted to be strikingly similar to the reported transcriptional start site for non-segmented virus genomes, namely, 5'-CUGUU-3'.

The invention in one aspect provides an immunostimulatory composition that includes an isolated nucleic acid molecule 10 to 30 nucleotides long including a sequence provided by a 3' end of a single-stranded minus-sense RNA virus genome, wherein the nucleic acid molecule has a stabilized backbone. A 3' end of a single-stranded minus-sense RNA virus genome in one embodiment refers to a 3' end of a single-stranded minus-sense RNA virus genome wherein the genome is nonsegmented. A 3' end of a single-stranded minus-sense RNA virus genome in another embodiment refers to a 3' end of a segment of a single-stranded minus-sense RNA virus genome wherein the genome is segmented. According to this aspect of the invention, the immunostimulatory composition thus includes at least the 10 most 3' nucleotides of a single-stranded minus-sense RNA virus genome or a linear segment thereof. In one embodiment the immunostimulatory composition includes the 10 most 3' nucleotides of a single-stranded minus-sense RNA virus genome or a linear segment thereof. In one embodiment the immunostimulatory composition includes the 11 most 3' nucleotides of a single-stranded minus-sense RNA virus genome or a linear segment thereof. In one embodiment the immunostimulatory composition includes the 12 most 3' nucleotides of a single-stranded minus-sense RNA virus genome or a linear segment thereof. In like manner, in certain specific embodiments the immunostimulatory composition includes the 13, 14, 15, 16, 17, 18, 19, 20, 21, 22, 23, 24, 25, 26, 27, 28, 29, or 30 most 3' nucleotides of a single-stranded minus-sense RNA virus genome or a linear segment thereof.

Following is a listing of unique 10- to 30-mer RNA sequences which occur at the 3' termini of single-stranded minus-sense RNA virus genomic RNAs. Where more than one sequence is listed in association with a particular accession number, the shorter sequences are 5' truncations of the longest sequence in the group.

### Viruses; ssRNA negative-strand viruses; Mononegavirales; Filoviridae

### Filoviridae; Marburg-like viruses.

GenBank Accession No. Z12132
Marburg virus genes for vp35, vp40, vp30, vp24, glycoprotein, nucleoprotein, polymerase, Length = 19104

| | |
|---|---|
| 5' aaaaucaucaucucuuguuuuugugugucu 3' | (SEQ ID NO:8) |
| 5' ucucuuguuuuugugugucu 3' | (SEQ ID NO:9) |
| 5' uguuuuugugugucu 3' | (SEQ ID NO:10) |
| 5' uugugugucu 3' | (SEQ ID NO:11) |

GenBank Accession No. X68495
Marburg Virus genomic RNA of NP gene, Length = 2852

| | |
|---|---|
| 5' caaaaucaucaucucuuguuuuuguguguc 3' | (SEQ ID NO:12) |
| 5' aucucuuguuuuuguguguc 3' | (SEQ ID NO:13) |
| 5' uuguuuuuguguguc 3' | (SEQ ID NO:14) |
| 5' uuuguguguc 3' | (SEQ ID NO:15) |

GenBank Accession No. AY358025
Marburg virus strain M/S.Africa/Johannesburg/1975/Ozolin, complete sequence, Length = 19151

| | |
|---|---|
| 5' uuugugugucucucuuguuuuugugugucu 3' | (SEQ ID NO:16) |

### Filoviridae; Ebola-like viruses.

GenBank Accession No. AY354458
Zaire Ebola virus strain Zaire 1995, complete genome, Length = 18961

| | |
|---|---|
| 5' uaaaaauucuucuuucuuuuuguguguccg 3' | (SEQ ID NO:17) |
| 5' ucuuucuuuuuguguguccg 3' | (SEQ ID NO:18) |
| 5' cuuuuuguguguccg 3' | (SEQ ID NO:19) |
| 5' uguguguccg 3' | (SEQ ID NO:20) |

GenBank Accession No. M33062
Zaire Ebola virus 3' proximal protein gene, 5' end, Length = 157

| | |
|---|---|
| 5' cuaaaaauucuucuuucuuuuugugugccc 3' | (SEQ ID NO:21) |
| 5' uucuuucuuuuugugugccc 3' | (SEQ ID NO:22) |
| 5' ucuuuuugugugccc 3' | (SEQ ID NO:23) |
| 5' uugugugccc 3' | (SEQ ID NO:24) |

GenBank Accession No. AF522874
Reston Ebola virus strain Pennsylvania, complete genome, Length = 18891

| | |
|---|---|
| 5' uaaaaaaccuuuuuucuuuuuguguguccg 3' | (SEQ ID NO:25) |
| 5' uuuuucuuuuuguguguccg 3' | (SEQ ID NO:26) |

### Viruses; ssRNA negative-strand viruses; Mononegavirales; Rhabdoviridae Rhabdoviridae; Novirhabdovirus.

GenBank Accession No. L40883
Infectious hematopoietic necrosis virus, complete genome, Length = 11131

| | |
|---|---|
| 5' cugagcuuagucaaguuacuuuucuuauac 3' | (SEQ ID NO:27) |
| 5' ucaaguuacuuuucuuauac 3' | (SEQ ID NO:28) |
| 5' uuacuuuucuuauac 3' | (SEQ ID NO:29) |
| 5' uuucuuauac 3' | (SEQ ID NO:30) |

GenBank Accession No. X89213
Infectious haematopoietic necrosis virus(IHNV), complete genome, Length = 11137

| | |
|---|---|
| 5' cugagcuuagucaaguuacuuuuuuuauac 3' | (SEQ ID NO:31) |
| 5' ucaaguuacuuuuuuuauac 3' | (SEQ ID NO:32) |
| 5' uuacuuuuuuuauac 3' | (SEQ ID NO:33) |
| 5' uuuuuuauac 3' | (SEQ ID NO:34) |

GenBank Accession No. Y18263
Viral hemorrhagic septicemia virus strain Fil3 RNA, complete genome, Length = 11158

| | |
|---|---|
| 5' uguaacauaacucaucaucuuuuaugauac 3' | (SEQ ID NO:35) |
| 5' cucaucaucuuuuaugauac 3' | (SEQ ID NO:36) |
| 5' caucuuuuaugauac 3' | (SEQ ID NO:37) |
| 5' uuuaugauac 3' | (SEQ ID NO:38) |

GenBank Accession No. AF147498
Snakehead rhabdovirus complete genome, Length = 11550

| | |
|---|---|
| 5' gtatcaaaaaagatgatgatacttggaaga 3' DNA | (SEQ ID NO:39) |
| 5' ucuuccaaguaucaucaucuuuuuugauac 3' | (SEQ ID NO:40) |
| 5' aucaucaucuuuuuugauac 3' | (SEQ ID NO:41) |
| 5' caucuuuuuugauac 3' | (SEQ ID NO:42) |
| 5' uuuuugauac 3' | (SEQ ID NO:43) |

### Rhabdoviridae; Lyssavirus.

GenBank Accession No. Y09762
Mokola virus genes encoding nucleoprotein, phosphoprotein, matrice protein, glycoprotein and polymerase, Length = 11940

| | |
|---|---|
| 5' ugugucuucuuugaucugguuguuaagcgu 3' | (SEQ ID NO:44) |

GenBank Accession No. M13215
Rabies virus M2, M1, G, N, and L genes, complete cds, Length = 11932

| | |
|---|---|
| 5' uguuuuuucuuugaucugguuguuaagcgu 3' | (SEQ ID NO:45) |
| 5' uugaucugguuguuaagcgu 3' | (SEQ ID NO: 6) |
| 5' cugguuguuaagcgu 3' | (SEQ ID NO:46) |
| 5' uguuaagcgu 3' | (SEQ ID NO:47) |

GenBank Accession No. AB085828
Rabies virus genomic RNA, complete genome, strain:HEP-Flury, Length = 11615

| | |
|---|---|
| 5' ugcuucuucuuugguuuuguuguuaagcgu 3' | (SEQ ID NO:48) |
| 5' uugguuuuguuguuaagcgu 3' | (SEQ ID NO:49) |
| 5' uuuguuguuaagcgu 3' | (SEQ ID NO:50) |

GenBank Accession No. X13357
Rabies virus 3' region of genome encoding leader RNA, N, M1, M2 and G proteins, Length = 3385

| | |
|---|---|
| 5' cuuuucuucucugguuuuguuguuaagcgu 3' | (SEQ ID NO:51) |
| 5' cugguuuuguuguuaagcgu 3' | (SEQ ID NO:52) |

GenBank Accession No. D13766
Mokola virus genomic RNA, 5' end of genome, Length = 79

| | |
|---|---|
| 5' ugauuuuuauaugguuuuuuuguuaagcgu 3' | (SEQ ID NO:53) |
| 5' augguuuuuuuguuaagcgu 3' | (SEQ ID NO:54) |
| 5' uuuuuuguuaagcgu 3' | (SEQ ID NO:55) |

### Rhabdoviridae; Vesiculovirus.

GenBank Accession No. J02428
Vesicular stomatitis Indiana virus, complete genome, Length = 11161

| | |
|---|---|
| 5' uaaugauaauaaugguuuguuugucuucgu 3' | (SEQ ID NO:56) |
| 5' aaugguuuguuugucuucgu 3' | (SEQ ID NO:7) |
| 5' uuuguuugucuucgu 3' | (SEQ ID NO:57) |
| 5' uugucuucgu 3' | (SEQ ID NO:58) |

GenBank Accession No. AF473866
Vesicular stomatitis Indiana virus strain 94GUB, complete genome, Length = 11336

| | |
|---|---|
| 5' uaaugguaauaaugguuuguuugucuucgu 3' | (SEQ ID NO:59) |

GenBank Accession No. M14712
Vesicular stomatitis virus (strain Cocal) defective interfering, particle N-protein RNA, 5' end, Length = 240

| | |
|---|---|
| 5' uaauuauauuaaugguuuguuugucuucgu 3' | (SEQ ID NO:60) |

GenBank Accession No. M14713
Vesicular stomatitis virus (strain New Jersey) defective interfering particle N-protein RNA, 5' end, Length = 236

| | |
|---|---|
| 5' uaauuguaauaaugguuuuuuugucuucgu 3' | (SEQ ID NO:61) |
| 5' aaugguuuuuuugucuucgu 3' | (SEQ ID NO:62) |
| 5' uuuuuuugucuucgu 3' | (SEQ ID NO:63) |

GenBank Accession No. K02747
Vesicular stomatitis virus (New Jersey) 3' end of (-) genome, Length = 202

| | |
|---|---|
| 5' uaauuguaagaaugguuuuuuugucuucgu 3' | (SEQ ID NO:64) |

GenBank Accession No. AY102918
Vesicular stomatitis virus isolate Hazelhurst serotype New Jersey panhandle-type defective interfering particle HDI, Length = 6110

| | |
|---|---|
| 5' uuguauuaggaaugguuuuuuugucuucgu 3' | (SEQ ID NO:65) |

GenBank Accession No. AJ318079
Spring Viremia of Carp complete genome, genomic RNA, Length = 11019

| | |
|---|---|
| 5' uaauguuaucaaugguuuauuugucuucgu 3' | (SEQ ID NO:66) |
| 5' aaugguuuauuugucuucgu 3' | (SEQ ID NO:67) |
| 5' uuuauuugucuucgu 3' | (SEQ ID NO:68) |

GenBank Accession No. M14714
Vesicular stomatitis virus (strain Piry) defective interfering particle N-protein RNA, 5' end, Length = 240

| | |
|---|---|
| 5' uaagaaugcuauugguuuguuuuucuucgu 3' | (SEQ ID NO:69) |
| 5' auugguuuguuuuucuucgu 3' | (SEQ ID NO:70) |
| 5' uuuguuuuucuucgu 3' | (SEQ ID NO:71) |
| 5' uuuucuucgu 3' | (SEQ ID NO:72) |

GenBank Accession No. AY102919
Vesicular stomatitis virus isolate Ogden serotype New Jersey panhandle-type defective interfering particle ODI, Length = 2016

| | |
|---|---|
| 5' uugucauauaauugguuuuuuugucuucgu 3' | (SEQ ID NO:73) |
| 5' auugguuuuuuugucuucgu 3' | (SEQ ID NO:74) |

### Rhabdoviridae; Ephemerovirus

GenBank Accession No. AF234533
Bovine ephemeral fever virus, complete genome, Length = 14900

| | |
|---|---|
| 5' auaucaauuaguuuuuuuguuuuuucucgu 3' | (SEQ ID NO:75) |
| 5' guuuuuuuguuuuuucucgu 3' | (SEQ ID NO:76) |
| 5' uuuguuuuuucucgu 3' | (SEQ ID NO:77) |
| 5' uuuuucucgu 3' | (SEQ ID NO:78) |

GenBank Accession No. U 10363
Adelaide River virus DPP61 nucleoprotein N gene, complete cds, Length = 1405

| | |
|---|---|
| 5' aaucacuauaguuuuuuuguuuuucuccgu 3' | (SEQ ID NO:79) |
| 5' guuuuuuuguuuuucuccgu 3' | (SEQ ID NO:80) |
| 5' uuuguuuuucuccgu 3' | (SEQ ID NO:81) |
| 5' uuuucuccgu 3' | (SEQ ID NO:82) |

### Viruses; ssRNA negative-strand viruses; Mononegavirales; Paramyxoviridae Paramyxoviridae; Paramyxovirinae; Morbillivirus.

GenBank Accession No. AF017149
Hendra virus, complete genome, Length = 18234

| | |
|---|---|
| 5' aacacguauccauauuuccccuuguucggu 3' | (SEQ ID NO:83) |
| 5' cauauuuccccuuguucggu 3' | (SEQ ID NO:84) |
| 5' uuccccuuguucggu 3' | (SEQ ID NO:85) |
| 5' cuuguucggu 3' | (SEQ ID NO:86) |

GenBank Accession No. Z66517
Measles virus (strain Edmonston B) RNA (infectious cDNA clone), Length = 15894

| | |
|---|---|
| 5' uugaacuauccuuacccaacuuuguuuggu 3' | (SEQ ID NO:87) |
| 5' cuuacccaacuuuguuuggu 3' | (SEQ ID NO:3) |
| 5' ccaacuuuguuuggu 3' | (SEQ ID NO:88) |
| 5' uuuguuuggu 3' | (SEQ ID NO:89) |

GenBank Accession No. AF266288
Measles virus strain Edmonston, complete genome, Length = 15894

| | |
|---|---|
| 5' uugaucuauccuuacccaacuuuguuuggu 3' | (SEQ ID NO:90) |

GenBank Accession No. Z33635
Rinderpest virus (Kabete 'O') terminal sequence and N gene (partial), Length = 181

| | |
|---|---|
| 5' uagaccgauccuuacccaacuuuguuuggu 3' | (SEQ ID NO:91) |

GenBank Accession No. Z34262
Rinderpest virus (Kuwait 82/1) genomic RNA 3' end, N gene, Length = 1742

| | |
|---|---|
| 5' uauaccuauccuuacccagcuuuguuuggu 3' | (SEQ ID NO:92) |
| 5' cuuacccagcuuuguuuggu 3' | (SEQ ID NO:93) |
| 5' ccagcuuuguuuggu 3' | (SEQ ID NO:94) |

GenBank Accession No. Z30701
Rinderpest virus (RBOK) mRNA for N protein (partial), Length = 150

| | |
|---|---|
| 5' uagaacgauccuuacccagcuuugucuggu 3' | (SEQ ID NO:95) |
| 5' cuuacccagcuuugucuggu 3' | (SEQ ID NO:96) |
| 5' ccagcuuugucuggu 3' | (SEQ ID NO:97) |
| 5' uuugucuggu 3' | (SEQ ID NO:98) |

GenBank Accession No. AF378705
Canine distemper virus strain Onderstepoort, complete genome, Length = 15690

| | |
|---|---|
| 5' uuuaacuauccuuagccaacuuugucuggu 3' | (SEQ ID NO:99) |
| 5' cuuagccaacuuugucuggu 3' | (SEQ ID NO:100) |
| 5' ccaacuuugucuggu 3' | (SEQ ID NO:101) |

GenBank Accession No. AY386316
Canine distemper virus strain 5804P, complete genome, Length = 15690

| | |
|---|---|
| 5' uuuaucuauccuuagccaacuuugucuggu 3' | (SEQ ID NO:102) |

GenBank Accession No. AF164967
Canine distemper virus strain A75/17, complete genome, Length = 15690

| | |
|---|---|
| 5' uuuaucuauccauagccaacuuuuucuggu 3' | (SEQ ID NO:103) |
| 5' cauagccaacuuuuucuggu 3' | (SEQ ID NO:104) |
| 5' ccaacuuuuucuggu 3' | (SEQ ID NO:105) |
| 5' uuuuucuggu 3' | (SEQ ID NO:106) |

### Paramyxoviridae; Paramyxovirinae; Rubulavirus.

GenBank Accession No. AF309418
Newcastle disease virus B1, complete genome, Length = 15186

| | |
|---|---|
| 5' uuaucguaacucaccgauucucuguuuggu 3' | (SEQ ID NO:107) |
| 5' ucaccgauucucuguuuggu 3' | (SEQ ID NO:108) |
| 5' gauucucuguuuggu 3' | (SEQ ID NO:109) |
| 5' ucuguuuggu 3' | (SEQ ID NO:110) |

GenBank Accession No. AF077761
Newcastle disease virus strain LaSota, complete genome, Length = 15186

| | |
|---|---|
| 5' uuaucguaacucacggauucucuguuuggu 3' | (SEQ ID NO:111) |
| 5' ucacggauucucuguuuggu 3' | (SEQ ID NO:112) |

GenBank Accession No. AY225110
Newcastle disease virus strain HB92 isolate V4, complete genome, Length = 15186

| | |
|---|---|
| 5' uuaucguaacuuacggauucucuguuuggu 3' | (SEQ ID NO:113) |
| 5' uuacggauucucuguuuggu 3' | (SEQ ID NO:114) |

GenBank Accession No. AF431744
Newcastle Disease virus strain ZJ1, complete genome, Length = 15192

| | |
|---|---|
| 5' uuaucguaccucacagauucucuguuuggu 3' | (SEQ ID NO:115) |
| 5' ucacagauucucuguuuggu 3' | (SEQ ID NO:116) |

GenBank Accession No. X04274
Newcastle disease virus genome (strain D26) 3' end (2.6 kb), Length = 2617

| | |
|---|---|
| 5' uuaucguaccuuacagauucucuguuuggu 3' | (SEQ ID NO:117) |
| 5' uuacagauucucuguuuggu 3' | (SEQ ID NO:118) |

GenBank Accession No. AF345290
Mumps virus (STRAIN JERYL-LYNN) live vaccine minor component JL2, complete genome, Length = 15384

| | |
|---|---|
| 5' ccgauaucccaucuucuuuuuccccuuggu 3' | (SEQ ID NO:119) |
| 5' aucuucuuuuuccccuuggu 3' | (SEQ ID NO:120) |
| 5' cuuuuuccccuuggu 3' | (SEQ ID NO:121) |
| 5' uccccuuggu 3' | (SEQ ID NO:122) |

GenBank Accession No. AB000388
Mumps virus cDNA sequence of the genomic RNA, complete sequence, polymorphism, Length = 15385

| | |
|---|---|
| 5' ccaacaucccaucuucuuuuuccccuuggu 3' | (SEQ ID NO:123) |

GenBank Accession No. AF280799
Mumps virus strain Glouc1/UK96, complete genome, Length = 15384

| | |
|---|---|
| 5' ccaauaucccaucuucauuuuccccuuggu 3' | (SEQ ID NO:124) |
| 5' aucuucauuuuccccuuggu 3' | (SEQ ID NO:125) |
| 5' cauuuuccccuuggu 3' | (SEQ ID NO:126) |

GenBank Accession No. M37750
Mumps virus nucleocapsid (NP) mRNA, complete cds, and P gene, 5'flank, Length = 1989

| | |
|---|---|
| 5' accgauaucccaucuucauuuuccccuugg 3' | (SEQ ID NO:127) |
| 5' caucuucauuuuccccuugg 3' | (SEQ ID NO:128) |
| 5' ucauuuuccccuugg 3' | (SEQ ID NO:129) |
| 5' uuccccuugg 3' | (SEQ ID NO:130) |

GenBank Accession No. AF338106
Mumps virus (STRAIN JERYL-LYNN) live vaccine major component, complete genome, Length = 15384

| | |
|---|---|
| 5' ccaauaucccauauucauucuccccuuggu 3' | (SEQ ID NO:131) |
| 5' auauucauucuccccuuggu 3' | (SEQ ID NO:132) |
| 5' cauucuccccuuggu 3' | (SEQ ID NO:133) |

### Paramyxoviridae; Paramyxovirinae; Respirovirus.

GenBank Accession No. X00087
Sendai virus genome RNA for proteins NP, P, C, M and part of F, Length = 5824

| | |
|---|---|
| 5' aucccauacauguuuuuucucuuguuuggu 3' | (SEQ ID NO:134) |
| 5' uguuuuuucucuuguuuggu 3' | (SEQ ID NO:4) |
| 5' uuucucuuguuuggu 3' | (SEQ ID NO:135) |
| 5' cuuguuuggu 3' | (SEQ ID NO:136) |

GenBank Accession No. X66908
Parainfluenza virus type 1 leader region, Length = 56

| | |
|---|---|
| 5' auuccaugcaaguuuuuucucuuguuuggu 3' | (SEQ ID NO:137) |
| 5' aguuuuuucucuuguuuggu 3' | (SEQ ID NO:138) |

GenBank Accession No. AB065189
Sendai virus genomic RNA, complete genome, clone:E30M15c15, viral complementary sequence, Length = 15384

| | |
|---|---|
| 5' auuccaaacauguuucuucucuuguuuggu 3' | (SEQ ID NO:139) |
| 5' uguuucuucucuuguuuggu 3' | (SEQ ID NO:144) |
| 5' cuucucuuguuuggu 3' | (SEQ ID NO:141) |

GenBank Accession No. AB005796
Sendai virus genomic RNA, antisense, complete sequence, Length = 15384

| | |
|---|---|
| 5' auuccaaacaaguuucuucucuuguuuggu 3' | (SEQ ID NO:142) |
| 5' aguuucuucucuuguuuggu 3' | (SEQ ID NO:143) |

GenBank Accession No. AB065188
Sendai virus genomic RNA, complete genome, clone:E50c19, viral complementary sequence, Length = 15384

| | |
|---|---|
| 5' auuccauacauguuucuucucuuguuuggu 3' | (SEQ ID NO:144) |

GenBank Accession No. M29343
Sendai virus NP gene encoding nucleocapsid protein, 5' end, Length = 626

| | |
|---|---|
| 5' auuccauacacguuuuuucucuugucuggu 3' | (SEQ ID NO:145) |
| 5' cguuuuuucucuugucuggu 3' | (SEQ ID NO:146) |
| 5' uuucucuugucuggu 3' | (SEQ ID NO:147) |
| 5' cuugucuggu 3' | (SEQ ID NO:148) |

GenBank Accession No. X03614
Sendai virus (strain Z) genome RNA 5' end, Length = 10603

| | |
|---|---|
| 5' ggauacauaucucuuaaacucuugucuggu 3' | (SEQ ID NO:149) |
| 5' cucuuaaacucuugucuggu 3' | (SEQ ID NO:150) |
| 5' aaacucuugucuggu 3' | (SEQ ID NO:151) |

GenBank Accession No. Z11575
Human parainfluenza virus 3 virus RNA, Length = 15462

| | |
|---|---|
| 5' uucccagacaaguuucuucucuuguuuggu 3' | (SEQ ID NO:152) |
| 5' aguuucuucucuuguuuggu 3' | (SEQ ID NO:143) |
| 5' cuucucuuguuuggu 3' | (SEQ ID NO:141) |

GenBank Accession No. U51116
Human parainfluenza virus 3, mutant cp-45, complete genome, Length = 15462

| | |
|---|---|
| 5' uuaccaagcaaguuucuucucuuguuuggu 3' | (SEQ ID NO:153) |

GenBank Accession No. Y00114
Bovine parainfluenza 3 virus 3' end with genes NP, P, C, M, F and HN, Length = 8700

| | |
|---|---|
| 5' uucccaagcaagucucuucucuuguuuggu 3' | (SEQ ID NO:154) |
| 5' agucucuucucuuguuuggu 3' | (SEQ ID NO:155) |

GenBank Accession No. AB012132
Human parainfluenza virus 3 genomic RNA, complete sequence, viral complementary strand, Length = 15462

| | |
|---|---|
| 5' uuuccaaacaagucucuucucuuguuuggu 3' | (SEQ ID NO:156) |

GenBank Accession No. AF457102
HPIV-1 strain Washington/1964, complete genome, Length = 15600

| | |
|---|---|
| 5' auuccaaacaaguuuuuccucuuguuuggu 3' | (SEQ ID NO:157) |
| 5' aguuuuuccucuuguuuggu 3' | (SEQ ID NO:158) |
| 5' uuccucuuguuuggu 3' | (SEQ ID NO:159) |

### Paramyxoviridae; Pneumovirinae; Pneumovirus.

GenBank Accession No. U39661
Respiratory syncytial virus, complete genome, Length = 15191

| | |
|---|---|
| 5' cgcaaguuuguuguacgcauuuuuucgcgu 3' | (SEQ ID NO:160) |
| 5' uuguacgcauuuuuucgcgu 3' | (SEQ ID NO:2) |
| 5' cgcauuuuuucgcgu 3' | (SEQ 1D NO:161) |
| 5' uuuuucgcgu 3' | (SEQ ID NO:162) |

GenBank Accession No. AF013255
Human respiratory syncytial virus mutant cp52, complete genome, Length = 13933

| | |
|---|---|
| 5' ugcaaguuuguaguacgcauuuuuucgcgu 3' | (SEQ ID NO:163) |
| 5' uaguacgcauuuuuucgcgu 3' | (SEQ ID NO:164) |

GenBank Accession No. AF295543
Bovine respiratory syncytial virus ATCC51908, complete genome, Length = 15140

| | |
|---|---|
| 5' ugcaaguuuguaguacgcauuuuuucgcgu 3' | (SEQ ID NO:163) |
| 5' uaguacgcauuuuuucgcgu 3' | (SEQ ID NO:164) |

GenBank Accession No. AF035006
Human respiratory syncytial virus, recombinant mutant rA2cp, complete genome Length = 15223

| | |
|---|---|
| 5' ugcaaguuuguuguacgcauuuuuucccgu 3' | (SEQ ID NO:165) |
| 5' uuguacgcauuuuuucccgu 3' | (SEQ ID NO:166) |
| 5' cgcauuuuuucccgu 3' | (SEQ ID NO:167) |
| 5' uuuuucccgu 3' | (SEQ ID NO:168) |

### Paramyxoviridae; Pneumovirinae; Metapneumovirus.

GenBank Accession No. AY297749
Human metapneumovirus isolate CAN97-83, complete genome, Length = 13335

| | |
|---|---|
| 5' uaacuuaauuuauacgcguuuuuuucgcgu 3' | (SEQ ID NO:169) |
| 5' uauacgcguuuuuuucgcgu 3' | (SEQ ID NO:170) |
| 5' gcguuuuuuucgcgu 3' | (SEQ ID NO:171) |

### Viruses; ssRNA negative-strand viruses; Orthomyxoviridae Orthomyxoviridae; Influenza A viruses

### PB2

GenBank Accession No. AF342824
Influenza A virus (A/Wisconsin/10/98 (H1N1)) PB2 gene, partial cds, Length = 1600

| | |
|---|---|
| 5' cauauugaauauaauugcgcugcuuucgcu 3' | (SEQ ID NO:172) |
| 5' auaauugcgcugcuuucgcu 3' | (SEQ ID NO:173) |
| 5' ugcgcugcuuucgcu 3' | (SEQ ID NO:174) |
| 5' ugcuuucgcu 3' | (SEQ ID NO:175) |

GenBank Accession No. AF389115
Influenza A virus (A/Puerto Rico/8/34/Mount Sinai(HIN1)) segment 1, complete sequence, Length = 2341

| | |
|---|---|
| 5' cauauugaauauaauugaccugcuuucgcu 3' | (SEQ ID NO:176) |
| 5' auaauugaccugcuuucgcu 3' | (SEQ ID NO:5) |
| 5' ugaccugcuuucgcu 3' | (SEQ ID NO:177) |

GenBank Accession No. AJ404632
Influenza A virus pb2 gene for polymerase Pb2, genomic RNA, strain A/Hong Kong/485/97, Length = 2341

| | |
|---|---|
| 5' cauauucaauauaauugaccugcuuuucgu 3' | (SEQ ID NO:178) |
| 5' auaauugaccugcuuuucgu 3' | (SEQ ID NO:179) |
| 5' ugaccugcuuuucgu 3' | (SEQ ID NO:180) |
| 5' ugcuuuucgu 3' | (SEQ ID NO:181) |

### PB1

GenBank Accession No. AF389116
Influenza A virus (A/Puerto Rico/8/34/Mount Sinai(H1N1)) segment 2, complete sequence, Length = 2341

| | |
|---|---|
| 5' auccauucaaaugguuugccugcuuucgcu 3' | (SEQ ID NO:182) |
| 5' augguuugccugcuuucgcu 3' | (SEQ ID NO:183) |
| 5' uugccugcuuucgcu 3' | (SEQ ID NO:184) |

GenBank Accession No. AF523440
Influenza A virus (A/Duck/Hong Kong/289/78(H9N2)) polymerase (PB1) gene, partial cds, Length = 1533

| | |
|---|---|
| 5' auccauucaaaugguuugccugcuuuugcu 3' | (SEQ ID NO:185) |
| 5' augguuugccugcuuuugcu 3' | (SEQ ID NO:186) |
| 5' uugccugcuuuugcu 3' | (SEQ ID NO:187) |
| 5' ugcuuuugcu 3' | (SEQ ID NO:188) |

GenBank Accession No. AF523431
Influenza A virus (A/Wild Duck/Shantou/4808/01(H9N2)) polymerase (PB1) gene, partial cds, Length = 1512

| | |
|---|---|
| 5' auccauucaagugguuugccugcuuuugcu 3' | (SEQ ID NO:189) |
| 5' gugguuugccugcuuuugcu 3' | (SEQ ID NO:190) |

GenBank Accession No. AF258527
Influenza A virus (A/Hong Kong/470/97(H1N1)) PB1 gene, complete cds, Length = 2341

| | |
|---|---|
| 5' auccauucaaaugguuucgcugcuuucgcu 3' | (SEQ ID NO:191) |
| 5' augguuucgcugcuuucgcu 3' | (SEQ ID NO:192) |
| 5' uucgcugcuuucgcu 3' | (SEQ ID NO:193) |

### PA

GenBank Accession No. AY253752
Influenza A virus (A/Chicken/Shanghai/F/98(H9N2)) polymerase acidic protein (PA) gene, complete cds, Length = 2233

| | |
|---|---|
| 5' uuccauuuuggaucaguaccugcuuucgcu 3' | (SEQ ID NO:194) |
| 5' gaucaguaccugcuuucgcu 3' | (SEQ ID NO:195) |
| 5' guaccugcuuucgcu 3' | (SEQ ID NO:196) |

GenBank Accession No. AF342822
Influenza A virus (A/Wisconsin/10/98 (H1N1)) PA gene, partial cds, Length = 1494

| | |
|---|---|
| 5' uuccauuuuggaucaguaccugcuuuugcu 3' | (SEQ ID NO:197) |
| 5' gaucaguaccugcuuuugcu 3' | (SEQ ID NO:198) |
| 5' guaccugcuuuugcu 3' | (SEQ ID NO:199) |

GenBank Accession No. D12779
Influenza virus type A PA gene Length = 462

| | |
|---|---|
| 5' uuccauuuugaaucaguaccugcuuucgcu 3' | (SEQ ID NO:200) |
| 5' aaucaguaccugcuuucgcu 3' | (SEQ ID NO:201) |

GenBank Accession No. M23974
Influenza A/Ann Arbor/6/60(H2N2) polymerase acidic protein (PA, segment 3) RNA, complete cds, Length = 2233

| | |
|---|---|
| 5' uuccauuucgaaucaguaccugcuuucgcu 3' | (SEQ ID NO:202) |

GenBank Accession No. X17336
Influenza A virus PA gene for RNA polymerase, strain A/WSN/33(H1N1), Length = 2233

| | |
|---|---|
| 5' uuccauuuugaaucaguaccugcuuucgcu 3' | (SEQ ID NO:200) |

GenBank Accession No. AF257196
Influenza A virus (A/Hong Kong/486/97(H5N1)) RNA polymerase (PA) gene, complete cds, Length = 2233

| | |
|---|---|
| 5' uuccauuucggaucaguaccugcuuuugcu 3' | (SEQ ID NO:203) |

GenBank Accession No. AJ311464
Influenza A virus PA gene for Polymerase A protein, genomic RNA, strain A/Swine/Cotes d'Armour/3633/84 (H3N2), Length = 2233

| | |
|---|---|
| 5' uuccauucugaaucaguaccugcuuuugcu 3' | (SEQ ID NO:204) |
| 5' aaucaguaccugcuuuugcu 3' | (SEQ ID NO:205) |

### HA

GenBank Accession No. AY289928
Influenza A virus (A/Beijing/262/95(H1N1)) hemagglutinin (HA) gene, complete cds, Length = 1775

| | |
|---|---|
| 5' ugguuguuuuuauuuuccccugcuuuugcu 3' | (SEQ ID NO:206) |
| 5' uauuuuccccugcuuuugcu 3' | (SEQ ID NO:207) |
| 5' uccccugcuuuugcu 3' | (SEQ ID NO:208) |

GenBank Accession No. AF342821
Influenza A virus (A/Wisconsin/10/98 (H1N1)) heamagglutinin precursor, gene, partial cds, Length = 1064

| | |
|---|---|
| 5' ugguugcuuuuauuuuccccugcuuuugcu 3' | (SEQ ID NO:209) |

GenBank Accession No. AF386779
Influenza A virus (A/Hong Kong/1035/98 (H1N1)) hemagglutinin gene, partial cds, Length = 1262

| | |
|---|---|
| 5' ugguugguuuuauuuuccccugcuuuugcu 3' | (SEQ ID NO:210) |

GenBank Accession No. D13574
Influenza A virus (A/Suita/1/89/(R)(H1N1)) gene for hemagglutinin, complete cds, Length = 1778

| | |
|---|---|
| 5' ugguuguauuuauuuuccccugcuuuugcu 3' | (SEQ ID NO:211) |

GenBank Accession No. U72669
Influenza A virus hemagglutinin (HA) gene, complete cds, Length = 1778

| | |
|---|---|
| 5' ugguugauuuuauuuuccccugcuuuugcu 3' | (SEQ ID NO:212) |

GenBank Accession No. AF091313
Influenza A virus (A/duck/Bavaria/1/77 (H1N1)) segment 4, hemagglutinin precursor (HA) mRNA, complete cds, Length = 1777

| | |
|---|---|
| 5' ugguugauuuaauuuuccccugcuuuugcu 3' | (SEQ ID NO:213) |
| 5' aauuuuccccugcuuuugcu 3' | (SEQ ID NO:214) |

GenBank Accession No. AF091312
Influenza A virus (A/duck/Australia/749/80(H1N1)) segment 4, hemagglutinin precursor (HA) mRNA, complete cds, Length = 1777

| | |
|---|---|
| 5' ugguugauuugauuucccccugcuuuugcu 3' | (SEQ ID NO:215) |
| 5' gauuucccccugcuuuugcu 3' | (SEQ ID NO:216) |

GenBank Accession No. AY060051
Influenza A virus (A/SW/MN/34893/01(H1N2)) hemagglutinin (HA) gene, complete cds, Length = 1771

| | |
|---|---|
| 5' gguugcuuuuauuuuccccugcuuuugcua 3' | (SEQ ID NO:217) |
| 5' auuuuccccugcuuuugcua 3' | (SEQ ID NO:218) |
| 5' ccccugcuuuugcua 3' | (SEQ ID NO:219) |
| 5' gcuuuugcua 3' | (SEQ ID NO:220) |

GenBank Accession No. AF222035
Influenza A virus (A/Swine/Wisconsin/458/98(H1N1)) hemagglutinin (HA) gene, complete cds, Length = 1757

| | |
|---|---|
| 5' guugcuuuuauuuuccccugcuuuugcuaa 3' | (SEQ ID NO:221) |
| 5' uuuuccccugcuuuugcuaa 3' | (SEQ ID NO:222) |
| 5' cccugcuuuugcuaa 3' | (SEQ ID NO:223) |
| 5' cuuuugcuaa 3' | (SEQ ID NO:224) |

### NP

GenBank Accession No. AY129159
Influenza A virus (A/Swine/Korea/CY02/02(H1N2)) nucleoprotein (NP) mRNA, complete cds, Length = 1542

| | |
|---|---|
| 5' cauugagugauuaucuacccugcuuuugcu 3' | (SEQ ID NO:225) |
| 5' uuaucuacccugcuuuugcu 3' | (SEQ ID NO:226) |
| 5' uacccugcuuuugcu 3' | (SEQ ID NO:227) |

GenBank Accession No. AY253753
Influenza A virus (A/Chicken/Shanghai/F/98(H9N2)) nucleoprotein (NP) gene, complete cds, Length = 1565

| | |
|---|---|
| 5' cggugagugauuaucuacccugcuuuugcu 3' | (SEQ ID NO:228) |

GenBank Accession No. AF261750
Influenza A virus (A/chicken/Taiwan/7-5/99(H6N1)) nucleocapsid, protein (NP) mRNA, complete cds, Length = 1565

| | |
|---|---|
| 5' cggugagagauuaucuacccugcuuuugcu 3' | (SEQ ID NO:229) |

GenBank Accession No. M22576
Influenza A virus (A/FPV/Rostock/34(H7N1)) nucleoprotein gene, complete cds, Length = 1565

| | |
|---|---|
| 5' cggugagagauuaucuacccugcuuuugcu 3' | (SEQ ID NO:229) |

GenBank Accession No. L07347
Influenza A virus (strain A/memphis/4/73) nucleoprotein (NP) gene, complete cds, Length = 1565

| | |
|---|---|
| 5' cagugagugauuaucaacccugcuuuugcu 3' | (SEQ ID NO:230) |
| 5' uuaucaacccugcuuuugcu 3' | (SEQ ID NO:231) |
| 5' aacccugcuuuugcu 3' | (SEQ ID NO:232) |

GenBank Accession No. X51972
Influenza A virus np gene for nucleoprotein, strain A/Kiev/59/79(H1N1), Length = 1565

| | |
|---|---|
| 5' cagugagugauuauuaacccugcuuuugcu 3' | (SEQ ID NO:233) |
| 5' uuauuaacccugcuuuugcu 3' | (SEQ ID NO:234) |

### NA

GenBank Accession No. K01025
Influenza A/New Jersey/11/76 (H1N1), neuraminidase (seg 6), 5' end, Length = 215

| | |
|---|---|
| 5' uuguauucauuuuaaaccccugcuuuugcu 3' | (SEQ ID NO:235) |
| 5' uuuaaaccccugcuuuugcu 3' | (SEQ ID NO:236) |
| 5' accccugcuuuugcu 3' | (SEQ ID NO:237) |

GenBank Accession No. D31946
Influenza A virus gene for neuraminidase, complete cds, Length = 1458

| | |
|---|---|
| 5' uuguauucauuuuaaacuccugcuuuugcu 3' | (SEQ ID NO:238) |
| 5' uuuaaacuccugcuuuugcu 3' | (SEQ ID NO:239) |
| 5' acuccugcuuuugcu 3' | (SEQ ID NO:240) |

GenBank Accession No. AY261521
Influenza A virus (A/turkey/Ontario/HR2/2000(H7N1)) neuraminidase (NA) gene, partial cds, Length = 1405

| | |
|---|---|
| 5' ggauucauuuugaacuccugcuuuugcuaa 3' | (SEQ ID NO:241) |
| 5' ugaacuccugcuuuugcuaa 3' | (SEQ ID NO:242) |
| 5' uccugcuuuugcuaa 3' | (SEQ ID NO:243) |

GenBank Accession No. AF144304
Influenza A virus (A/Goose/Guangdong/1/96(H5N1)) neuraminidase (NA) gene, complete cds, Length = 1458

| | |
|---|---|
| 5' uuggauucauuuuaaucuccugcuuuugcu 3' | (SEQ ID NO:244) |
| 5' uuuaaucuccugcuuuugcu 3' | (SEQ ID NO:245) |
| 5' ucuccugcuuuugcu 3' | (SEQ ID NO:246) |

GenBank Accession No. AF509114
Influenza A virus (A/Goose/Hong Kong/ww100/01 (H5N1)) neuraminidase (NA) gene, partial cds, Length = 1155

| | |
|---|---|
| 5' auuuggauucauuuuaaucuccugcuuuug 3' | (SEQ ID NO:247) |
| 5' auuuuaaucuccugcuuuug 3' | (SEQ ID NO:248) |
| 5' aaucuccugcuuuug 3' | (SEQ ID NO:249) |
| 5' ccugcuuuug 3' | (SEQ ID NO:250) |

### M1 M2

GenBank Accession No. AF386772
Influenza A virus (A/Hong Kong/1180/99(H3N2)) matrix protein M1 and matrix protein M2 genes, complete cds, Length = 1027

| | |
|---|---|
| 5' cucaucuuucaauaucuaccugcuuuugcu 3' | (SEQ ID NO:251) |
| 5' aauaucuaccugcuuuugcu 3' | (SEQ ID NO:252) |
| 5' cuaccugcuuuugcu 3' | (SEQ ID NO:253) |

GenBank Accession No. AY253755
Influenza A virus (A/Chicken/Shanghai/F/98(H9N2)) matrix protein M1 and membrane ion channel M2 genes, complete cds, Length = 1027

| | |
|---|---|
| 5' cucaucuuucaacaucuaccugcuuuugcu 3' | (SEQ ID NO:254) |
| 5' aacaucuaccugcuuuugcu 3' | (SEQ ID NO:255) |

GenBank Accession No. AF389121
Influenza A virus (A/Puerto Rico/8/34/Mount Sinai(H1N1)) segment 7, complete sequence, Length = 1027

| | |
|---|---|
| 5' cucaucuuucaauaucuaccugcuuucgcu 3' | (SEQ ID NO:256) |
| 5' aauaucuaccugcuuucgcu 3' | (SEQ ID NO:257) |
| 5' cuaccugcuuucgcu 3' | (SEQ ID NO:258) |

### NS1 NS2

GenBank Accession No. AF389122
Influenza A virus (A/Puerto Rico/8/34/Mount Sinai(H1N1)) segment 8, complete sequence, Length = 890

| | |
|---|---|
| 5' ccauuaugucuuugucacccugcuuuugcu 3' | (SEQ ID NO:259) |
| 5' cacccugcuuuugcu 3' | (SEQ ID NO:260) |

### Orthomyxoviridae; Influenza B viruses

### PB1

GenBank Accession No. M20170
Influenza B/Ann Arbor/1/66 (wild-type) polymerase (seg 1) RNA, complete cds, Length = 2369

| | |
|---|---|
| 5' uauauucaucuuaaaggcuccgcuucugcu 3' | (SEQ ID NO:261) |
| 5' uuaaaggcuccgcuucugcu 3' | (SEQ ID NO:262) |
| 5' ggcuccgcuucugcu 3' | (SEQ ID NO:263) |
| 5' cgcuucugcu 3' | (SEQ ID NO:264) |

GenBank Accession No. M14880
Influenza B/Lee/40, PB1 polymerase (seg 1) RNA, complete, Length = 2368

| | |
|---|---|
| 5' uuauauucaucuuaaagcuccgcuucugcu 3' | (SEQ ID NO:265) |
| 5' cuuaaagcuccgcuucugcu 3' | (SEQ ID NO:266) |
| 5' agcuccgcuucugcu 3' | (SEQ ID NO:267) |

### PB2

GenBank Accession No. AF005737
Influenza B virus B/Panama/45/90 polymerase (PB2) mRNA, complete cds, Length = 2396

| | |
|---|---|
| 5' augucaucuugaaaacgcuccgcuucugcu 3' | (SEQ ID NO:268) |
| 5' gaaaacgcuccgcuucugcu 3' | (SEQ ID NO:269) |
| 5' cgcuccgcuucugcu 3' | (SEQ ID NO:270) |

### PA

GenBank Accession No. AF005738
Influenza B virus B/Panama/45/90 polymerase (PA) mRNA, complete cds, Length = 2305

| | |
|---|---|
| 5' uuauggcaaaucaaacgcaccgcuucugcu 3' | (SEQ ID NO:271) |
| 5' ucaaacgcaccgcuucugcu 3' | (SEQ ID NO:272) |
| 5' cgcaccgcuucugcu 3' | (SEQ ID NO:273) |

GenBank Accession No. M16711
Influenza type B/Singapore/222/79 polymerase acidic protein gene, complete cds, Length = 2304

| | |
|---|---|
| 5' uuauggcaaaucaaacguaucgcuucugcu 3' | (SEQ ID NO:274) |
| 5' ucaaacguaucgcuucugcu 3' | (SEQ ID NO:275) |
| 5' cguaucgcuucugcu 3' | (SEQ ID NO:276) |

### HA

GenBank Accession No. AF387504
Influenza B virus (B/Switzerland/4291/97) hemagglutinin mRNA, complete cds, Length = 1882

| | |
|---|---|
| 5' guggauauuagaaaaugcucugcuucugcu 3' | (SEQ ID NO:277) |
| 5' gaaaaugcucugcuucugcu 3' | (SEQ ID NO:278) |
| 5' ugcucugcuucugcu 3' | (SEQ ID NO:279) |
| 5' ugcuucugcu 3' | (SEQ ID NO:280) |

GenBank Accession No. AF306548
Infectious salmon anemia virus putative polymerase mRNA, complete cds, Length = 1805

| | |
|---|---|
| 5' uauccaucuugaaaauagccaaucuuagcu 3' | (SEQ ID NO:281) |
| 5' gaaaauagccaaucuuagcu 3' | (SEQ ID NO:282) |
| 5' uagccaaucuuagcu 3' | (SEQ ID NO:283) |
| 5' aaucuuagcu 3' | (SEQ ID NO:284) |

It has now been found by the inventors that oligonucleotides as short as 7 nucleotides long and containing the immunostimulatory 4-mer RNA motif 5'-C/U-U-G/U-U-3' are immunostimulatory. This sequence motif occurs in many of the viral sequences just described above. Accordingly, in one aspect the invention provides an immunostimulatory oligonucleotide as short as 7 nucleotides long and containing the immunostimulatory 4-mer RNA motif 5'-C/U-U-G/U-U-3'. Sequence outside the 4-mer RNA motif can be any sequence. In one embodiment the oligonucleotide does not include the sequence 5'-GUUGU-3'. The sequence outside the 4-mer RNA motif can be RNA, DNA, or a mixture of RNA and DNA. Sequence outside the motif can include one or more modified ribonucleosides, one or more modified deoxyribonucleosides, one or more modified internucleoside linkages, or any combination thereof. In various embodiments the immunostimulatory oligonucleotide can be 7, 8, 9, 10, 11, 12, 13, 14, 15, 16, 17, 18, 19, 20, 21, 22, 23, 24, 25, 26, 27, 28, 29, or 30 nucleotides long. In one embodiment the oligonucleotide has a stabilized backbone. Such oligonucleotides can be used in any of the methods disclosed herein, including methods for stimulating an immune response, stimulating a Thl-like immune response, stimulating TLR signaling, stimulating an immune response in a subject, stimulating a Th1-like immune response in a subject, stimulating an antigen-specific immune response in a subject, treating an allergic condition in a subject, treating asthma in a subject, treating an infection in a subject, treating cancer in a subject, and screening for an antagonist of a TLR.

As disclosed in Example 6 below, it has also been discovered by the inventors that a non-immunostimulatory oligonucleotide at least 3 nucleotides long can be converted to an immunostimulatory oligonucleotide by introducing into such non-immunostimulatory oligonucleotide the immunostimulatory 4-mer RNA motif 5'-CIU-U-GIU-U-3'. The resulting immunostimulatory oligonucleotide is at least 7 nucleotides long. The motif can be added or introduced anywhere in the oligonucleotide, e.g., at a 5' end, at a 3' end, or internal to the 5' and 3' ends. The sequence outside the 4-mer motif can be any sequence. In one embodiment the resulting oligonucleotide does not include the sequence 5'-GUUGU-3'. The sequence outside the 4-mer RNA motif can be RNA, DNA, or a mixture of RNA and DNA. In various embodiments the resulting immunostimulatory oligonucleotide can be 7, 8, 9, 10, 11, 12, 13, 14, 15, 16, 17, 18, 19, 20, 21, 22, 23, 24, 25, 26, 27, 28, 29, or 30 nucleotides long. In one embodiment the resulting oligonucleotide has a stabilized backbone. Such oligonucleotides can be used in any of the methods disclosed herein, including methods for stimulating an immune response, stimulating a Th1-like immune response, stimulating TLR signaling, stimulating an immune response in a subject, stimulating a Th1-like immune response in a subject, stimulating an antigen-specific immune response in a subject, treating an allergic condition in a subject, treating asthma in a subject, treating an infection in a subject, treating cancer in a subject, and screening for an antagonist of a TLR.

It has also been discovered by the inventors that a weakly immunostimulatory oligonucleotide at least 3 nucleotides long can be converted to a more potent immunostimulatory oligonucleotide by introducing into such non-immunostimulatory oligonucleotide the immunostimulatory 4-mer RNA motif 5'-C/U-U-G/U-U-3'. The resulting immunostimulatory oligonucleotide is at least 7 nucleotides long. The motif can be added or introduced anywhere in the oligonucleotide, e.g., at a 5' end, at a 3' end, or internal to the 5' and 3' ends. The sequence outside the 4-mer RNA motif can be any sequence. In one embodiment the resulting oligonucleotide does not include the sequence 5'-GUUGU-3'. The sequence outside the 4-mer RNA motif can be RNA, DNA, or a mixture of RNA and DNA. In various embodiments the resulting immunostimulatory oligonucleotide can be 7, 8, 9, 10, 11, 12, 13, 14, I5, 16, 17, 18, 19, 20, 21, 22, 23, 24, 25, 26, 27, 28, 29, or 30 nucleotides long. In one embodiment the resulting oligonucleotide has a stabilized backbone. Such oligonucleotides can be used in any of the methods disclosed herein, including methods for stimulating an immune response, stimulating a Th1-like immune response, stimulating TLR signaling, stimulating an immune response in a subject, stimulating a Th1-like immune response in a subject, stimulating an antigen-specific immune response in a subject, treating an allergic condition in a subject, treating asthma in a subject, treating an infection in a subject, treating cancer in a subject, and screening for an antagonist of a TLR.

It has now been found by the inventors that at least certain immunostimulatory CpG DNA oligonucleotide sequences can be converted into immunostimulatory RNA oligonucleotide sequences of the invention by substituting U for both T and C or, alternatively, by substituting U for C. In one embodiment the starting CpG DNA oligonucleotide has the sequence 5'-tcgtcgttttgtcgttttgtcgtt-3' (ODN 2006, SEQ ID NO:285). A corresponding RNA oligonucleotide of the invention has the sequence 5'-uuguuguuuuguuguuuuguuguu-3' (SEQ ID NO:286). Another corresponding RNA oligonucleotide of the invention has the sequence 5'-tugtugttttgtugttttgtugtt-3' (SEQ ID N0:287). In one embodiment the starting CpG DNA oligonucleotide has the sequence 5'-tcgtcgttttcggcggccgccg-3' (SEQ ID NO:288). A corresponding RNA oligonucleotide of the invention has the sequence 5'-uuguuguuuuuggugguuguug-3' (SEQ ID NO:289). Another corresponding RNA oligonucleotide of the invention has the sequence 5'-tugtugttttuggugguuguug-3' (SEQ ID NO:290). Such changes are believed to represent, surprisingly, relatively conservative nucleotide substitutions to immunostimulatory CpG DNA oligonucleotides that may permit the resulting RNA oligonucleotides to interact with TLRs, other than TLR9, which are paralogs of TLR9.

In particular, it is believed that conversion of a CpG ODN to include the immunostimulatory 4-mer RNA motif 5'-C/U-U-G/U-U-3' can confer a new immunostimulatory profile upon the resulting oligonucleotide, that is, the resulting oligonucleotide stimulates TLRs in addition to and/or different from TLR9 stimulated by the starting CpG ODN. For example, complete conversion of a CpG ODN such as ODN 2006 into an oligoribonucleotide (ORN) containing the immunostimulatory 4-mer RNA motif 5'-C/U-U-G/U-U-3' may result in loss of capacity to stimulate TLR9 and gain of capacity to stimulate TLR7, TLR8, or both TLR7 and TLR8. Partial conversion may result in yet a different profile.

In addition to conversion or partial conversion of a CpG ODN from DNA to RNA, just described, it has been discovered by the inventors that existing CpG ODN can be modified to have a new profile of immunostimulatory activity by adding or otherwise introducing into the CpG ODN the immunostimulatory 4-mer RNA motif 5'-C/U-U-G/U-U-3'. The resulting combination motif oligonucleotide stimulates TLRs in addition to and/or different from TLR9 stimulated by the starting CpG ODN.

The compositions of the invention can include certain artificially synthesized oligonucleotides having a base sequence that corresponds to a base sequence found in nature, i.e., a base sequence found in the 3' end of a single-stranded minus-sense RNA virus genome. The compositions are artificially synthesized in order to include the feature of the stabilized backbone. The backbone of an oligonucleotide can be stabilized using any suitable chemical method or modification, provided the oligonucleotide having a stabilized backbone is relatively more resistant to nuclease degradation than a corresponding oligonucleotide having an all-phosphodiester backbone.

The immunostimulatory oligonucleotides of the instant invention can encompass various chemical modifications and substitutions, in comparison to natural RNA and DNA, involving a phosphodiester internucleoside bridge, a β-D-ribose unit, and/or a natural nucleoside base (adenine, guanine, cytosine, thymine, uracil). Examples of chemical modifications are known to the skilled person and are described, for example, in Uhlmann E et al. (1990) Chem Rev 90:543; "Protocols for Oligonucleotides and Analogs" Synthesis and Properties & Synthesis and Analytical Techniques, S. Agrawal, Ed, Humana Press, Totowa, USA 1993; Crooke ST et al. (1996) Annu Rev Pharmacol Toxicol 36:107-29; and Hunziker J et al. (1995) Mod Synth Methods 7:331-417. An oligonucleotide according to the invention may have one or more modifications, wherein each modification is located at a particular internucleoside bridge and/or at a particular β-D-ribose unit and/or at a particular natural nucleoside base position in comparison to an oligonucleotide of the same sequence which is composed of natural DNA or RNA.

For example, the oligonucleotides may include one or more modifications wherein each modification is independently selected from:
a) the replacement of a phosphodiester internucleoside bridge located at the 3' and/or the 5' end of a nucleoside by a modified internucleoside bridge,
b) the replacement of a phosphodiester internucleoside bridge located at the 3' and/or the 5' end of a nucleoside by a dephospho bridge,
c) the replacement of a sugar phosphate unit from the sugar phosphate backbone by another unit,
d) the replacement of a β-D-ribose unit by a modified sugar unit, and
e) the replacement of a natural nucleoside base by a modified nucleoside base.

More detailed examples for the chemical modification of an oligonucleotide are as follows.

The oligonucleotides may include modified internucleoside linkages, such as those described in a or b above. These modified linkages may be partially resistant to degradation (e.g., are stabilized). A "stabilized oligonucleotide molecule" shall mean an oligonucleotide that is relatively resistant to in vivo degradation (e.g., via an exo- or endo-nuclease) resulting from such modifications. Oligonucleotides having phosphorothioate linkages, in some embodiments, may provide maximal activity and protect the oligonucleotide from degradation by intracellular exo- and endo-nucleases.

A phosphodiester internucleoside bridge located at the 3' and/or the 5' end of a nucleoside can be replaced by a modified internucleoside bridge, wherein the modified internucleoside bridge is for example selected from phosphorothioate, phosphorodithioate, NR¹R²-phosphoramidate, boranophosphate, α-hydroxybenzyl phosphonate, phosphate-(C₁-C₂₁)-O-alkyl ester, phosphate-[(C₆-C-₁₂)aryl-(C₁-C₂₁)-O-alkyl]ester, (C₁-C₈)alkylphosphonate and/or (C₆-C₁₂)arylphosphonate bridges, (C₇-C₁₂)-α-hydroxymethyl-aryl (e.g., disclosed in WO 95/01363), wherein (C₆-C₁₂)aryl, (C₆-C₂₀)arYl, and (C₆-C₁₄)aryl are optionally substituted by halogen, alkyl, alkoxy, nitro, cyano, and where R¹ and R² are, independently of each other, hydrogen, (C₁-C₁₈)-alkyl, (C₆-C₂₀)-arYl, (C₆-C₁₄)-aryl-(C₁-C₈)-alkyl, preferably hydrogen, (C₁-C₈)-alkyl, preferably (C₁-C₄)-alkyl and/or methoxyethyl, or R¹ and R² form, together with the nitrogen atom carrying them, a 5-6-membered heterocyclic ring which can additionally contain a further heteroatom from the group O, S, and N.

The replacement of a phosphodiester bridge located at the 3' and/or the 5' end of a nucleoside by a dephospho bridge (dephospho bridges are described, for example, in Uhlmann E and Peyman A in "Methods in Molecular Biology", Vol. 20, "Protocols for Oligonucleotides and Analogs", S. Agrawal, Ed., Humana Press, Totowa 1993, Chapter 16, pp. 355 ff), may be a dephospho bridge selected from the dephospho bridges formacetal, 3'-thioformacetal, methylhydroxylamine, oxime, methylenedimethyl-hydrazo, dimethylenesulfone, and/or silyl groups.

A sugar phosphate unit (i.e., a β-D-ribose and phosphodiester intemucleoside bridge together forming a sugar phosphate unit) from the sugar phosphate backbone (i.e., a sugar phosphate backbone is composed of sugar phosphate units) can be replaced by another unit, wherein the other unit is for example suitable to build up a "morpholino-derivative" oligomer (as described, for example, in Stirchak EP et al. (1989) Nucleic Acids Res 17:6129-41), that is, e.g., the replacement by a morpholino-derivative unit; or to build up a polyamide nucleic acid ("PNA"; as described for example, in Nielsen PE et al. (1994) Bioconjug Chem 5:3-7), that is, e.g., the replacement by a PNA backbone unit, e.g., by 2-aminoethylglycine. The oligonucleotide may have other carbohydrate backbone modifications and replacements, such as peptide nucleic acids with phosphate groups (PHONA), locked nucleic acids (LNA), and oligonucleotides having backbone sections with alkyl linkers or amino linkers. The alkyl linker may be branched or unbranched, substituted or unsubstituted, and chirally pure or a racemic mixture.

In addition to the stabilized backbones disclosed above, the compositions of the instant invention can alternatively or in addition contain pyrophosphate internucleoside linkages. The synthesis and ribonuclease inhibition by *3*',*5*'-pyrophosphate-linked nucleotides have been described, for example, in Russo N et al. (1999) J Biol Chem 274:14902-8.

The compositions of the instant invention can alternatively or in addition contain a chimeric RNA:DNA backbone in which at least one nucleotide is a deoxynucleotide, e.g., a deoxyribonucleotide. The number and position of the at least one deoxynucleotide may affect immunostimulatory activity of the oligonucleotide. In various embodiments the number of deoxynucleotides in an immunostimulatory nucleic acid of the invention having the 4-mer sequence motif 5'-C/U-U-G/CT-U-3' may be 1, 2, 3, 4, 5, 6, 7, 8, 9, 10, 11, 12, 13, 14, 15, 16, 17, 18, 19, 20, 21, 22, 23, 24, 25, or 26. In some embodiments in which there is more than one deoxynucleotide, deoxynucleotides are adjacent (i.e., directly linked) to one another. In various embodiments the number of consecutive adjacent deoxynucleotides may be 2, 3, 4, 5, 6, 7, 8, 9, 10, 11, 12, 13, 14, 15, 16, 17, 18, 19, 20, 21, 22, 23, 24, 25, or 26. Groups of adjacent deoxynucleotides can also be present, separated from one another by at least one intervening nucleotide that is not a deoxynucleotide. In some embodiments in which there is more than one deoxynucleotide, no deoxynucleotide is adjacent to another deoxynucleotide. In some embodiments the position of the at least one deoxynucleotide may increase the immunostimulatory effect of the oligonucleotide compared to a corresponding oligonucleotide that is strictly RNA. In other embodiments the position of the at least one deoxynucleotide may decrease the immunostimulatory effect of the oligonucleotide compared to a corresponding oligonucleotide that is strictly RNA.

In one embodiment it has been discovered according to the instant invention that chimeric RNA:DNA oligonucleotides of the invention include conjugates capable of stimulating different TLRs. More specifically, it has been discovered that certain chimeric RNA:DNA oligonucleotides of the invention are capable of stimulating both TLR9 and TLR8. In one embodiment the DNA portion of the chimeric RNA:DNA oligonucleotide is a CpG DNA that stimulates TLR9 activity; the RNA portion of the same chimeric RNA:DNA oligonucleotide is an immunostimulatory RNA of the invention that stimulates TLR8. In one embodiment such a chimeric conjugate is provided as 5'-tcgtcgttttguuguuuuguuguu-3' (SEQ ID NO:291), wherein tcgtcgtttt (SEQ ID NO:292) is CpG DNA and guuguuuuguuguu (SEQ ID NO:293) is RNA. It is to be noted that guuguuuuguuguu (SEQ ID NO:293) includes the 4-mer sequence motifs 5'-UUGU-3' and 5'-UUUU-3'. In another embodiment such a chimeric conjugate is provided as 5'-tcgtcgttttuggugguuguug -3'(SEQ ID NO:294), wherein tcgtcgtttt (SEQ ID NO:292) is again CpG DNA and uggugguuguug (SEQ ID NO:295) is RNA. It is to be noted that uggugguuguug (SEQ ID NO:295) includes the 4-mer sequence motif 5'-UUGU-3'.

In one embodiment both the DNA and the RNA portions of the chimeric RNA:DNA oligonucleotide include 3'-5' internucleotide linkages. In another embodiment the RNA portion of the chimeric RNA:DNA oligonucleotide includes 2'-5' internucleotide linkages (rather than 3'-5' internucleotide linkages). For example, in one embodiment the RNA:DNA chimeric conjugate has the sequence 5'-tcgtcgtttguuguguaat-3' (SEQ ID NO:296), wherein tcgtcgttt and aat are DNA and wherein guugugu is RNA and all internucleotide linkages are 3'-5' internucleotide linkages. This chimeric RNA:DNA conjugate was found to stimulate both TLR9 and TLR8 and to induce IFN-α, TNF-α, and IFN-γ. In contrast, an oligonucleotide with the identical sequence and DNA and RNA composition but in which guugugua are interconnected by 2'-5' internucleotide linkages, rather than 3'-5' internucleotide linkages, was found to stimulate TLR9 but not TLR8 and to induce IFN-α, but neither TNF-α nor IFN-γ.

Nucleic acid compositions of the invention can include modified sugar units. A β-ribose unit or a β-D-2'-deoxyribose unit can be replaced by a modified sugar unit, wherein the modified sugar unit is for example selected from β-D-ribose, α-D-2'-deoxyribose, L-2'-deoxyribose, 2'-F-2'-deoxyribose, 2'-F-arabinose, 2'-O-(C₁-C₆)alkyl-ribose, 2'-O-methylribose, 2'-O-(C₂-C₆)alkenyl-ribose, 2'-[O-(C₁-C₆)alkyl-O-(C₁-C₆)alkyl]-ribose, 2'-NH₂-2'-deoxyribose, β-D-xylo-furanose, α-arabinofuranose, 2,4-dideoxy-β-D-erythro-hexo-pyranose, and carbocyclic (described, for example, in Froehler (1992) JAm Chem Soc 114:8320) and/or open-chain sugar analogs (described, for example, in Vandendriessche et al. (1993) Tetrahedron 49:7223) and/or bicyclosugar analogs (described, for example, in Tarkov M et al. (1993) Helv Chim Acta 76:481).

In one embodiment the 2' hydroxyl group of the ribose of the U in position 2 of the 4-mer sequence motif 5'-C/U-U-G/U-U-3' is intact, i.e., the β-ribose unit at this position is not replaced by any of the foregoing modified sugar units. In one embodiment 2' hydroxyl group of the ribose of the U in position 2 of the 4-mer sequence motif 5'-C/U-U-G/U-U-3' is not replaced by 2'-O-methylribose. It is believed by the inventors that the 2' hydroxyl groups in these positions may be involved in the interaction between the RNA oligonucleotide and the TLR. In support of this notion, it has been discovered that replacement within the 4-mer motif of usual 3'-5' intemucteotide linkages with 2'-5' internucleotide linkages significantly reduces the immunostimulatory activity of the oligonucleotide. It may be possible, however, to include such 2'-5' internucleotide linkages, or other nuclease-resistant linkages, in positions outside of the 4-mer sequence motif. Such RNA oligonucleotides would retain both the ability to signal through TLR and the property of being relatively resistant to degradation.

Nucleic acid compositions of the invention can include nucleosides found in nature, including guanosine, cytidine, adenosine, thymidine, and uridine, but the nucleic acid compositions are not so limited. Nucleic acid compositions of the invention can include modified nucleosides. Modified nucleosides include nucleoside derivatives with modifications involving the base, the sugar, or both the base and the sugar.

Nucleic acids also include substituted purines and pyrimidines such as C-5 propyne pyrimidine and 7-deaza-7-substituted purine modified bases. Wagner RW et al. (1996) Nat Biotechnol 14:840-4*.* Purines and pyrimidines include but are not limited to adenine, cytosine, guanine, thymine, and uracil, and other naturally and non-naturally occurring nucleobases, substituted and unsubstituted aromatic moieties.

A modified base is any base which is chemically distinct from the naturally occurring bases typically found in DNA and RNA, such as T, C, G, A, and U, but which shares basic chemical structure with at least one of these naturally occurring bases. The modified nucleoside base may be, for example, selected from hypoxanthine, dihydrouracil, pseudouracil, 2-thiouracil, 4-thiouracil, 5-aminouracil, 5-(C₁-C₆)-alkyluracil, 5-(C₂-C₆)-alkenyluracil, 5-(C₂-C₆)-alkynyluracil, 5-(hydroxymethyl)uracil, 5-chlorouracil, 5-fluorouracil, 5-bromouracil, 5-hydroxycytosine, 5-(C₁-C₆)-alkylcytosine, 5-(C₂-C₆)-alkenylcytosine, 5-(C₂-C₆)-alkynylcytosine, 5-chlorocytosine, 5-fluorocytosine, 5-bromocytosine, N²-dimethylguanine, 2,4-diamino-purine, 8-azapurine, a substituted 7-deazapurine (e.g., 7-deaza-7-substituted purine and/or 7-deaza-8-substituted purine), 5-hydroxymethylcytosine, N4-alkylcytosine, e.g., N4-ethylcytosine, 5-hydroxydeoxycytidine, 5-hydroxymethyldeoxycytidine, N4-alkyldeoxycytidine, e.g., N4-ethyldeoxycytidine, 6-thiodeoxyguanosine, and deoxyribonucleosides of nitropyrrole, C5-propynylpyrimidine, and diaminopurine e.g., 2,6-diaminopurine, inosine, 5-methylcytosine, 2-aminopurine, 2-amino-6-chloropurine, or other modifications of a natural nucleoside base. This list is meant to be exemplary and is not to be interpreted to be limiting.

In particular embodiments described herein modified bases may be incorporated. For instance a cytosine may be replaced with a modified cytosine. A modified cytosine as used herein is a naturally occurring or non-naturally occurring pyrimidine base analog of cytosine which can replace this base without impairing the immunostimulatory activity of the oligonucleotide.

Modified cytosines include but are not limited to 5-substituted cytosines (e.g., 5-methyl-cytosine, 5-fluoro-cytosine, 5-chloro-cytosine, 5-bromo-cytosine, 5-iodo-cytosine, 5-hydroxy-cytosine, 5-hydroxymethyl-cytosine, 5-difluoromethyl-cytosine, and unsubstituted or substituted 5-alkynyl-cytosine), 6-substituted cytosines, N4-substituted cytosines (e.g., N4-ethyl-cytosine), 5-aza-cytosine, 2-mercapto-cytosine, isocytosine, pseudo-isocytosine, cytosine analogs with condensed ring systems (e.g., N,N'-propylene cytosine or phenoxazine), and uracil and its derivatives (e.g., 5-fluoro-uracil, 5-bromo-uracil, 5-brornovinyl-uracil, 4-thio-uracil, 5-hydroxy-uracil, 5-propynyl-uracil). In certain embodiments of the invention, the cytosine base is substituted by a universal base (e.g., 3-nitropyrrole, P-base), an aromatic ring system (e.g., fluorobenzene or difluorobenzene), or a hydrogen atom (Spacer or dSpacer).

Cytidine derivatives generally will also include, without limitation, cytidines with modified sugars. Cytidines with modified sugars include but are not limited to cytosine-β-D-arabinofuranoside (Ara-C), ribo-C, and 2'-O-(C₁-C₆)alkyl-cytidine (e.g., 2'-O-methylcytidine, 2'-OMe-C).

A guanine may be replaced with a modified guanine base. A modified guanine as used herein is a naturally occurring or non-naturally occurring purine base analog of guanine which can replace this base without impairing the immunostimulatory activity of the oligonucleotide.

Modified guanines include but are not limited to 7-deazaguanine, 7-deaza-7-substituted guanine (such as 7-deaza-7-(C2-C6)alkynylguanine), 7-deaza-8-substituted guanine, hypoxanthine, N2-substituted guanines (e.g., N2-methyl-guanine), 5-amino-3-methyl-3H,6H-thiazolo[4,5-d]pyrimidine-2,7-dione, 2,6-diaminopurine, 2-aminopurine, purine, indole, adenine, substituted adenines (e.g., N6-methyl-adenine, 8-oxo-adenine), 8-substituted guanine (e.g., 8-hydroxyguanine and 8-bromoguanine), and 6-thioguanine. In certain embodiments of the invention, the guanine base is substituted by a universal base (e.g., 4-methyl-indole, 5-nitro-indole, and K-base), an aromatic ring system (e.g., benzimidazole or dichloro- benzimidazole, 1-methyl-1H-[1,2,4]triazole-3-carboxylic acid amide), or a hydrogen atom (Spacer or dspacer).

The nucleic acid compositions of the invention are oligonucleotides 10 to 30 nucleotides long. It is the belief of the inventors, however, that oligonucleotides as short as 4 or 5 nucleotides in length may be sufficient to bind to a TLR. In various embodiments the oligonucleotide is 10, 11, 12, 13, 14, 15, 16, 17, 18, 19, 20, 21, 22, 23, 24, 25, 26, 27, 28, 29, or 30 nucleotides long. In one embodiment the oligonucleotide is 10 to 20 nucleotides long. In one embodiment the oligonucleotide is 10 nucleotides long.

The nucleic acid compositions of the invention can be single-stranded or double-stranded, including partially double-stranded. When the oligonucleotide includes double-stranded nucleic acid, the double-stranded portion includes sufficient complementary sequence to maintain the double-stranded structure under physiological conditions. This may include a plurality of adjacent or nonadjacent basepairs chosen from G-C, A-U, A-T, G-T, and G-U. In one embodiment the basepairs are chosen from G-C, A-U, and G-U. The double-stranded structure can involve RNA-RNA duplex formation, RNA-DNA duplex formation, DNA-DNA duplex formation, or duplex formation involving at least one chimeric RNA:DNA sequence (i.e., chimeric RNA:DNA-DNA duplex, chimeric RNA:DNA-RNA duplex, or chimeric RNA:DNA-chimeric RNA:DNA duplex).

### Source and Preparation of Immunostimulatory Oligonucleotides of the Invention

For use in the instant invention, the oligonucleotides of the invention can be synthesized de novo using any of a number of procedures well known in the art, for example, the β-cyanoethyl phosphoramidite method (Beaucage SL et al. (1981) Tetrahedron Lett 22:1859); or the nucleoside H-phosphonate method (Garegg et al. (1986) Tetrahedron Lett 27:4051-4; Froehler BC et al. (1986) Nucleic Acids Res 14:5399-407; Garegg et al. (1986) Tetrahedron Left 27:4055-8; Gaffney et al. (1988) Tetrahedron Lett 29:2619-22). These chemistries can be performed by a variety of automated nucleic acid synthesizers available in the market. These oligonucleotides are referred to as synthetic oligonucleotides. An isolated oligonucleotide generally refers to an oligonucleotide which is separated from components with which it is normally associated in nature. As an example, an isolated oligonucleotide may be one which is separated from a cell, from a nucleus, from mitochondria or from chromatin. In one embodiment an isolated oligonucleotide is a synthetic oligonucleotide.

Modified backbones such as phosphorothioates may be synthesized using automated techniques employing either phosphoramidate or H-phosphonate chemistries. Aryl-and alkyl-phosphonates can be made, e.g., as described in U.S. Pat. No. 4,469,863; and alkylphosphotriesters (in which the charged oxygen moiety is alkylated as described in U.S. Pat. No. 5,023,243 and European Patent No. 092,574) can be prepared by automated solid phase synthesis using commercially available reagents. Methods for making other DNA and RNA backbone modifications and substitutions have been described (e.g., Uhlmann E et al. (1990) Chem Rev 90:544; Goodchild J (1990) Bioconjugate Chem 1:165).

In certain embodiments the immunostimulatory nucleic acid molecules of the invention may be conjugated with another agent. In one embodiment an agent that may be conjugated with the nucleic acid molecule of the invention can be a TLR ligand, including, without limitation, another nucleic acid molecule of the invention. In one embodiment an agent that may be conjugated with the nucleic acid molecule of the invention can be an immunostimulatory nucleic acid molecule that is not an immunostimulatory nucleic acid of the invention. For example, the other agent can be a CpG-DNA molecule (see, for example, U.S. Pat. Nos. 6,194,388; 6,207,646; 6,214,806; 6,218,371; 6,239,116; 6,339,086; 6,406,705; 6,429,199; and 6,653,292). In one embodiment an agent that may be conjugated with the nucleic acid molecule of the invention can be a TLR agonist. A TLR agonist is any agent that induces or augments a TLR-mediated signal. TLR agonists include, e.g., a small molecule such as R-837 (imiquimod) or R-848 (resiquimod). In one embodiment an agent that may be conjugated with the nucleic acid molecule of the invention can be a TLR antagonist. A TLR antagonist is any agent that inhibits a TLR-mediated signal. TLR antagonists include certain small molecules (see, for example, U.S. Pat. Nos. 6,221,882; 6,399,630; and 6,479,504, issued to Macfarlane, et al.) as well as certain immunoinhibitory oligonucleotides (see, for example, Lenart P et al. (2001) Antisense Nucleic Acid Drug Dev 11:247-56; Stunz LL et al. (2002) Eur J Immunol 32:1212-22; Lenert P et al. (2003) Antisense Nucleic Acid Drug Dev 13:143-50; and Lenert P et al. (2003) DNA Cell Biol 22:621-31). In one embodiment an agent that may be conjugated with the nucleic acid molecule of the invention can be an antigen, including an antigen per se or a nucleic acid molecule that encodes an antigen. In one embodiment an agent that may be conjugated with the nucleic acid molecule of the invention can be a medicament. In each of these embodiments the immunostimulatory nucleic acid molecule of the invention can be conjugated with the other agent through any suitable direct or indirect physicochemical linkage. In one embodiment the linkage is a covalent bond. In one embodiment the immunostimulatory nucleic acid molecule of the invention can be conjugated with the other agent through a linker.

In one aspect the invention provides a composition including a conjugate of an antigen or other therapeutic agent and an isolated immunostimulatory oligonucleotide of the invention. In one embodiment the antigen or other therapeutic agent is linked directly to the immunostimulatory oligonucleotide of the invention, for example through a covalent bond. In one embodiment the antigen or other therapeutic agent is linked indirectly to the immunostimulatory oligonucleotide of the invention, for example through a linker. When the antigen or other therapeutic agent of the conjugate is a polynucleotide encoding a peptide or polypeptide, the antigen or other therapeutic agent and the isolated immunostimulatory oligonucleotide can be incorporated into a single expression vector. When the antigen or other therapeutic agent of the conjugate is a preformed polypeptide or polysaccharide, the antigen or other therapeutic agent and the isolated immunostimulatory oligonucleotide can be linked using methods well known in the art.

In one embodiment the immunostimulatory nucleic acid molecule of the invention can be conjugated with the antigen or other therapeutic agent through a linkage that involves the 3' end of the nucleic acid molecule of the invention. In one embodiment the immunostimulatory nucleic acid molecule of the invention can be conjugated with the antigen or other therapeutic agent through a linkage that involves the 5' end of the nucleic acid molecule of the invention.

In one embodiment the immunostimulatory nucleic acid molecule of the invention can be conjugated with the antigen or other therapeutic agent through a linkage that does not involve the 3' end of the nucleic acid molecule of the invention. In one embodiment the immunostimulatory nucleic acid molecule of the invention can be conjugated with the antigen or other therapeutic agent through a linkage that does not involve the 5' end of the nucleic acid molecule of the invention.

For administration in vivo, immunostimulatory nucleic acid molecules of the invention may be associated with a molecule that results in higher affinity binding to target cell (e.g., B cell, monocytic cell, NK cell, dendritic cell) surfaces and/or increased cellular uptake by target cells to form a "nucleic acid delivery complex". Nucleic acids can be ionically or covalently associated with appropriate molecules using techniques which are well known in the art. A variety of coupling or crosslinking agents can be used, e.g., protein A, carbodiimide, and N-succinimidyl-3-(2-pyridyldithio) propionate (SPDP). Nucleic acids can alternatively be encapsulated in liposomes or virosomes using well-known techniques.

In some embodiments the immunostimulatory nucleic acid molecules of the invention may be mixed with or otherwise associated with a cationic lipid. Immunostimulatory nucleic acid molecules of the invention that are mixed with or otherwise associated with a cationic lipid may take the form of cationic lipid/nucleic acid complexes, including liposomes. Although immunostimulatory nucleic acid molecules of the invention are biologically active when used alone (i.e., as "naked" oligonucleotides), association with cationic lipid has been observed to increase biological activity of the immunostimulatory nucleic acid molecules of the invention. Without meaning to be bound to any particular theory or mechanism, it is believed that the increased biological activity associated with the use of cationic lipid is due to increased efficiency of cellular uptake of the immunostimulatory nucleic acid molecules of the invention. Such lipids are commonly used for transfection applications in molecular biology. Cationic lipids useful in the invention include, without limitation, DOTAP (N-[1-(2,3-dioleoyloxy)propyl]-N,N,N-trimethylammonium methylsulfate), DOTMA (N-[1-(2,3-dioleoyloxy)propyl]-N,N,N-trimethylammonium chloride), DOSPA (2,3-dioleyloxy-N-[2(sperminecarboxamido)ethyl]-N, N-dimethyl-1-propanaminium trifluoroacetate), DMRIE (N,N-dimethyl-2,3-bis(tetradecylaxy)-1-propanaminium bromide), DOGS (dioctadecylamidoglycyl spermine), cholesterol, liposomes, and any combination thereof.

As an alternative to association with cationic lipids, the immunostimulatory nucleic acid molecules of the invention may advantageously be associated with other types of cationic moieties, including, for example, polycationic peptides including polyarginine, polyarginine/polylysine, and protamine.

In each of the foregoing aspects of the invention, the immunostimulatory nucleic acid molecule of the invention may be present optionally as a salt or hydrate of the free nucleic acid.

In each of the foregoing aspects of the invention, the composition can also further include a pharmaceutically acceptable carrier, such that the invention also provides pharmaceutical compositions containing the isolated immunostimulatory oligonucleotides of the invention. Such pharmaceutical compositions can be prepared by placing an isolated immunostimulatory oligonucleotide of the invention in contact with a pharmaceutically acceptable carrier.

### Methods and Uses

Compositions of the invention can be used in the treatment of allergy, asthma, infection, cancer, or autoimmune disease.

The compositions of the invention can be used in the preparation of a medicament for the treatment of allergy, asthma, infection, cancer, or autoimmune disease. The use involves placing a therapeutically effective amount of a composition of the invention to treat allergy, asthma, infection, cancer, or autoimmune disease in contact with a pharmaceutically acceptable carrier.

The invention in one aspect provides a method for stimulating an immune response. The method according to this aspect of the invention involves the step of contacting a cell of the immune system with an effective amount of a composition of the invention to stimulate an immune response. The method can be practiced in vitro or in vivo. In certain embodiments the cell of the immune system can be part of a population of cells of the immune system, wherein the population can be a mixed population of various types of cells of the immune system or, alternatively, a purified population of a single type of cell of the immune system. When the population is a purified population of a single type of cell of the immune system, in one embodiment the selected single type of cell accounts for at least 90 percent of the population of cells. In other embodiments involving a purified population of a single type of cell of the immune system, the selected single type of cell accounts for at least 95 percent or at least 99 percent of the population of cells. In one embodiment the method involves the step of contacting peripheral blood mononuclear cells (PBMC) with an effective amount of a composition of the invention to stimulate an immune response.

An immune response can be measured using any suitable method capable of detecting at least one feature of an immune response. Methods for detecting and measuring immunostimulatory effects, i.e., an immune response, are described below.

The invention in one aspect provides a method for stimulating a Th1-like immune response. The method according to this aspect of the invention involves the step of contacting a cell of the immune system with an effective amount of a composition of the invention to stimulate a Th1-like immune response. The method can be practiced in vitro or in vivo. In one embodiment method involves the step of contacting peripheral blood mononuclear cells (PBMC) with an effective amount of a composition of the invention to stimulate a Th1-like immune response. The Th1-like immune response can include expression of certain cytokines and chemokines, including IFN-α, IFN-β, IFN-γ, TNF-α, IL-12, IL-18, IP-10, and any combination thereof. In some embodiments the Th1-like immune response can include suppression of certain Th2-associated cytokines, including IL-4, IL-5, and IL-13. The Th1-like immune response can include expression of certain antibody isotypes, including (in the mouse) IgG2a, with or without suppression of certain Th2-associated antibody isotypes, including IgE and (in the mouse) IgG1.

The invention in one aspect provides a method for stimulating TLR signaling. The method according to this aspect involves the step of contacting a cell expressing a TLR with an effective amount of a composition of the invention to stimulate signaling by the TLR. The method can be practiced in vitro or in vivo. It is the belief of the inventors that the highly conserved RNA sequences present at the 3' termini of single-stranded minus-sense RNA virus genomic RNAs are naturally occurring agonists of, and possibly ligands for, certain TLRs, including TLR8, TLR7, and TLR3. It is the belief of the inventors that the immunostimulatory nucleic acid molecules of the invention, which incorporate the highly conserved RNA sequences present at the 3' termini of single-stranded minus-sense RNA virus genomic RNAs, are agonists of, and possibly ligands for, these same TLRs, namely TLR8, TLR7, and TLR3.

Accordingly, in one embodiment the method involves the step of contacting a cell expressing TLR8 with an effective amount of a composition of the invention to stimulate signaling by the TLR8. In one embodiment the method involves the step of contacting a cell expressing TLR7 with an effective amount of a composition of the invention to stimulate signaling by the TLR7. In one embodiment the method involves the step of contacting a cell expressing TLR3 with an effective amount of a composition of the invention to stimulate signaling by the TLR3.

In each of the foregoing embodiments, the cell expressing a TLR may be a cell that naturally expresses the TLR. Such cells may include cells found in nature, e.g., PBMC. Alternatively and in addition, such cells may include cells that are cloned or are part of cell line.

Alternatively, in each of the foregoing embodiments, the cell expressing a TLR may be a cell that artificially expresses the TLR. Such cells specifically may include cells that have been transiently or stably transfected with a vector encoding the TLR, such that the transfected cells express the TLR encoded by the vector. Vectors encoding specific TLRs include coding region nucleotide sequences for the specific TLRs. Such nucleotide sequences are publicly available from databases such as GenBank, as described in more detail further below.

An artificially expressed TLR may be a human TLR. In one embodiment the transfected cells are 293HEK human fibroblast cells stably transfected with an expression vector for human TLR8. In one embodiment the transfected cells are 293HEK human fibroblast cells stably transfected with an expression vector for human TLR7. In one embodiment the transfected cells are 293HEK human fibroblast cells stably transfected with an expression vector for human TLR3.

An artificially expressed TLR may be a non-human TLR. In one embodiment the transfected cells are 293HEK human fibroblast cells stably transfected with an expression vector for murine TLR8. In one embodiment the transfected cells are 293HEK human fibroblast cells stably transfected with an expression vector for murine TLR7. In one embodiment the transfected cells are 293HEK human fibroblast cells stably transfected with an expression vector for murine TLR3.

Cells that naturally or artificially express a specific TLR can optionally include a reporter construct that is sensitive to signaling mediated by the TLR. The reporter construct can be used to detect TLR signaling activity. Any of a number of such reporter constructs may be used in the practice of the methods of the invention. In one embodiment the reporter construct includes a reporter gene, the transcription of which is under the control of a transcription factor that is induced by TLR signaling, e.g., NF-κB. In one embodiment the reporter construct includes a luciferase (luc) gene placed under the control of NF-κB response element, i.e., NF-κB-luc. Such constructs are commercially available.

The invention in one aspect provides a method for stimulating an immune response in a subject. The method according to this aspect of the invention involves the step of administering to a subject an effective amount of a composition of the invention to stimulate an immune response in the subject. In this and all aspects of the invention involving administration of a composition of the invention to a subject, the effective amount may be administered in a single dose or it may be administered in more than a single dose. Furthermore, the administering may be accomplished using any suitable route or combination of suitable routes of administration, including, without limitation, enteral administration, parenteral administration, mucosal administration, local administration, and systemic administration. Methods of detecting an immune response in the subject include any suitable method, including, without limitation, methods that are described herein.

The term "effective amount" of a nucleic acid molecule refers to that amount of the nucleic acid molecule that is necessary or sufficient to bring about a desired biologic effect. For example, an effective amount of a nucleic acid molecule of the invention for treating a disorder could be that amount necessary to induce an immune response of sufficient magnitude to eliminate a cancer or a viral, bacterial, fungal, or parasitic infection. An effective amount for use as a vaccine could be that amount useful for priming and boosting a protective immune response in a subject. The effective amount for any particular application can vary depending on such factors as the disease or condition being treated, the particular nucleic acid being administered, the size of the subject, or the severity of the disease or condition. One of ordinary skill in the art can empirically determine the effective amount of a particular oligonucleotide without necessitating undue experimentation. An effective amount for use as a prophylactic vaccine is that amount useful for priming and boosting a protective immune response in a subject. In one embodiment the protective immune response is an antigen-specific immune response.

The invention in one aspect provides a method for stimulating a Th1-like immune response in a subject. The method according to this aspect of the invention involves the step of administering to a subject an effective amount of a composition of the invention to stimulate a Th1-like immune response in the subject.

The invention in one aspect provides a method for stimulating an antigen-specific immune response in a subject. The method according to this aspect of the invention involves the steps of administering to a subject an effective amount of a composition of the invention and contacting the subject with an antigen to stimulate an antigen-specific immune response in the subject. The step of contacting the subject with an antigen may involve active contact (e.g., deliberate administration) or passive contact (e.g., environmental exposure) with the antigen. In one embodiment the method involves the steps of administering to a subject an effective amount of a composition of the invention and administering to the subject an effective amount of an antigen to stimulate an antigen-specific immune response in the subject. In one embodiment the antigen is an allergen and the antigen-specific response is specific for the allergen. In one embodiment the antigen is a viral antigen and the antigen-specific response is specific for the viral antigen. In one embodiment the antigen is a bacterial antigen and the antigen-specific response is specific for the bacterial antigen. In one embodiment the antigen is a fungal antigen and the antigen-specific response is specific for the fungal antigen. In one embodiment the antigen is an antigen of a parasite and the antigen-specific response is specific for the antigen of the parasite. In one embodiment the antigen is a cancer antigen and the antigen-specific response is specific for the cancer antigen.

As used herein, the terms "cancer antigen" and "tumor antigen" are used interchangeably to refer to antigens which are differentially expressed by cancer cells and can thereby be exploited in order to target cancer cells. Cancer antigens are antigens which can potentially stimulate apparently tumor-specific immune responses. Some of these antigens are encoded, although not necessarily expressed, by normal cells. These antigens can be characterized as those which are normally silent (i.e., not expressed) in normal cells, those that are expressed only at certain stages of differentiation and those that are temporally expressed such as embryonic and fetal antigens. Other cancer antigens are encoded by mutant cellular genes, such as oncogenes (e.g., activated ras oncogene), suppressor genes (e.g., mutant p53), fusion proteins resulting from internal deletions or chromosomal translocations. Still other cancer antigens can be encoded by viral genes such as those carried on RNA and DNA tumor viruses.

A cancer antigen as used herein is a compound, such as a peptide, protein, or glycoprotein, which is associated with a tumor or cancer cell surface and which is capable of provoking an immune response when expressed on the surface of an antigen-presenting cell in the context of a major histocompatibility complex (MHC) molecule. Cancer antigens can be prepared from cancer cells either by preparing crude extracts of cancer cells, for example, as described in Cohen PA et al. (1994) Cancer Res 54:1055-8, by partially purifying the antigens, by recombinant technology, or by de novo synthesis of known antigens. Cancer antigens include but are not limited to antigens that are recombinantly expressed, an immunogenic portion of, or a whole tumor or cancer or cell thereof. Such antigens can be isolated or prepared recombinantly or by any other means known in the art.

Examples of tumor antigens include MAGE, MART-1/Melan-A, gp100, dipeptidyl peptidase IV (DPPIV), adenosine deaminase-binding protein (ADAbp), cyclophilin b, colorectal associated antigen (CRC)-C017-1A/GA733, carcinoembryonic antigen (CEA) and its immunogenic epitopes CAP-1 and CAP-2, etv6, aml1, prostate specific antigen (PSA) and its immunogenic epitopes PSA-1, PSA-2, and PSA-3, prostate-specific membrane antigen (PSMA), T-cell receptor/CD3-zeta chain, MAGE-family of tumor antigens (e.g., MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5), GAGE-family of tumor antigens (e.g., GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family, HER2/neu, p21ras, RCAS1, α-fetoprotein, E-cadherin, α-catenin, β-catenin and γ-catenin, p120ctn, gp100^{Pmel117}, PRAME, NY-ESO-1, cdc27, adenomatous polyposis coli protein (APC), fodrin, Connexin 37, Ig-idiotype, p15, gp75, GM2 and GD2 gangliosides, viral products such as human papillomavirus proteins, Smad family of tumor antigens, Imp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, and c-erbB-2. This list is not meant to be limiting.

A microbial antigen as used herein is an antigen of a microorganism and includes but is not limited to viruses, bacteria, parasites, and fungi. Such antigens include the intact microorganism as well as natural isolates and fragments or derivatives thereof and also synthetic compounds which are identical to or similar to natural microorganism antigens and induce an immune response specific for that microorganism. A compound is similar to a natural microorganism antigen if it induces an immune response (humoral and/or cellular) to a natural microorganism antigen. Such antigens are used routinely in the art and are well known to those of ordinary skill in the art.

The antigen may be an antigen that is encoded by a nucleic acid vector or it may be not encoded in a nucleic acid vector. In the former case the nucleic acid vector is administered to the subject and the antigen is expressed in vivo. In the latter case the antigen may be administered directly to the subject. An antigen not encoded in a nucleic acid vector as used herein refers to any type of antigen that is not a nucleic acid. For instance, in some aspects of the invention the antigen not encoded in a nucleic acid vector is a polypeptide. Minor modifications of the primary amino acid sequences of polypeptide antigens may also result in a polypeptide which has substantially equivalent antigenic activity as compared to the unmodified counterpart polypeptide. Such modifications may be deliberate, as by site-directed mutagenesis, or may be spontaneous. All of the polypeptides produced by these modifications are included herein as long as antigenicity still exists. Other types of antigens not encoded by a nucleic acid vector such as polysaccharides, small molecule, mimics, etc., are included within the invention.

The invention in some embodiments utilizes polynucleotides encoding the antigenic polypeptides. It is envisioned that the antigen may be delivered to the subject in a nucleic acid molecule which encodes for the antigen such that the antigen may be expressed in vivo. Such antigens delivered to the subject in a nucleic acid vector are referred to as antigens encoded by a nucleic acid vector. The nucleic acid encoding the antigen is operatively linked to a gene expression sequence which directs the expression of the antigen nucleic acid within a eukaryotic cell. The gene expression sequence is any regulatory nucleotide sequence, such as a promoter sequence or promoter-enhancer combination, which facilitates the efficient transcription and translation of the antigen nucleic acid to which it is operatively linked. The gene expression sequence may be, for example, a mammalian or viral promoter, such as a constitutive or inducible promoter. Constitutive mammalian promoters include, but are not limited to, the promoters for the following genes: hypoxanthine phosphoribosyl transferase (HPRT), adenosine deaminase, pyruvate kinase, β-action, and other constitutive promoters. Exemplary viral promoters which function constitutively in eukaryotic cells include, for example, promoters from the cytomegalovirus (CMV), simian virus (e.g., SV40), papilloma virus, adenovirus, human immunodeficiency virus (HIV), Rous sarcoma virus, the long terminal repeats (LTR) of Moloney leukemia virus and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined antigen nucleic acid. The gene expression sequences optionally include enhancer sequences or upstream activator sequences as desired.

The antigen nucleic acid is operatively linked to the gene expression sequence. As used herein, the antigen nucleic acid sequence and the gene expression sequence are said to be operably linked when they are covalently linked in such a way as to place the expression or transcription and/or translation of the antigen coding sequence under the influence or control of the gene expression sequence. Two DNA sequences are said to be operably linked if induction of a promoter in the 5' gene expression sequence results in the transcription of the antigen sequence and if the nature of the linkage between the two DNA sequences does not (1) result in the introduction of a frame-shift mutation, (2) interfere with the ability of the promoter region to direct the transcription of the antigen sequence, or (3) interfere with the ability of the corresponding RNA transcript to be translated into a protein. Thus, a gene expression sequence would be operably linked to an antigen nucleic acid sequence if the gene expression sequence were capable of effecting transcription of that antigen nucleic acid sequence such that the resulting transcript is translated into the desired protein or polypeptide.

The antigen nucleic acid of the invention may be delivered to the immune system alone or in association with a vector. In its broadest sense, a vector is any vehicle capable of facilitating the transfer of the antigen nucleic acid to the cells of the immune system so that the antigen can be expressed and presented on the surface of the immune cell. The vector generally transports the nucleic acid to the immune cells with reduced degradation relative to the extent of degradation that would result in the absence of the vector. The vector optionally includes the above-described gene expression sequence to enhance expression of the antigen nucleic acid in immune cells. In general, the vectors useful in the invention include, but are not limited to, plasmids, phagemids, viruses, other vehicles derived from viral or bacterial sources that have been manipulated by the insertion or incorporation of the antigen nucleic acid sequences. Viral vectors are a preferred type of vector and include, but are not limited to, nucleic acid sequences from the following viruses: retrovirus, such as Moloney murine leukemia virus, Harvey murine sarcoma virus, murine mammary tumor virus, and Rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known in the art.

Preferred viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell line with plasmid, production of recombinant retroviruses by the packaging cell line, collection of viral particles from tissue culture media, and infection of the target cells with viral particles) are provided in Kriegler, M., Gene Transfer and Expression: A Laboratory Manual, W.H. Freeman and Co., New York (1991) and Murray, E.J., Methods in Molecular Biology, vol. 7, Humana Press, Inc., Cliffton, New Jersey (1991).

A preferred virus for certain applications is the adeno-associated virus, a double-stranded DNA virus. The adeno-associated virus can be engineered to be replication-deficient and is capable of infecting a wide range of cell types and species. It further has advantages, such as heat and lipid solvent stability; high transduction frequencies in cells of diverse lineages, including hemopoietic cells; and lack of superinfection inhibition, thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well-known to those of skill in the art. See, e.g., Sambrook et al., Molecular Cloning: A Laboratory Manual, Second Edition, Cold Spring Harbor Laboratory Press, 1989. In the last few years, plasmid vectors have been found to be particularly advantageous for delivering genes to cells in vivo because of their inability to replicate within and integrate into a host genome. These plasmids, however, having a promoter compatible with the host cell, can express a peptide from a gene operatively encoded within the plasmid. Some commonly used plasmids include pBR322, pUC18, pUC19, pRc/CMV, SV40, and pBlueScript. Other plasmids are well-known to those of ordinary skill in the art. Additionally, plasmids may be custom designed using restriction enzymes and ligation reactions to remove and add specific fragments of DNA.

It has recently been discovered that gene-carrying plasmids can be delivered to the immune system using bacteria. Modified forms of bacteria such as *Salmonella* can be transfected with the plasmid and used as delivery vehicles. The bacterial delivery vehicles can be administered to a host subject orally or by other administration means. The bacteria deliver the plasmid to immune cells, e.g., B cells and DC, likely by passing through the gut barrier. High levels of immune protection have been established using this methodology. Such methods of delivery are useful for the aspects of the invention utilizing systemic delivery of antigen, immunostimulatory nucleic acid, and/or other therapeutic agent.

The step of contacting the subject with antigen or administering the antigen to the subject can take place before, essentially simultaneously with, or following administering an effective amount of immunostimulatory oligonucleotide. For example, the administering the immunostimulatory oligonucleotide in certain embodiments takes place at least one day before the subject contacts the antigen. As another example, the administering the immunostimulatory oligonucleotide in certain embodiments takes place at least one day after the subject contacts the antigen. At least one day includes any time that is more than 24 hours and up to and including four weeks. In individual embodiments the at least one day is at least: 2 days, 3 days, 4 days, 5 days, 6 days, one week, two weeks, three weeks, or four weeks. In other embodiments the administering the immunostimulatory oligonucleotide can take place within 24 hours of the contacting or administering the antigen.

The invention in one aspect provides a method for treating an allergic condition in a subject. The method according to this aspect of the invention involves the step of administering to a subject having or at risk of developing an allergic condition an effective amount of a composition of the invention to treat the allergic condition.

A subject having an allergic condition is a subject that has or is at risk of developing an allergic reaction in response to an allergen. Allergic conditions are typically episodic, triggered by exposure to allergen. In one embodiment the allergic condition is active at the time of administration of the immunostimulatory composition of the invention.

A subject at risk of developing an allergic condition includes those subjects that have been identified as having an allergic condition but that do not have the active disease at the time of immunostimulatory nucleic acid treatment, as well as subjects that are considered to be at risk of developing an allergic condition because of genetic or environmental factors.

The list of allergens is enormous and can include pollens, insect venoms, animal dander dust, fungal spores and drugs (e.g., penicillin). Examples of natural animal and plant allergens include proteins specific to the following genuses: Canis (*Canis familiaris*); Dermatophagoides (e.g., *Dermatophagoides farinae*); Felis (*Felis domesticus*); Ambrosia *(Ambrosia artemiisfolia;* Lolium (e.g., *Lolium perenne* and *Lolium multflorum);* Cryptomeria *(Cryptomeria japonica);* Alternaria *(Alternaria alternata);* Alder; Alnus *(Alnus gultinosa*); Betula *(Betula verrucosa);* Quercus *(Quercus alba);* Olea *(Olea europa*); Artemisia *(Artemisia vulgaris);* Plantago (e.g., *Plantago lanceolata*); Parietaria (e.g., *Parietaria officinalis* and *Parietaria judaica*); Blattella (e.g., *Blattella germanica);* Apis (e.g., *Apis multijlorum);* Cupressus (e.g., *Cupressus sempervirens, Cupressus arizonica* and *Cupressus macrocarpa);* Juniperus (e.g., *Juniperus sabinoides, Juniperus virginiana, Juniperus communis,* and *Juniperus ashei*); Thuya (e.g., *Thuya orientalis);* Chamaecyparis (e.g., *Chamaecyparis obtusa);* Periplaneta (e.g., *Periplaneta americana);* Agropyron (e.g., *Agropyron repens);* Secale (e.g., *Secale cereale);* Triticum (e.g., *Triticum aestivum);* Dactylis (e.g., *Dactylis glomerata);* Festuca (e.g., *Festuca elatior);* Poa (e.g., *Poa pratensis* and *Poa compressa*); Avena (e.g., *Avena sativa);* Holcus (e.g., *Holcus lanatus);* Anthoxanthum (e.g., *Anthoxanthum odoratum);* Arrhenatherum (e.g., *Arrhenatherum elatius*); Agrostis (e.g., *Agrostis alba);* Phleum (e.g., *Phleum pratense);* Phalaris (e.g., *Phalaris arundinacea);* Paspalum (e.g., *Paspalum notatum*); Sorghum (e.g., *Sorghum halepensis);* and Bromus (e.g., *Bromus inermis).*

The invention in one aspect provides a method for treating asthma in a subject. The method according to this aspect of the invention involves the step of administering to a subject having or at risk of developing asthma an effective amount of a composition of the invention to treat the asthma. In one embodiment the asthma is allergic asthma.

A subject having asthma is a subject that has or is at risk of developing asthma. Asthma typically is episodic, active at some times and quiescent at other times. In one embodiment the asthma is active at the time of administration of the immunostimulatory composition of the invention.

A subject at risk of developing asthma includes those subjects that have been identified as having asthma but that do not have the active disease at the time of immunostimulatory nucleic acid treatment, as well as subjects that are considered to be at risk of developing asthma because of genetic or environmental factors.

The invention in one aspect provides a method for treating an infection in a subject. The method according to this embodiment involves the step of administering to a subject having or at risk of developing an infection an effective amount of a composition of the invention to treat the infection.

A subject having an infection is a subject that has been exposed to an infectious pathogen and has acute or chronic detectable levels of the pathogen in the body. The immunostimulatory nucleic acids can be used with an antigen to mount an antigen-specific systemic or mucosal immune response that is capable of reducing the level of or eradicating the infectious pathogen.

A subject at risk of developing an infection may be a subject that lives in or that is planning to travel to an area where a particular type of infectious agent is found. A subject at risk of developing an infection may be a subject that through lifestyle, circumstance, or medical procedures is exposed infectious organisms. Subjects at risk of developing infection also include general populations to which a medical agency recommends vaccination with a particular infectious organism antigen.

In one embodiment the infection is a viral infection. It is believed by the inventors that this method may be useful even in the treatment of a viral infection with a single-stranded minus-sense RNA virus, particularly if the effective amount of the composition of the invention is administered early in the viral infection. Without meaning to be bound to any particular theory or mechanism, it is the belief of the inventors that early administration of the composition of the invention will boost or accelerate an immune response effective against the virus, thereby treating the viral infection.

Examples of viruses that have been found in humans include, but are not limited to: *Retroviridae* (e.g., human immunodeficiency viruses, such as HIV-1 (also referred to as HTLV-III, LAV or HTLV-III/LAV, or HIV-III; and other isolates, such as HIV-LP; *Picornaviridae* (e.g., polio viruses, hepatitis A virus; enteroviruses, human Coxsackie viruses, rhinoviruses, echoviruses); *Calciviridae* (e.g., strains that cause gastroenteritis); *Togaviridae* (e.g., equine encephalitis viruses, rubella viruses); *Flaviviridae* (e.g., dengue viruses, encephalitis viruses, yellow fever viruses); *Coronaviridae* (e.g., coronaviruses); *Rhabdoviridae* (e.g., vesicular stomatitis viruses, rabies viruses); *Filoviridae* (e.g., ebola viruses); *Paramyxoviridae* (e.g., parainfluenza viruses, mumps virus, measles virus, respiratory syncytial virus); *Orthomyxoviridae* (e.g., influenza viruses); *Bunyaviridae* (e.g., Hantaan viruses, bunya viruses, phleboviruses and Nairo viruses); *Arenaviridae* (hemorrhagic fever viruses); *Reoviridae* (e.g., reoviruses, orbiviruses and rotaviruses); *Bornaviridae; Hepadnaviridae* (Hepatitis B virus); *Parvoviridae* (parvoviruses); *Papovaviridae* (papilloma viruses, polyoma viruses); *Adenoviridae* (most adenoviruses); *Herpesviridae* (herpes simplex virus (HSV) 1 and 2, varicella zoster virus, cytomegalovirus (CMV), herpes virus; *Poxviridae* (variola viruses, vaccinia viruses, pox viruses); and *Iridoviridae* (e.g., African swine fever virus); and unclassified viruses (e.g., the agent of delta hepatitis (thought to be a defective satellite of hepatitis B virus), Hepatitis C; Norwalk and related viruses, and astroviruses).

In another embodiment the infection is a bacterial infection. Bacteria include, but are not limited to, *Pasteurella* species, *Staphylococci* species, *Streptococcus* species, *Escherichia coli, Pseudomonas* species, and *Salmonella* species. Specific examples of infectious bacteria include but are not limited to, *Helicobacter pyloris, Borrelia burgdorferi, Legionella pneumophilia, Mycobacteria* sps (e.g., *M. tuberculosis, M. avium, M intracellulare, M. kansasii, M gordonae), Staphylococcus aureus, Neisseria gonorrhoeae, Neisseria meningitidis, Listeria monocytogenes, Streptococcus pyogenes* (Group A Streptococcus), *Streptococcus agalactiae* (Group B Streptococcus), *Streptococcus* (viridans group), *Streptococcus faecalis, Streptococcus bovis, Streptococcus* (anaerobic sps.), *Streptococcus pneumoniae,* pathogenic *Campylobacter sp., Enterococcus sp., Haemophilus influenzae, Bacillus anthracis, Corynebacterium diphtheriae, Corynebacterium sp., Erysipelothrix rhusiopathiae, Clostridium perfringens, Clostridium tetani, Enterobacter aerogenes, Klebsiella pneumoniae, Pasturella multocida, Bacteroides sp., Fusobacterium nucleatum, Streptobacillus moniliformis, Treponema pallidum, Treponema pertenue, Leptospira, Rickettsia,* and *Actinomyces israelii.*

In another embodiment the infection is a fungal infection. Fungi include yeasts and molds. Examples of fungi include without limitation *Aspergillus* spp including *Aspergillus fumigatus, Blastomyces dermatitidis, Candida* spp including *Candida albicans, Coccidioides immitis, Cryptococcus neoformans, Histoplasma capsulatum, Pneumocystis carinii, Rhizomucor* spp, and *Rhizopus* spp.

Other infectious organisms (i.e., protists) include *Plasmodium spp.* such as *Plasmodium falciparum, Plasmodium malariae, Plasmodium ovale,* and *Plasmodium* vivax and *Toxoplasma gondii.* Blood-borne and/or tissue parasites include *Plasmodium* spp., *Babesia microti, Babesia divergens, Chlamydia trachomatis, Leishmania tropica, Leishmania* spp., *Leishmania braziliensis, Leishmania donovani, Trypanosoma gambiense* and *Trypanosoma rhodesiense* (African sleeping sickness), *Trypanosoma cruzi* (Chagas' disease), and *Toxoplasma gondii.*

Other medically relevant microorganisms have been described extensively in the literature, e.g., see C.G.A Thomas, Medical Microbiology, Bailliere Tindall, Great Britain 1983, the entire contents of which is hereby incorporated by reference.

The invention in one aspect provides a method for treating cancer in a subject. The method according to this aspect of the invention involves the step of administering to a subject having or at risk of developing cancer an effective amount of a composition of the invention to treat the cancer.

A subject having a cancer is a subject that has detectable cancerous cells. The cancer may be a malignant or non-malignant cancer. Cancers or tumors include but are not limited to biliary tract cancer; brain cancer; breast cancer; cervical cancer; choriocarcinoma; colon cancer; endometrial cancer; esophageal cancer; gastric cancer; intraepithelial neoplasms; lymphomas; liver cancer; lung cancer (e.g., small cell and non-small cell); melanoma; neuroblastomas; oral cancer; ovarian cancer; pancreas cancer; prostate cancer; rectal cancer; renal cancer; sarcomas; skin cancer; testicular cancer; and thyroid cancer, as well as other carcinomas and sarcomas. In one embodiment the cancer is hairy cell leukemia, chronic myelogenous leukemia, cutaneous T-cell leukemia, multiple myeloma, follicular lymphoma, malignant melanoma, squamous cell carcinoma, renal cell carcinoma, prostate carcinoma, bladder cell carcinoma, or colon carcinoma.

A subject at risk of developing a cancer is one who is who has a high probability of developing cancer. These subjects include, for instance, subjects having a genetic abnormality, the presence of which has been or can be demonstrated to have a correlative relation to a higher likelihood of developing a cancer and subjects exposed to cancer-causing agents such as tobacco, asbestos, or other chemical toxins, or a subject who has previously been treated for cancer and is in apparent remission. When a subject at risk of developing a cancer is treated with an antigen specific for the type of cancer to which the subject is at risk of developing and an immunostimulatory nucleic acid, the subject may be able to kill the cancer cells as they develop. If a tumor begins to form in the subject, the subject will develop a specific immune response against the tumor antigen.

### Screening Methods

The invention in another aspect provides a method for screening for an antagonist of a TLR. The method according to this aspect of the invention involves the steps of contacting a reference cell expressing a TLR with an effective amount of a composition of the invention, in the absence of a candidate antagonist of the TLR, to measure a reference amount of signaling by the TLR; contacting a test cell expressing the TLR with an effective amount of the composition, in the presence of the candidate antagonist of the TLR, to measure a test amount of signaling by the TLR; and determining the candidate antagonist of the TLR is an antagonist of the TLR when the reference amount of signaling exceeds the test amount of signaling. The reference cell and the test cell may each express the TLR naturally or artificially, as described above. In one embodiment the reference cell and the test cell are each cells that are representative of a common population of cells, e.g., PBMC taken from a single donor, or 293HEK cells stably transfected with an expression vector for the TLR. In various specific embodiments the TLR may be chosen from TLR8, TLR7, or TLR3.

### Measuring Immunostimulatory Effects

The immunostimulatory effect of the immunostimulatory oligonucleotides of the invention can be measured using any suitable method, in vitro or in vivo. A basis for such measurement can involve a measurement of cell proliferation; intracellular signaling, specifically including but not limited to TLR signaling; expression of a soluble product, such as a cytokine, chemokine, or antibody; expression of a cell surface marker, such as a cluster of differentiation (CD) antigen; or functional activity, such as apoptosis and NK cell cytotoxicity. Methods for making such types of measurements are well known in the art and can include, without limitation, tritiated thymidine incorporation, enzyme-linked immunosorbent assay (ELISA), radioimmunosassay (RIA), bioassay, fluorescence-activated cell sorting, immunoblot (Western blot) assay, Northern blot assay, terminal deoxynucleotide transferase dUTP nick end labeling (TUNEL) assay, reverse transcriptase-polymerase chain reaction (RT-PCR) assay, and chromium release assay. The measurements may be quantitative or qualitative.

In certain embodiments measurements are made specifically for Thl-like immune response. Such measurements can include measurements of specific cytokines, chemokines, antibody isotypes, and cell activity that are associated with a Th1-like immune response, as described above.

In one embodiment measurements are made specifically for TLR signaling activity. Such measurements can be direct or indirect, and typically they involve measurement of expression or activity of a gene affected by some component of the intracellular signaling pathway mediated by a TLR.

Nucleotide and amino acid sequences of human and murine TLR8 are known. See, for example, GenBank Accession Nos. AF246971, AF245703, NM_016610, XM_045706, AY035890, NM_133212; and AAF64061, AAF78036, NP_057694, XP_045706, AAK62677, and NP_573475, the contents of all of which are incorporated in their entirety herein by reference. Human TLR8 is reported to exist in at least two isoforms, one 1041 amino acids long and the other 1059 amino acids long. Murine TLR8 is 1032 amino acids long. TLR8 polypeptides include an extracellular domain having a leucine-rich repeat region, a transmembrane domain, and an intracellular domain that includes a TIR domain.

Nucleotide and amino acid sequences of human and murine TLR7 are known. See, for example, GenBank Accession Nos. AF240467, AF245702, NM_016562, AF334942, NM_133211; and AAF60188, AAF78035, NP_057646, AAL73191, and AAL73192, the contents of all of which are incorporated in their entirety herein by reference. Human TLR7 is reported to be 1049 amino acids long. Murine TLR7 is reported to be 1050 amino acids long. TLR7 polypeptides include an extracellular domain having a leucine-rich repeat region, a transmembrane domain, and an intracellular domain that includes a TIR domain.

Nucleotide and amino acid sequences of human and murine TLR3 are known. See, for example, GenBank Accession Nos. NM_003256 and U88879 (human, cDNA); NP_003256 and AAC34134 (human, amino acid); NM_126166 and AF355152 (mouse, cDNA); and NP_569054 and AAK26117 (mouse, amino acid), the contents of all of which are incorporated in their entirety herein by reference. Human TLR3 is a 904 amino acid polypeptide characterized at least in part by an extracellular domain with leucine-rich repeats, a transmembrane domain, and an intracellular segment similar to the signaling domains of the family of interleukin-1-type receptors. Murine TLR3 is a 905 amino acid polypeptide characterized at least in part by an extracellular domain with leucine-rich repeats, a transmembrane domain, and an intracellular segment similar to the signaling domains of the family of interleukin-1-type receptors.

Signaling by TLR3, like signaling by other TLR family members, results in NF-κB activation. TLR3 signaling has recently been reported to be somewhat more complex than signaling by some other TLR family members. In particular, although TLR3 induces cytokine production through a signaling pathway dependent on MyD88, poly(I:C) can still induce activation of NF-κB and MAP kinases in MyD88-deficient macrophages, and, furthermore, TLR3-mediated activation of NF-KB and MAP kinases reportedly can occur through an IRAK-independent pathway employing the signaling components TLR3, TRAF6, TAK1, TAB2, and protein kinase RNA-regulated (PKR). Jiang Z et al. (2003) J Biol Chem 278:16713-9. It is to be noted that, despite some of the specific details of TLR3 signaling mechanisms, TLR3 signaling does result in NF-κB activation.

### Dosing and Administration

The immunostimulatory oligonucleotides of the invention can be used alone, in combination with themselves, in combination with another agent, or in combination with themselves and with another agent. In addition to the conjugates described herein, the immunostimulatory oligonucleotide in combination with another agent can also be separate compositions that are used together to achieve a desired effect. For example, an immunostimulatory oligonucleotide and a second agent can be mixed together and administered to a subject or placed in contact with a cell as a combination. As another example, an immunostimulatory oligonucleotide and a second agent can be administered to a subject or placed in contact with a cell at different times. As yet another example, an immunostimulatory oligonucleotide and a second agent can be administered to a subject at different sites of administration.

The immunostimulatory oligonucleotide and/or the antigen and/or other therapeutics may be administered alone (e.g., in saline or buffer) or using any delivery vehicle known in the art. For instance the following delivery vehicles have been described: cochleates (Gould-Fogerite et al., 1994, 1996); emulsomes (Vancott et al., 1998, Lowell et al., 1997); ISCOMs (Mowat et al., 1993, Carlsson et al., 1991, Hu et., 1998, Morein et al., 1999); liposomes (Childers et al., 1999, Michalek et al., 1989, 1992, de Haan 1995a, 1995b); live bacterial vectors (e.g., *Salmonella, Escherichia coli,* bacillus Calmette-Guérin, *Shigella, Lactobacillus)* (Hone et al., 1996, Pouwels et al., 1998, Chatfield et al., 1993, Stover et al., 1991, Nugent et al., 1998); live viral vectors (e.g., Vaccinia, adenovirus, Herpes simplex) (Gallichan et al., 1993, 1995, Moss et al 1996, Nugent et al., 1998, Flexner et al., 1988, Morrow et al., 1999); microspheres (Gupta et al., 1998, Jones et al., 1996, Maloy et al., 1994, Moore et al., 1995, O'Hagan et al., 1994, Eldridge et al., 1989); nucleic acid vaccines (Fynan et al., 1993, Kuklin et al., 1997, Sasaki et al., 1998, Okada et al., 1997, Ishii et al., 1997); polymers (e.g., carboxymethylcellulose, chitosan) (Hamajima et al., 1998, Jabbal-Gill et al., 1998); polymer rings (Wyatt et al., 1998); proteosomes (Vancott et al., 1998, Lowell et al., 1988, 1996, 1997); sodium fluoride (Hashi et al., 1998); transgenic plants (Tacket et al., 1998, Mason et al., 1998, Haq et al., 1995); Virosomes (Gluck et al., 1992, Mengiardi et al., 1995, Cryz et al., 1998); virus-like particles (Jiang et al., 1999, Leibl et al., 1998). Other delivery vehicles are known in the art.

As mentioned above, the term "effective amount" refers generally to an amount necessary or sufficient to bring about a desired biologic effect. Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned which does not cause substantial toxicity and yet is entirely effective to treat the particular subject. The effective amount for any particular application can vary depending on such factors as the disease or condition being treated, the particular oligonucleotide being administered, the size of the subject, or the severity of the disease or condition. One of ordinary skill in the art can empirically determine the effective amount of a particular immunostimulatory oligonucleotide and/or antigen and/or other therapeutic agent without necessitating undue experimentation.

Subject doses of the compounds described herein for systemic or local delivery typically range from about 10 ng to 10 mg per administration, which depending on the application could be given daily, weekly, or monthly and any other amount of time therebetween or as otherwise required. More typically systemic or local doses range from about 1 µg to 1 mg per administration, and most typically from about 10 µg to 100 µg, with 2 - 4 administrations being spaced days or weeks apart. Higher doses may be required for parenteral administration. In some embodiments, however, parenteral doses for these purposes may be used in a range of 5 to 10,000 times higher than the typical doses described above.

For any compound described herein the therapeutically effective amount can be initially determined from animal models. The applied dose can be adjusted based on the relative bioavailability and potency of the administered compound. Adjusting the dose to achieve maximal efficacy based on the methods described above and other methods as are well-known in the art is well within the capabilities of the ordinarily skilled artisan.

### Route of Administration

For clinical use the immunostimulatory oligonucleotide of the invention can be administered alone or formulated as a delivery complex via any suitable route of administration that is effective to achieve the desired therapeutic result. Routes of administration include enteral and parenteral routes of administration. Examples of enteral routes of administration include oral, gastric, intestinal, and rectal. Nonlimiting examples of parenteral routes of administration include intravenous, intramuscular, subcutaneous, intraperitoneal, intrathecal, local injection, topical, intranasal, mucosal, and pulmonary.

### Formulation

The immunostimulatory oligonucleotide of the invention may be directly administered to the subject or may be administered in conjunction with a nucleic acid delivery complex. A nucleic acid delivery complex shall mean a nucleic acid molecule associated with (e.g., ionically or covalently bound to; or encapsulated within) a targeting means (e.g., a molecule that results in higher affinity binding to target cell. Examples of nucleic acid delivery complexes include nucleic acids associated with a sterol (e.g., cholesterol), a lipid (e.g., a cationic lipid, virosome, virus-like particle (VLP), or liposome), or a target cell-specific binding agent (e.g., a ligand recognized by target cell-specific receptor). Preferred complexes may be sufficiently stable in vivo to prevent significant uncoupling prior to internalization by the target cell. However, the complex can be cleavable under appropriate conditions within the cell so that the oligonucleotide is released in a functional form.

For oral administration, the compounds (i.e., immunostimulatory oligonucleotide, antigens and/or other therapeutic agents) can be formulated readily by combining the active compound(s) with pharmaceutically acceptable carriers well known in the art. Such carriers enable the compounds of the invention to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers for neutralizing internal acid conditions or may be administered without any carriers.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner.

The compounds may be administered by inhalation to pulmonary tract, especially the bronchi and more particularly into the alveoli of the deep lung, using standard inhalation devices. The compounds may be delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide, or other suitable gas. In the case of a pressurized aerosol, the dosage unit may be determined by providing a valve to deliver a metered amount. An inhalation apparatus may be used to deliver the compounds to a subject. An inhalation apparatus, as used herein, is any device for administering an aerosol, such as dry powdered form of the compounds. This type of equipment is well known in the art and has been described in detail, such as that description found in Remington: The Science and Practice of Pharmacy, 19th Edition, 1995, Mac Publishing Company, Easton, Pennsylvania, pages 1676-1692. Many U.S. patents also describe inhalation devices, such as U.S. Pat. No. 6,116,237.

"Powder" as used herein refers to a composition that consists of finely dispersed solid particles. Preferably the compounds are relatively free flowing and capable of being dispersed in an inhalation device and subsequently inhaled by a subject so that the compounds reach the lungs to permit penetration into the alveoli. A "dry powder" refers to a powder composition that has a moisture content such that the particles are readily dispersible in an inhalation device to form an aerosol. The moisture content is generally below about 10% by weight (% w) water, and in some embodiments is below about 5% w and preferably less than about 3% w. The powder may be formulated with polymers or optionally may be formulated with other materials such as liposomes, albumin and/or other carriers.

Aerosol dosage and delivery systems may be selected for a particular therapeutic application by one of skill in the art, such as described, for example in Gonda, I. "Aerosols for delivery of therapeutic and diagnostic agents to the respiratory tract," in Critical Reviews in Therapeutic Drug Carrier Systems, 6:273-313 (1990), and in Moren, "Aerosol dosage forms and formulations," in Aerosols in Medicine. Principles, Diagnosis and Therapy, Moren, et al., Eds., Elsevier, Amsterdam, 1985.

The compounds, when it is desirable to deliver them systemically, may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active compounds may be in powder form for constitution with a suitable vehicle, e.g., sterile pyrogen-free water, before use.

The compounds may also be formulated in rectal or vaginal compositions such as suppositories or retention enemas, e.g., containing conventional suppository bases such as cocoa butter or other glycerides.

In addition to the formulations described previously, the compounds may also be formulated as a depot preparation. Such long acting formulations may be formulated with suitable polymeric or hydrophobic materials (for example as an emulsion in an acceptable oil) or ion exchange resins, or as sparingly soluble derivatives, for example, as a sparingly soluble salt.

The pharmaceutical compositions also may include suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer R (1990) Science 249:1527-33, which is incorporated herein by reference.

The immunostimulatory oligonucleotides and optionally other therapeutics and/or antigens may be administered *per se* (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

The pharmaceutical compositions of the invention contain an effective amount of an immunostimulatory oligonucleotide and optionally antigens and/or other therapeutic agents optionally included in a pharmaceutically acceptable carrier. The term pharmaceutically acceptable carrier means one or more compatible solid or liquid filler, diluents or encapsulating substances which are suitable for administration to a human or other vertebrate animal. The term carrier denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

The present invention is further illustrated by the following Examples, which in no way should be construed as further limiting.

### EXAMPLES

### Example 1

### In Vitro Methods for Detecting TLR Signaling

Analysis was performed for immune stimulation in an NF-κB-luciferase readout on HEK293 cells stably transfected with a human TLR and an NF-κB-luciferase reporter construct (hTLR3-NFκB-293). Briefly, cells were contacted with immunostimulatory oligonucleotide or other test or control agent for a defined period, typically 16 hours, and then analyzed with a luminometer. Emitted light varied in direct proportion to NF-κB activation.

Analysis for immune stimulation was also performed in human peripheral blood monocytic cell (PBMC) assays for TNF-α, IFN-α, and IL-12 p40 cytokine production. Briefly, PBMC obtained from healthy human donors were contacted with immunostimulatory oligonucleotide or other test or control agent for a defined period, typically 16 hours, and then cell supernatants were analyzed for cytokine using a suitable cytokine-specific enzyme-linked immunosorbent assay (ELISA).

The effect of immunostimulatory oligonucleotide was assessed by titrating the amount or concentration of oligonucleotide concentration in a given experiment. The effect of immunostimulatory oligonucleotide concentration was expressed in terms of EC50 (concentration at which immunostimulatory oligonucleotide was 50 percent effective compared to maximum effect). The potency of a given immunostimulatory oligonucleotide was expressed as maximum stimulation index (SI max; the maximum fold increase in signal over that of untreated control) or maximum activity.

### Example 2

### Orthomyxoviridae

Following are listed 3' termini (terminal 26-mers, shown 5' to 3', reading from left to right) of the following viruses belonging to the family *Orthomyxoviridae.* Dashes (-) indicate sequence identity at the indicated position with Influenza A PR/8/34 (Cambridge) H1N1 PB2.

### Influenza A PR/8/34 (Cambridge) H1N1

| | | | |
|---|---|---|---|
| PB2 | 1 | UUGAAUAUAAUUGACCUGCUUUCGCU | (SEQ ID NO:297) |
| PB1 | 2 | A-UC-A--GG--UG------------ | (SEQ ID NO:298) |
| PA | 3 | A-UUUGGAUCAGU------------- | (SEQ ID NO:299) |
| HA | 4 | -GUUU-UAUU--CC--------U--- | (SEQ ID NO:300) |
| NP | 5 | GA-UGAU--UC-AC--------U--- | (SEQ ID NO:301) |
| NA | 6 | A-UC--U-U-AACC------------ | (SEQ ID NO:302) |
| M1, M2 | 7 | -CUUUC-AU--CU------------- | (SEQ ID NO:303) |
| NS1 NS2 | 8 | -AUGUCU-UG-CAC--------U--- | (SEQ ID NO:304) |

### Influenza A Hong Kong/ 156/97 HSN1

| | | | |
|---|---|---|---|
| NS1 NS2 | 8 | -AUGUCU-UG-CAC--------U--- | (SEQ ID NO:304) |

### Influenza A duck/Australia/341/83 H15N8

| | | | |
|---|---|---|---|
| HA | 4 | --C-U-U-GUA-CC--------U--- | (SEQ ID NO:305) |

### Influenza B Ann Arbor/1/66 (segments 4 and 6 from Lee/40)

| | | | |
|---|---|---|---|
| PB1 | 1 | --C-UCU---AG-CU-C----CU--- | (SEQ ID NO:306) |
| PB2 | 2 | CAUCU-GA--AC-CU-C----CU--- | (SEQ ID NO:307) |
| PA | 3 | GGC--AUC--AC-CA-C----CU--- | (SEQ ID NO:308) |
| HA | 4 | A-AUUAGA--A--CAAC----CU--- | (SEQ ID NO:309) |
| NP | 5 | -GAG-AGA--A--CUG-----CU--- | (SEQ ID NO:310) |
| NB NA | 6 | --CU-AGA-UA--CU------CU--- | (SEQ ID NO:311) |
| M1 | 7 | A-UUUA-G--AGUG-G-----CU--- | (SEQ ID NO:312) |
| NS1 NS2 | 8 | GACU-A-C--A--CU------CU--- | (SEQ ID NO:313) |

### Influenza C JJ/50 (segment 4 from Johannesburg/66; segment 7 from California/78)

| | | |
|---|---|---|
| 1 | GAC-U-UCC-A-CC-------CU--- | (SEQ ID NO:314) |
| 2 | GAUUUCCAU-A-CC-------CU--- | (SEQ ID NO:315) |
| 3 | GAC-U-UCGGA-CC-------CU--- | (SEQ ID NO:316) |
| 4 | AAC-U---U-ACCC-------CU--- | (SEQ ID NO:317) |
| 6 | ---UU--G--A-CC-------CU--- | (SEQ ID NO:318) |
| 7 | --UGGA-A-G-ACC-------CU--- | (SEQ ID NO:319) |

### Thogoto SiAr 126

| | | | |
|---|---|---|---|
| PB2 | 1 | --CUC-G-CCA-CG-U--U---U--- | (SEQ ID NO:320) |
| PB1 | 2 | ---UG-U-GGAGCG----U---U--- | (SEQ ID NO:321) |
| PA | 3 | -GUC--G-C-AGUG-U--U---U--- | (SEQ ID NO:322) |
| gp75 | 4 | -GA-GG-AC--CUG-U--U---U--- | (SEQ ID NO:323) |
| NP | 5 | AGCC--U-UGACUG----U---U--- | (SEQ ID NO:324) |

### Example 3

### Paramyxoviridae

Following are listed 3' termini (terminal 20-mers, shown 5' to 3', reading from left to right) of the following viruses belonging to the family *Paramyxoviridae.* Dashes (-) indicate sequence identity at the indicated position with Sendai virus.

| | | |
|---|---|---|
| Sendai | UGUUUUUUCUCUUGUUUGGU | (SEQ ID NO:4) |
| HPIV3 | A----C-------------- | (SEQ ID NO:143) |
| measles | CU-ACCCAACU----- | (SEQ ID NO:3) |
| Hendra | CA-A---C-C------C--- | (SEQ ID NO:84) |
| RSV | -UG-ACGCA-U--U-CGC-- | (SEQ ID NO:2) |

### Example 4

### Filoviridae

Following are listed 3' termini (terminal 24-mers, shown 5' to 3', reading from left to right) of the following viruses belonging to the family *Filoviridae.* Dashes (-) indicate sequence identity at the indicated position with Marburg virus.

| | | |
|---|---|---|
| Marburg | AUCAUCUCUUGUUUUUGUGUGUCU | (SEQ ID NO:325) |
| Ebola | --UC-UCU--C-----CG | (SEQ ID NO:326) |

### Example 5

### Human PBMC Secrete Cytokines in Response to Stabilized ORN Representative of 3' Termini of Genomic RNA of Single-Stranded Minus-Sense RNA Viruses

The hypothesis as to whether the 3' genomic RNA of single-stranded negative-strand RNA viruses was immune stimulatory was tested. 20-mer oligoribonucleotides (ORN) from five example viruses were selected and chemically synthesized with a phosphorothioate backbone. As a control sequence, to demonstrate sequence specificity, the complementary strand of the Influenza A sequence was used (ORN 5). The test sequences were dose titrated into human PBMC in vitro cultures and the cytokine release pattern was monitored. The dose titration results were analyzed to give EC50 and maximal cytokine release values. All test sequences were immune stimulating while the control sequence was not. The data demonstrate that the 3' genomic RNA of single-stranded negative-strand RNA viruses is immune stimulatory in a sequence-dependent fashion.

Peripheral blood buffy coat preparations from healthy male and female human donors were obtained from the Blood Bank of the University of Düsseldorf (Germany) and from these, PBMC were purified by centrifugation over Ficoll-Hypaque (Sigma). The purified PBMC were used fresh in every assay and therefore resuspended in RPMI 1640 culture medium supplemented with 5% (v/v) heat-inactivated human AB serum (BioWhittaker, Belgium) or 10% (v/v) heat-inactivated fetal calf serum (FCS), 1.5 mM L-glutamine, 100 U/ml penicillin and 100 µg/ml streptomycin (all from Sigma).

Fresh PBMC were resuspended at a concentration of 3x1⁰6/ml to 5x10⁶/ml and added to 96-well round-bottomed plates (150 µl/well). After cell plating, oligoribonucleotides (ORN) plus DOTAP were added at different concentrations, using a three-fold serial dilution. The starting concentration for DOTAP was 50 µg/ml and for ORN 5 µM. The cells were cultured in a humidified incubator at 37°C. Culture supernatants were collected after 16h and, if not used immediately, frozen at -20°C until required.

Quantitative analysis of cytokines in the supernatants was assessed using commercially available enzyme-linked immunosorbent assay (ELISA) kits (IFN-γ or TNF-α; Diaclone, USA, IL-12 p40; Pharmingen) or proprietary ELISA (IFN-α) developed using commercially available antibodies (from Becton Dickinson/Pharmingen or PBL; Germany or USA, respectively). Representative results are presented in Tables 2-4.

**Table 1: Sequence list**

| RNA | Sequence 5' to 3' | SEQ ID NO: | Source |
|---|---|---|---|
| ORN 1 | UUGUACGCAUUUUUUCGCGU | 2 | Respiratory syncytial virus GenBank Accession No. U39661 |
| ORN 2 | CUUACCCAACUUUGUUUGGU | 3 | Measles virus GenBank Accession No. Z66517 |
| ORN 3 | UGUUUUUUCUCUUGUUUGGU | 4 | Sendai virus GenBank Accession No. X00087 |
| ORN 4 | AUAAUUGACCUGCUUUCGCU | 5 | Influenza A virus GenBank Accession No. AF389115 |
| ORN 5 | AGCGAAAGCAGGUCAAUUAU | 327 | Control complementary strand |
| ORN 6 | UUGAUCUGGUUGUUAAGCGU | 6 | Rabies virus GenBank Accession No. M 13215 |
| ORN 7 | AAUGGUUUGUUUGUCUUCGU | 7 | Vesicular stomatitis virus Indiana GenBank Accession No. J02428 |

**Table 2: TNF production: four individual Donors (EC50 in µM and max activity in pg/ml)**

| RNA | Donor A | | Donor B | | Donor C | | Donor D | | Mean | |
|---|---|---|---|---|---|---|---|---|---|---|
| | EC50 | max | EC50 | max | EC50 | max | EC50 | max | EC50 | max |
| ORN 1 | 0.494 | 8000 | 0.417 | 8000 | 0.473 | 10000 | 0.529 | 8000 | 0.478 | 8500 |
| ORN 2 | 0.336 | 8000 | 0.267 | 13000 | 0.538 | 10000 | 0.500 | 7000 | 0.410 | 9500 |
| ORN 3 | 0.419 | 15000 | 0.421 | 10000 | 0.470 | 9000 | 0.337 | 5000 | 0.412 | 9750 |
| ORN 4 | 0.412 | 8000 | 0.400 | 5000 | 0.559 | 5000 | 0.512 | 5000 | 0.471 | 5750 |
| ORN 5 | -- | 0 | -- | 0 | -- | 0 | -- | 0 | -- | 0 |
| ORN 6 | 0.462 | 10000 | 0.409 | 7000 | 0.604 | 10000 | 0.498 | 8000 | 0.493 | 8750 |
| ORN 7 | 0.357 | 10000 | 0.325 | 7000 | 0.544 | 8000 | 0.468 | 5000 | 0.424 | 7500 |

**Table 3: IFN-α production: 4 individual Donors (EC50 in µM and max activity in pg/ml)**

| RNA | Donor A | | Donor B | | Donor C | | Donor D | | Mean | |
|---|---|---|---|---|---|---|---|---|---|---|
| | EC50 | max | EC50 | max | EC50 | max | EC50 | max | EC50 | max |
| ORN 1 | 0.051 | 6000 | 0.063 | 4000 | 0.150 | 600 | 0.039 | 700 | 0.074 | 2825 |
| ORN 2 | 0.034 | 6000 | 0.068 | 4000 | 0.060 | 1000 | 0.038 | 2500 | 0.050 | 3375 |
| ORN 3 | 0.026 | 5500 | 0.017 | 4000 | 0.089 | 600 | 0.021 | 1500 | 0.039 | 2900 |
| ORN 4 | 0.092 | 2000 | 0.459 | 2000 | -- | 0 | -- | 0 | 0.276 | 2000 |
| ORN 5 | -- | 0 | -- | 0 | -- | 0 | -- | 0 | -- | 0 |
| ORN 6 | 0.045 | 4000 | 0.099 | 9000 | 0.097 | 800 | 0.086 | 2500 | 0.082 | 4075 |
| ORN 7 | 0.016 | 4000 | 0.044 | 10000 | 0.051 | 1400 | 0.019 | 3000 | 0.033 | 4600 |

**Table 4: IFN-γ production: two individual Donors (EC50 in µM and max activity in pg/ml)**

| RNA | Donor A | | Donor B | | Mean | |
|---|---|---|---|---|---|---|
| | EC50 | max | EC50 | max | EC50 | max |
| ORN 1 | 0.592 | 50000 | 0.517 | 60000 | 0.555 | 55000 |
| ORN 2 | 0.514 | 50000 | 0.529 | 40000 | 0.522 | 45000 |
| ORN 3 | 0.528 | 60000 | 0.606 | 70000 | 0.567 | 65000 |
| ORN 4 | 0.637 | 25000 | 0.490 | 60000 | 0.564 | 42500 |
| ORN 5 | -- | 0 | -- | 0 | -- | 0 |
| ORN 6 | 0.635 | 60000 | 0.586 | 30000 | 0.611 | 45000 |
| ORN 7 | 0.490 | 50000 | 0.642 | 80000 | 0.566 | 65000 |

### Example 6

### Proof of Principle for 4-mer Motif

This example demonstrates the immunostimulatory potential of the proposed 4-mer motif 5'-C/U-U-G/U-U-3'. For proof of principle, a 4-mer RNA motif selected from UUGU or UUUU was nested within a phosphorothioate poly-N composition, where each N independently is any base A, G, U, or C. The backbone consisted of either an RNA backbone or a chimeric RNA:DNA backbone, wherein N denotes RNA and dN denotes DNA. Oligonucleotides included the following:

| | | |
|---|---|---|
| ORN 8 | dNdNdNdNdNN**UUUU**NNdNdNdNdNdNdN | SEQ ID NO:328 |
| ORN 9 | dNdNdNdNdNN**UUGU**NNdNdNdNdNdNdN | SEQ ID NO:329 |
| ORN 10 | NNNNNN**UUUU**NNNNNNNN | SEQ ID NO:330 |
| ORN 11 | NNNNNN**UUGU**NNNNNNNN | SEQ ID NO:331 |

Human PBMCs were stimulated and assayed for cytokine production similar to as described in Example 5. Representative results are presented in Tables 5-7.

**Table 5: TNF production: four individual Donors (EC50 in µM and max activity in pg/ml)**

| oligo | Donor | EC50 | max | Donor | EC50 | max |
|---|---|---|---|---|---|---|
| ORN 8 | A | 0.741 | 35000 | C | 0.306 | 20000 |
| ORN 9 | A | 0.632 | 35000 | C | 0.095 | 25000 |
| ORN 10 | B | 0.068 | 60000 | D | 0.053 | 60000 |
| ORN 11 | B | 0.054 | 80000 | D | 0.008 | 40000 |

**Table 6: IFN-α production: four individual Donors (EC50 in µM and max activity in pg/ml)**

| oligo | Donor | EC50 | max | Donor | EC50 | max |
|---|---|---|---|---|---|---|
| ORN 8 | A | 0.511 | 4500 | C | 0.605 | 5000 |
| 9 | A | 0.134 | 2000 | C | 0.101 | 1000 |
| ORN 10 | B | 0.300 | 3000 | D | 0.041 | 2500 |
| ORN 11 | B | 0.031 | 2500 | D | 0.040 | 3000 |

**Table 7: IFN-γ production: four individual Donors (EC50 in µM and max activity in pg/ml)**

| oligo | Donor | EC50 | max | Donor | EC50 | max |
|---|---|---|---|---|---|---|
| ORN 8 | A | 1.557 | 30000 | C | -- | 0 |
| ORN 9 | A | 0.686 | 70000 | C | -- | 0 |
| ORN 10 | B | 0.040 | 12000 | D | 0.131 | 18000 |
| ORN 11 | B | 0.038 | 12000 | D | 0.032 | 12000 |

### Example 7

### Cytokine Induction by Chimeric RNA: DNA Oligonucleotides

This example describes the ability of certain chimeric RNA:DNA oligonucleotides of the invention to stimulate cytokine secretion by human PBMC. Cytokine induction and detection were performed as described in Example 5, using as oligonucleotides the following chimeric RNA:DNA oligonucleotides, wherein dT, dC, dG, and dA denote deoxyribonucleotides and G and U denote ribonucleotides:

| | | |
|---|---|---|
| ORN 12 | dTdCdGdTdCdGdTdTdTGUUGUGUdAdAdT | (SEQ ID NO:332) |
| ORN 13 | dTdCdGdTdCdGdTdTdT 2'-5' (GUUGUGU) dAdAdT | (SEQ ID NO:333) |

ORN 12 and ORN 13 both have phosphorothioate backbones. In addition, ORN 12 has exclusively 3'-5' internucleotide linkages, whereas ORN 13 has 2'-5' internucleotide linkages interconnecting GUUGUGUdA. Representative results are provided in Tables 8-10.

**Table 8: TNF production: one Donor per ORN (EC50 in µM and max activity in pg/ml)**

| oligo | Donor | EC50 | max |
|---|---|---|---|
| ORN 12 | A | 0.087 | 20000 |
| ORN 13 | A | -- | 0 |

**Table 9: IFN-α production: two individual Donors (EC50 in µM and max activity in pg/ml)**

| oligo | Donor A | | Donor B | |
|---|---|---|---|---|
| | EC50 | max | EC50 | max |
| ORN 12 | 0.008 | 4500 | 0.009 | 2000 |
| ORN 13 | 0.015 | 4500 | 0.025 | 1200 |

**Table 10: IFN-γ production: two individual Donors (EC50 in µM and max activity in µg/ml)**

| oligo | Donor A | | Donor B | |
|---|---|---|---|---|
| | EC50 | max | EC50 | max |
| ORN 12 | 0.191 | 25000 | 0.109 | 40000 |
| ORN 13 | -- | 0 | -- | 0 |

### Example 8

### TLR Stimulation by Chimeric RNA: DNA Oligonucleotides

This example demonstrates combined stimulation of TLR8 and TLR9 by a chimeric RNA:DNA conjugate oligonucleotide with a phosphorothioate backbone having exclusively 3'-5' internucleotide linkages. Stimulation and measurement of signal transduction in HEK293 cells stably transfected with either human TLR8 or human TLR9 and an NF-κB-luciferase reporter construct was performed essentially as described in Example 1. Chimeric RNA:DNA oligonucleotides were as provided in Example 7. Results are provided in Table 11. Results are expressed in terms of EC50 in µM and stimulation index (SI) = fold induction of NF-κB-luciferase activity in ligand-pulsed cells.

**Table 11: TLR stimulation by chimeric RNA:DNA oligonucleotides**

| oligo | TLR8 | | TLR9 | |
|---|---|---|---|---|
| | EC50 | SI | EC50 | SI |
| ORN 12 | 0.508 | 3 | 0.078 | 17 |
| ORN 13 | -- | 0 | | 20 |

The chimeric RNA:DNA oligonucleotide ORN 12 effectively acted through TLR8 and TLR9. However, when the inter-ribonucleotide linkages in the ORN were changed to 2'-5' (ORN 13), TLR8 activity was lost but TLR9 activity was maintained. This result demonstrates that the chimeric by virtue of having two TLR motifs, one for TLR8 and one for TLR9, is able to stimulate the respective receptor specifically.

### Example 9

### Conversion of CpG Oligodeoxynucleotides into RNA-Containing Oligonucleotides with New Immunostimulatory Profile

This example demonstrates that CpG oligodeoxynucleotides, which have an immunostimulatory profile reflective of their ability to stimulate TLR9, can be modified, by substitution of certain deoxynucleotides by certain ribonucleotides, to have new and additional immunostimulatory properties, believed to be reflective of their ability to stimulate TLR7 and/or TLR8. Also as shown in this example, even very well characterized CpG oligonucleotides can be modified in this manner.

Sequences of CpG ODN 2006 (5'-tcgtcgtmgtcgtmgtcgtt-3', SEQ ID NO:285), ODN 10101 (5'-tcgtcgttttcggcggccgccg-3', SEQ ID NO:288), and ODN 8954 (5'-ggggatgatgttgtggggggg-3', SEQ ID NO: ) were taken as starting points. These CpG ODN were remade as ORN by substituting U for T, U for C, or U for both T and C. Resulting ORN corresponding to ODN 2006, 10101, and 8954 were ORN 14-16, ORN 17-18, and ORN 19-20.

| | | |
|---|---|---|
| ORN 14 | UCGUCGUUUUGUCGUUUUGUCGUU | SEQ ID NO:334 |
| ORN 15 | UUGUUGUUUUGUUGUUUUGUUGUU | SEQ ID NO:286 |
| ORN 16 | TUGTUGTTTTGTUGTTTTGTUGTT | SEQ ID NO:287 |
| ORN 17 | UUGUUGUUUUUGGUGGUUGUUG | SEQ ID NO:289 |
| ORN 18 | TUGTUGTTTTUGGUGGUUGUUG | SEQ ID NO:290 |
| ORN 19 | GGGGAUGAUGUUGUGGGGGGG | SEQ ID NO:335 |
| ORN 20 | GGGGAUGAUGTUGTGGGGGGG | SEQ ID NO:336 |

Human PBMC's were stimulated and assayed for cytokine production similar to as described in Example 5. Representative results are presented in Tables 12 and 13.

**Table 12: TNF production (EC50 in µM and max activity in pg/ml)**

| oligo | EC50 | max |
|---|---|---|
| ORN 14 | 0.0780 | 53833 |
| ORN 15 | 0.0400 | 60000 |
| ORN 16 | 0.2338 | 56667 |
| ORN 17 | 0.0575 | 70000 |
| ORN 18 | 0.1998 | 61667 |
| ORN 19 | 0.4153 | 37667 |
| ORN 20 | -- | 0 |

**Table 13: IFN-α production (EC50 in µM and max activity in pg/ml)**

| oligo | EC50 | max |
|---|---|---|
| ORN 14 | 0.0373 | 2783 |
| ORN 15 | 0.0050 | 2500 |
| ORN 16 | 1.0992 | 2267 |
| ORN 17 | 0.0152 | 3000 |
| ORN 18 | 0.0795 | 2617 |
| ORN 19 | 0.1287 | 2867 |
| ORN 20 | -- | 0 |

### Example 10

### Further Proof of Principle for 4-mer Motif 5'-C/U-U-G/U-U/3'

This example extends observations in Example 6 by showing that a 4-mer RNA motif selected from UUGU and UUUU can be used to convert a non-immunostimulatory ORN to become an immunostimulatory ORN. ORN 21, which exhibits no immunostimulatory activity, was taken as the starting point. Related ORN bearing UUGU (ORN 22) or UUUU (ORN 23) were then synthesized and used in assays to assess their immunostimulatory activity.

| | | |
|---|---|---|
| ORN 21 | GCCACCGAGCCGA**AGGC**ACC | SEQ ID NO:337 |
| ORN 22 | GCCACCGAGCCGA**UUGU**ACC | SEQ ID NO:338 |
| ORN 23 | GCCACCGAGCCGA**UUUU**ACC | SEQ ID NO:339 |

Human PBMC's were stimulated and assayed for cytokine production similar to as described in Example 5. Representative results are presented in Tables 14 and 15.

**Table 14: TNF production (EC50 in µM and max activity in pg/ml)**

| oligo | EC50 | max |
|---|---|---|
| ORN 21 | -- | 0 |
| ORN 22 | 0.244 | 23833 |
| ORN 23 | 0.228 | 25500 |

**Table 15: IFN-α production (EC50 in µM and max activity in µg/ml)**

| oligo | EC50 | max |
|---|---|---|
| ORN 21 | -- | 0 |
| ORN 22 | 1.107 | 2417 |
| ORN 23 | 0.041 | 2333 |

### Example 11

### Further Proof of Principle for 4-mer Motif 5'-C/U- U-G/UU-3'

This example further extends observations in Examples 6 and 11 by showing that reduction of the 4-mer RNA motif to a 3-mer results in dramatic loss in immunostimulatory activity. Beginning with the RNA sequence GUUGUGU embedded in a random-sequence deoxynucleotide context, ribonucleosides were successively exchanged for dN deoxynucleosides. As can be seen from the data below, the 4-mer motif UUGU was active in this experiment while the 3-mer UUG and the 2-mer UG were not.

| | | |
|---|---|---|
| ORN 30 | dNdNdNdNdN**GUUGUGU**dNdNdNdNdNdN | SEQ ID NO:340 |
| ORN 31 | dNdNdNdNdNdN**UUGUG**dNdNdNdNdNdNdN | SEQ ID NO:341 |
| ORN 32 | dNdNdNdNdNdN**UUGU**dNdNdNdNdNdNdNdN | SEQ ID NO:342 |
| ORN 33 | dNdNdNdNdNdN**UUG**dNdNdNdNdNdNdNdNdN | SEQ ID NO:343 |
| ORN 34 | dNdNdNdNdNdNdN**UG**dNdNdNdNdNdNdNdNdN | SEQ ID NO:344 |

Human PBMC's were stimulated and assayed for cytokine production similar to as described in Example 5. Representative results are presented in Tables 16 and 17.

**Table 16: TNF production (EC50 in µM and max activity in pg/ml)**

| oligo | EC50 | max |
|---|---|---|
| ORN 30 | 0.466 | 30000 |
| ORN 31 | 4.893 | 18000 |
| ORN 32 | 7.977 | 30000 |
| ORN 33 | -- | 0 |
| ORN 34 | -- | 0 |

**Table 17: IFN-α production (EC50 in µM and max activity in pg/ml)**

| oligo | EC50 | max |
|---|---|---|
| ORN 30 | 3.422 | 875 |
| ORN 31 | 3.873 | 766.7 |
| ORN 32 | 4.556 | 666.7 |
| ORN 33 | -- | 0 |
| ORN 34 | -- | 0 |

### Example 12

### Viral-Derived ORN Stimulate a Broad Pattern of Cytokines from Human PBMC

Human PBMC were stimulated with 2.5 µM ORN plus DOTAP, DOTAP alone, 2.5 µM R-848, 25 ng/ml LPS, or 0.5µM CpG ODN 2395 (5'-TCGTCGTTTTCGGCGCGCGCCG-3', SEQ ID NO:343). ORN used in this experiment include ORN 4 and ORN 5 (Example 5, supra). Supernatants were harvested after 16h of stimulation and used for different ELISA. Results are shown in **FIG. 1**. As shown in this figure, the profile of immunostimulation by ORN 4, derived from influenza virus, was very broad, including induction of TNF-α, IL-6, IL-12 p40, IFN-α, and IFN-γ, and was distinct from the profile characteristic of CpG ODN 2395.

### Example 13

### Viral-Derived ORN Stimulate Cytokine Production In Vivo

Mice were injected with the 50 µg of ORN 21 (see Example 10), ORN 3 (see Example 5), or ORN 35 (5'-CCGUCUGUUGUGUGACUC-3', SEQ ID NO:344), each ORN combined with 100 µg DOTAP, or DOTAP alone. The mice were bled at 1h or 3h following injection, and separate ELISAs specific for IL-12 and IP-10 were performed. Results are presented in **FIG. 2** (IL-12) and **FIG. 3** (IP-10). The presence of cytokine induction demonstrated immune stimulation by the ORN in a sequence-dependent manner. Additionally, it was demonstrated that the ORN can be useful in immunomodulatory formulations directed toward disease. The response of IL-12 correlates with the potential for Th1 induction. The response of IP-10 is a surrogate marker for type 1 IFN which correlates with the potential for Th 1 induction.

### Example 14

### Human CD14+ Cells Up-Regulate CD80 Upon Stimulation with Viral-Derived ORN

This example demonstrates that human CD14+ cells (monocytes, myeloid linage cells) up-regulate the co-stimulatory molecule CD80 upon stimulation with viral-derived ORN. Human PBMC were stimulated with varied concentrations, ranging from 1 nM to 10 µM, of ORN (ORN 3, ORN 4, or ORN 5; see Example 5) mixed with DOTAP, or R-848, CpG ODN 2395, DOTAP alone, or media alone. After 16h cells were stained for CD19, CD14, and CD80 and then FACS analyzed. The cells were gated for CD14+ staining and the level of CD80 surface staining is shown in **FIG. 4**. This figure demonstrates that ORN3 and ORN4, as well as R-848, induce co-stimulatory molecule CD80 on the surface of CD 14+ cells, in a sequence dependent manner.

### Example 15

### Human CD19+ Cells Up-Regulate CD80 Upon Stimulation with Viral-Derived ORN

This example demonstrates that human CD19+ cells (B cells) up-regulate the co-stimulatory molecule CD80 upon stimulation with viral-derived ORN. Human PBMC were stimulated with varied concentrations, ranging from 1 nM to 10 µM, of ORN (ORN 3, ORN 4, or ORN 5; see Example 5) mixed with DOTAP, or R-848, CpG ODN 2395, DOTAP alone, or media alone. After 16h cells were stained for CD19, CD14, and CD80 and then FACS analyzed. The cells were gated for CD14+ staining and the level of CD80 surface staining is shown in **FIG. 5****.** This figure demonstrates that ORN3 and ORN4, as well as CpG ODN 2395 and R-848, induce co-stimulatory molecule CD80 on the surface of CD14+ cells, in a sequence dependent manner.

### EQUIVALENTS

The foregoing written specification is considered to be sufficient to enable one skilled in the art to practice the invention. The present invention is not to be limited in scope by examples provided, since the examples are intended as a single illustration of one aspect of the invention and other functionally equivalent embodiments are within the scope of the invention. Various modifications of the invention in addition to those shown and described herein will become apparent to those skilled in the art from the foregoing description and fall within the scope of the appended claims. The advantages of the invention are not necessarily encompassed by each embodiment of the invention.

All references, patents and patent publications that are recited in this application are incorporated in their entirety herein by reference.

### ASPECTS OF THE INVENTION

According to a first aspect, the present invention provides an immunostimulatory composition comprising an isolated nucleic acid molecule 10 to 30 nucleotides long comprising a sequence provided by a 3' end of a single-stranded minus-sense RNA virus genome, wherein the nucleic acid molecule has a stabilized backbone.

According to a second aspect, the present invention provides the immunostimulatory composition of the first aspect, wherein the nucleic acid molecule is 10 to 20 nucleotides long.

According to a third aspect, the present invention provides the immunostimulatory composition of the first aspect, wherein the nucleic acid molecule is 10 nucleotides long.

According to a fourth aspect, the present invention provides the immunostimulatory composition of the first aspect, wherein the sequence provided by the 3' end of a single-stranded minus-sense RNA virus genome comprises a sequence motif 5'-C/U-U-G/U-U-3', wherein U is uracil (U) oxyribonucleoside, C/U is cytosine (C) oxyribonucleoside or uracil (U) oxyribonucleoside, and G/U is guanine (G) oxyribonucleoside or uracil (U) oxyribonucleoside.

According to a fifth aspect, the present invention provides the immunostimulatory composition of the fourth aspect, wherein the sequence motif is 5'-CUGU-3'.

According to a sixth aspect, the present invention provides the immunostimulatory composition of the fourth aspect, wherein the sequence motif is 5'-UUGU-3'.

According to a seventh aspect, the present invention provides the immunostimulatory composition of the fourth aspect, wherein the sequence motif is 5'-CUUU-3'.

According to a eighth aspect, the present invention provides the immunostimulatory composition of the fourth aspect, wherein the sequence motif is 5'-UUUU-3'.

According to a ninth aspect, the present invention provides the immunostimulatory composition of the first aspect, wherein the stabilized backbone comprises at least one phosphorothioate internucleoside linkage.

According to a tenth aspect, the present invention provides the immunostimulatory composition of the first aspect, wherein the stabilized backbone is a phosphorothioate backbone.

According to an eleventh aspect, the present invention provides the immunostimulatory composition of the first aspect, wherein the stabilized backbone comprises at least one pyrophosphate internucleoside linkage.

According to a twelfth aspect, the present invention provides the immunostimulatory composition of the first aspect, wherein the stabilized backbone is a pyrophosphate backbone.

According to a thirteenth aspect, the present invention provides the immunostimulatory composition of the first aspect, wherein the nucleic acid molecule comprises at least one deoxyribonucleotide.

According to a fourteenth aspect, the present invention provides the immunostimulatory composition of the first aspect, wherein the nucleic acid molecule is RNA.

According to a fifteenth aspect, the present invention provides the immunostimulatory composition of the first aspect, wherein the nucleic acid molecule is a Toll-like receptor (TLR) agonist.

According to a sixteenth aspect, the present invention provides the immunostimulatory composition of the first aspect, wherein the nucleic acid molecule is an agonist of TLR8.

According to a seventeenth aspect, the present invention provides the immunostimulatory composition of the first aspect, wherein the nucleic acid molecule is an agonist of TLR7.

According to an eighteenth aspect, the present invention provides the immunostimulatory composition of the first aspect, wherein the nucleic acid molecule is an agonist of TLR3.

According to a nineteenth aspect, the present invention provides the immunostimulatory composition of the first aspect, wherein the single-stranded minus-sense RNA virus is an orthomyxovirus.

According to a twentieth aspect, the present invention provides the immunostimulatory composition of the first aspect, wherein the single-stranded minus-sense RNA virus is a paramyxovirus.

According to a twenty first aspect, the present invention provides the immunostimulatory composition of the first aspect, wherein the single-stranded minus-sense RNA virus is a rhabdovirus.

According to a twenty second aspect, the present invention provides the immunostimulatory composition of the first aspect, wherein the single-stranded minus-sense RNA virus is a filovirus.

According to a twenty third aspect, the present invention provides the immunostimulatory composition of the first aspect, wherein the single-stranded minus-sense RNA virus is a bornavirus.

According to a twenty fourth aspect, the present invention provides the immunostimulatory composition of the first aspect, wherein the single-stranded minus-sense RNA virus is an influenza A virus.

According to a twenty fifth aspect, the present invention provides the immunostimulatory composition of the first aspect, wherein the single-stranded minus-sense RNA virus is an influenza B virus.

According to a twenty sixth aspect, the present invention provides the immunostimulatory composition of the first aspect, wherein the nucleic acid molecule is associated with a cationic lipid.

According to a twenty seventh aspect, the present invention provides the immunostimulatory composition of the first aspect, further comprising an antigen.

According to a twenty eighth aspect, an immunostimulatory composition comprising an isolated nucleic acid molecule 4 to 30 nucleotides long comprising a sequence provided by a 3' end of a single-stranded minus-sense RNA virus genome, wherein the nucleic acid molecule has a stabilized backbone, and an antigen.

According to a twenty ninth aspect, an immunostimulatory composition comprising an isolated oligoribonucleotide 7-40 nucleotides long comprising 5'-N₁-C/U-U-G/U-U-N₂-3' wherein U is uracil (U) oxyribonucleoside, C/U is cytosine (C) oxyribonucleoside or uracil (U) oxyribonucleoside, G/U is guanine (G) oxyribonucleoside or uracil (U) oxyribonucleoside, N₁ and N₂ independently are RNA sequences 0-10 nucleotides long, and the oligoribonucleotide has a stabilized backbone.

According to a thirtieth aspect, the present invention provides the immunostimulatory composition of the twenty ninth aspect, wherein 5'-C/U-U-G/U-3' is 5'-CUGU-3'.

According to a thirty first aspect, the present invention provides the immunostimulatory composition of the twenty ninth aspect, wherein 5'-C/U-U-G/U-3' is 5'-UUGU-3'.

According to a thirty second aspect, the present invention provides the immunostimulatory composition of the twenty ninth aspect, wherein 5'-C/U-U-G/U-3' is 5'-CUUU-3'.

According to a thirty third aspect, the present invention provides the immunostimulatory composition of the twenty ninth aspect, wherein 5'-C/U-U-G/U-3' is 5'-UUUU-3'.

According to a thirty fourth aspect, the present invention provides the immunostimulatory composition of the twenty ninth aspect, wherein 5'-N,-C/U-U-G/U-UN₂-3'does not comprise an RNA sequence 5'-GUUGU-3".

According to a thirty fifth aspect, the present invention provides the immunostimulatory composition of the twenty ninth aspect, wherein the oligoribonucleotide is associated with a cationic lipid.

According to a thirty sixth aspect, the present invention provides the immunostimulatory composition of the twenty ninth aspect, further comprising an antigen.

According to a thirty seventh aspect, an immunostimulatory composition comprising a chimeric DNA:RNA oligonucleotide 7-40 nucleotides long comprising 5'-dX₁-N₁-C/U-U-G/U-U-N₂-dX₂-3' wherein U is uracil (U) oxyribonucleoside, C/U is cytosine (C) oxyribonucleoside or uracil (U) oxyribonucleoside, G/U is guanine (G) oxyribonucleoside or uracil (U) oxyribonucleoside, dX₁ and dX₂ independently are DNA sequences 0-6 nucleotides long wherein at least one of dX₁ and dX₂ is at least 1 nucleotide long, and N₁ and N₂ independently are RNA sequences 0-10 nucleotides long.

According to a thirty eighth aspect the chimeric oligonucleotide of the thirty seventh aspect, wherein N₁ and N₂ are both 0 nucleotides long.

According to a thirty ninth aspect, the present invention provides the immunostimulatory composition of the thirty seventh aspect, wherein dX₁ is 0 nucleotides long.

According to a fortieth aspect, the present invention provides the immunostimulatory composition of the thirty seventh aspect, wherein dX₂ is 0 nucleotides long.

According to a forty first aspect, the present invention provides the immunostimulatory composition of the thirty seventh aspect, wherein dX₁, dX₂, or both dXᵢ and dX₂ comprise a CpG motif.

According to a forty second aspect, the present invention provides the immunostimulatory composition of the thirty seventh aspect, further comprising at least one stabilized internucleotide linkage.

According to a forty third aspect, the present invention provides the immunostimulatory composition of the thirty seventh aspect, further comprising at least one phosphorothioate internucleotide linkage.

According to a forty forth aspect, the present invention provides the immunostimulatory composition of the thirty seventh aspect, wherein 5'-C/U-U-G/U-3' is 5'-CUGU-3'.

According to a forty fifth aspect, the present invention provides the immunostimulatory composition of the thirty seventh aspect, wherein 5'-C/U-U-G/U-3' is 5'-UUGU-3'.

According to a forty sixth aspect, the present invention provides the immunostimulatory composition of the thirty seventh aspect, wherein 5'-C/U-U-G/U-3' is 5'-CUUU-3'.

According to a forty seventh aspect, the present invention provides the immunostimulatory composition of the thirty seventh aspect, wherein 5'-C/U-U-G/U-3' is 5'-UUUU-3'.

According to a forty eighth aspect, the present invention provides the immunostimulatory composition of the thirty seventh aspect, wherein 5'-dX₁-N₁-C/U-U-G/U-U-N₂-dX₂-3' does not comprise an RNA sequence 5'-GUUGU-3'.

According to a forty ninth aspect, the present invention provides the immunostimulatory composition of the thirty seventh aspect, wherein the chimeric DNA:RNA oligonucleotide is associated with a cationic lipid.

According to a fifty aspect, the present invention provides the immunostimulatory composition of the thirty seventh aspect, further comprising an antigen.

According to a fifty first aspect, the present invention provides a method for altering an immunostimulatory profile of a reference oligonucleotide having a reference immunostimulatory profile, the method comprising altering a reference oligonucleotide 3-40 nucleotides long to include an RNA motif 5'-C/U-U-G/U-U-3', wherein U is uracil (U) oxyribonucleoside, C/U is cytosine (C) oxyribonucleoside or uracil (U) oxyribonucleoside, G/U is guanine (G) oxyribonucleoside or uracil (U) oxyribonucleoside, wherein the reference oligonucleotide does not include the immunostimulatory RNA motif 5'-C/U-U-G/U-U-3', wherein the altering results in an altered oligonucleotide having an altered immunostimulatory profile distinct from the reference immunostimulatory profile.

According to a fifty second aspect, the present invention provides the method of the fifty first aspect, wherein the reference oligonucleotide is an oligoribonucleotide.

According to a fifty third aspect, the present invention provides the method of the fifty first aspect, wherein the reference oligonucleotide is an oligodeoxyribonucleotide (ODN).

According to a fifty fourth aspect, the present invention provides the method of the fifty third aspect, wherein the reference oligonucleotide is a CpG ODN.

According to a fifty fifth aspect, the present invention provides the method of the fifty first aspect, wherein 5'-C/U-U-G/U-3' is 5'-CUGU-3'.

According to a fifty sixth aspect, the present invention provides the method of the fifty first aspect, wherein 5'-C/U-U-G/U-3' is 5'-UUGU-3'.

According to a fifty seventh aspect, the present invention provides the method of the fifty first aspect, wherein 5'-C/U-U-G/U-3' is 5'-CUUU-3'.

According to a fifty eighth aspect, the present invention provides the method of the fifty first aspect, wherein 5'-C/U-U-G/U-3' is 5'-UUUU-3'.

According to a fifty ninth aspect, the present invention provides the method of the fifty first aspect, wherein the reference oligonucleotide has a stabilized backbone.

According to a sixtieth aspect, the present invention provides the method of the fifty first aspect, wherein the reference immunostimulatory profile is non-immunostimulatory.

According to a sixty first aspect, the present invention provides a method for altering an immunostimulatory profile of a CpG oligodeoxynucleotide (CpG ODN) having a reference immunostimulatory profile, the method comprising replacing at least one dC of the CpG ODN, at least one dT of the CpG ODN, or at least one dC of the CpG ODN and at least one dT of the CpG ODN with U, wherein U is uracil oxyribonucleoside, and wherein the replacing results in an altered oligonucleotide having an altered immunostimulatory profile distinct from the reference immunostimulatory profile.

According to a sixty second aspect, the present invention provides the method of the sixty first aspect, wherein every dC of the CpG ODN is replaced by U.

According to a sixty third aspect, the present invention provides the method of the sixty first aspect, wherein every dT of the CpG ODN is replaced by U.

According to a sixty fourth aspect, the present invention provides the method of the sixty first aspect, wherein every dC and every dT of the CpG ODN is replaced by U.

According to a sixty fifth aspect, the present invention provides the method of the sixty first aspect, further comprising replacing at least one dG of the CpG ODN by G, at least one dA of the CpG ODN by A, or at least one dG of the CpG ODN by G and at least one dA of the CpG ODN by A, wherein G is guanine oxyribonucleoside and wherein A is adenine oxyribonucleoside.

According to a sixty sixth aspect, the present invention provides the method of the sixty fifth aspect, wherein every dG of the CpG ODN is replaced by G.

According to a sixty seventh aspect, the present invention provides the method of the sixty fifth aspect, wherein every dA of the CpG ODN is replaced by A.

According to a sixty eighth aspect, the present invention provides the method of the sixty fifth aspect, further wherein every dC and every dT of the CpG ODN is replaced by U.

According to a sixty ninth aspect, the present invention provides the method of the sixty fifth aspect, wherein every dG of the CpG ODN is replaced by G and every dA of the CpG ODN is replaced by A.

According to a seventieth aspect, the present invention provides the method of the sixty ninth aspect, further wherein every dC and every dT of the CpG ODN is replaced by U.

According to a seventy first aspect, the present invention provides the method of the sixty first aspect, wherein the altered oligonucleotide comprises a sequence motif 5'-C/U-U-G/U-U-3', wherein U is uracil (U) oxyribonucleoside, C/U is cytosine (C) oxyribonucleoside or uracil (U) oxyribonucleoside, G/U is guanine (G) oxyribonucleoside or uracil (U) oxyribonucleoside.

According to a seventy second aspect, the present invention provides the method of the sixty first aspect, wherein the altered oligonucleotide has a stabilized backbone.

According to a seventy third aspect, the present invention provides a composition comprising an isolated immunostimulatory oligoribonucleotide provided as 5'-UUGUUGUUUUGUUGUUUUGUUGUU-3' (SEQ ID NO: 286).

According to a seventy fourth aspect, the present invention provides a composition comprising an isolated immunostimulatory oligoribonucleotide provided as 5'-TUGTUGTTTTGTUGTTTTGTUGTT-3' (SEQ ID NO: 287).

According to a seventy fifth aspect, the present invention provides a method for stimulating an immune response, comprising: contacting a cell of the immune system with an effective amount of a composition of any one of the first to the twenty ninth aspects and the thirty seventh aspect to stimulate an immune response.

According to a seventy sixth aspect, the present invention provides a method for stimulating a Th1-like immune response, comprising: contacting a cell of the immune system with an effective amount of a composition of any one of the first to the twenty ninth aspects and the thirty seventh aspect to stimulate a Th1-like immune response.

According to a seventy seventh aspect, the present invention provides the method of the seventy sixth aspect, wherein the Th1-like immune response comprises expression of a type 1 interferon.

According to a seventy eighth aspect, the present invention provides the method of the seventy sixth aspect, wherein the Th1-like immune response comprises expression of interleukin 12 (IL-12).

According to a seventy ninth aspect, the present invention provides a method for stimulating TLR signaling, comprising: contacting a cell expressing a TLR with an effective amount of a composition of any one of the first to the twenty ninth aspects and the thirty seventh aspect to stimulate signaling by the TLR.

According to an eightieth aspect, the present invention provides the method of the seventy ninth aspect, wherein the TLR is TLR9.

According to an eighty first aspect, the present invention provides the method of the seventy ninth aspect, wherein the TLR is TLR8.

According to an eighty second aspect, the present invention provides the method of the seventy ninth aspect, wherein the TLR is TLR7.

According to an eighty third aspect, the present invention provides the method of the seventy ninth aspect, wherein the TLR is TLR3.

According to an eighty fourth aspect, the present invention provides a method for stimulating an immune response in a subject, comprising: administering to a subject an effective amount of a composition of the first to the twenty ninth aspects and the thirty seventh aspect to stimulate an immune response in the subject.

According to an eighty fifth aspect, the present invention provides a method for stimulating a Th1-like immune response in a subject, comprising: administering to a subject an effective amount of a composition of the first to the twenty ninth aspects and the thirty seventh aspect to stimulate a Th1-like immune response in the subject.

According to an eighty sixth aspect, the present invention provides the method of the eighty fifth aspect, wherein the Th1-like immune response comprises expression of a type 1 interferon in the subject.

According to an eighty seventh aspect, the present invention provides the method of the eighty fifth aspect, wherein the Th1-like immune response comprises expression of IL-12 in the subject.

According to an eighty eighth aspect, the present invention provides a method for stimulating an antigen-specific immune response in a subject, comprising: administering to a subject an effective amount of a composition of any one of the first to the twenty ninth aspects and the thirty seventh aspect and an antigen to stimulate an antigen-specific immune response in the subject.

According to an eighty ninth aspect, the present invention provides the method of the eighty eighth aspect, wherein the antigen comprises an allergen.

According to a ninetieth aspect, the present invention provides the method of the eighty eighth aspect, wherein the antigen comprises a viral antigen.

According to a ninety first aspect, the present invention provides the method of the eighty eighth aspect, wherein the antigen comprises a bacterial antigen.

According to a ninety second aspect, the present invention provides the method of the eighty eighth aspect, wherein the antigen comprises a cancer antigen.

According to a ninety third aspect, the present invention provides a method for treating an allergic condition in a subject, comprising: administering to a subject having or at risk of developing an allergic condition an effective amount of a composition of any one of the first to the twenty ninth aspects and the thirty seventh aspect to treat the allergic condition.

According to a ninety fourth aspect, the present invention provides a method for treating asthma in a subject, comprising: administering to a subject having or at risk of developing asthma an effective amount of a composition of any one of the first to the twenty ninth aspects and the thirty seventh aspect to treat the asthma.

According to a ninety fifth aspect, the present invention provides a method for treating an infection in a subject, comprising: administering to a subject having or at risk of developing an infection an effective amount of a composition of any one of the first to the twenty ninth aspects and the thirty seventh aspect to treat the infection.

According to a ninety sixth aspect, the present invention provides the method of the ninety fifth aspect, wherein the infection comprises a viral infection.

According to a ninety seventh aspect, the present invention provides the method of the ninety fifth aspect, wherein the infection comprises a bacterial infection.

According to a ninety eighth aspect, the present invention provides a method for treating cancer in a subject, comprising: administering to a subject having or at risk of developing cancer an effective amount of a composition of any one of the first to the twenty ninth aspects and the thirty seventh aspect to treat the cancer.

According to a ninety ninth aspect, the present invention provides a method for screening for an antagonist of a TLR, comprising contacting a reference cell expressing a TLR with an effective amount of a composition of any one of the first to the twenty ninth aspects and the thirty seventh aspect, in absence of a candidate antagonist of the TLR, and measuring a reference amount of signaling by the TLR; contacting a test cell expressing the TLR with an effective amount of the composition, in presence of the candidate antagonist of the TLR, and measuring a test amount of signaling by the TLR; and determining the candidate antagonist of the TLR is an antagonist of the TLR when the reference amount of signaling exceeds the test amount of signaling.

According to a hundredth aspect, the present invention provides the method of the ninety ninth aspect, wherein the TLR is TLR9.

According to a hundred and first aspect, the present invention provides the method of the ninety ninth aspect, wherein the TLR is TLR8.

According to a hundred and second aspect, the present invention provides the method of the ninety ninth aspect, wherein the TLR is TLR7.

According to a hundred and third aspect, the present invention provides the method of the ninety ninth aspect, wherein the TLR is TLR3.

## Claims

1. An immunostimulatory composition comprising an isolated oligoribonucleotide 7-40 nucleotides long comprising
5'-N₁-U-U-G-U-N₂-3'
wherein U is uracil (U) oxyribonucleoside, G is guanine (G) oxyribonucleoside, N₁ and N₂ independently are RNA sequences 0-10 nucleotides long, and the oligoribonucleotide has a stabilized backbone.

2. The immunostimulatory composition of claim 1, wherein the oligoribonucleotide is associated with a cationic lipid.

3. The immunostimulatory composition of claim 1, further comprising an antigen.

4. A method for stimulating an immune response, comprising:
contacting a cell of the immune system with an effective amount of a composition of any one of claims 1-3 to stimulate an immune response.

5. A method for stimulating a Th1-like immune response, comprising:
contacting a cell of the immune system with an effective amount of a composition of any one of claims 1-4 to stimulate a Th1-like immune response.

6. The method of claim 5, wherein the Th1-like immune response comprises expression of a type 1 interferon.

7. The method of claim 6, wherein the Th1-like immune response comprises expression of interleukin 12.

8. A method for stimulating TLR signaling, comprising:
contacting a cell expressing a TLR with an effective amount of a composition of any one of claims 1-3 to stimulate signaling by the TLR wherein the TLR is TLR9, TLR8, TLR7, and TLR3, or a combination thereof.

9. A composition of any one of claims 1-3 for use in (a) stimulating an immune response in a subject, or (b) treating an allergic condition, asthma, viral infection, bacterial infection, or cancer in a subject.

10. A composition of any one of claims 1-3 and an antigen for use in stimulating an antigen-specific immune response in a subject.

11. The composition of claim 10, wherein the antigen comprises an allergen, viral antigen, bacterial antigen, or cancer antigen.


SEQUENCE LISTING

<110>  Lipford, Grayson B
       Forsbach, Alexandra

<120>  IMMUNOSTIMULATORY DNA:RNA OLIGONUCLEOTIDES

<130>  PC31837A-EP-DIV

<150>  05756145.8
<151>  2005-02-18

<160>  344

<170>  PatentIn version 3.5

<210>  1
<211>  18
<212>  RNA
<213>  Artificial Sequence

<220>
<223>  Synthetic oligonucleotide

<400>  1
guugugguug ugguugug                                                    18


<210>  2
<211>  20
<212>  RNA
<213>  Artificial Sequence

<220>
<223>  Synthetic oligonucleotide

<400>  2
uuguacgcau uuuuucgcgu                                                  20


<210>  3
<211>  20
<212>  RNA
<213>  Artificial Sequence

<220>
<223>  Synthetic oligonucleotide

<400>  3
cuuacccaac uuuguuuggu                                                  20


<210>  4
<211>  20
<212>  RNA
<213>  Artificial Sequence

<220>
<223>  Synthetic oligonucleotide

<400>  4
uguuuuuucu cuuguuuggu                                                  20


<210>  5
<211>  20
<212>  RNA
<213>  Artificial Sequence

<220>
<223>  Synthetic oligonucleotide

<400>  5
auaauugacc ugcuuucgcu                                                  20


<210>  6

<211>  20
<212>  RNA
<213>  Artificial Sequence

<220>
<223>  Synthetic oligonucleotide

<400>  6
uugaucuggu uguuaagcgu                                                   20


<210>  7
<211>  20
<212>  RNA
<213>  Artificial Sequence

<220>
<223>  Synthetic oligonucleotide

<400>  7
aaugguuugu uugucuucgu                                                   20


<210>  8
<211>  30
<212>  RNA
<213>  Artificial Sequence

<220>
<223>  Synthetic oligonucleotide

<400>  8
aaaaucauca ucucuuguuu uugugugucu                                        30


<210>  9
<211>  20
<212>  RNA
<213>  Artificial Sequence

<220>
<223>  Synthetic oligonucleotide

<400>  9
ucucuuguuu uugugugucu                                                   20


<210>  10
<211>  15
<212>  RNA
<213>  Artificial Sequence

<220>
<223>  Synthetic oligonucleotide

<400>  10
uguuuuugug ugucu                                                        15


<210>  11
<211>  10
<212>  RNA
<213>  Artificial Sequence

<220>
<223>  Synthetic oligonucleotide

<400>  11
uugugugucu                                                              10


<210>  12
<211>  30
<212>  RNA
<213>  Artificial Sequence

<220>

<223>  Synthetic oligonucleotide

<400>  12
caaaaucauc aucucuuguu uuugugugu c                                    30

<210>  13
<211>  20
<212>  RNA
<213>  Artificial Sequence

<220>
<223>  Synthetic oligonucleotide

<400>  13
aucucuuguu uuugugugu c                                               20

<210>  14
<211>  15
<212>  RNA
<213>  Artificial Sequence

<220>
<223>  Synthetic oligonucleotide

<400>  14
uuguuuuugu guguc                                                     15

<210>  15
<211>  10
<212>  RNA
<213>  Artificial Sequence

<220>
<223>  Synthetic oligonucleotide

<400>  15
uuugugugu c                                                          10

<210>  16
<211>  30
<212>  RNA
<213>  Artificial Sequence

<220>
<223>  Synthetic oligonucleotide

<400>  16
uuugugugu c ucucuuguuu uugugugucu                                    30

<210>  17
<211>  30
<212>  RNA
<213>  Artificial Sequence

<220>
<223>  Synthetic oligonucleotide

<400>  17
uaaaaauucu ucuuucuuuu ugugugu ccg                                    30

<210>  18
<211>  20
<212>  RNA
<213>  Artificial Sequence

<220>
<223>  Synthetic oligonucleotide

<400>  18
ucuuucuuuu ugugugu ccg                                               20

<210>  19
<211>  15
<212>  RNA
<213>  Artificial Sequence

<220>
<223>  Synthetic oligonucleotide

<400>  19
cuuuuugugu guccg                                                        15


<210>  20
<211>  10
<212>  RNA
<213>  Artificial Sequence

<220>
<223>  Synthetic oligonucleotide

<400>  20
   ugugaguccg                                                             10

<210>  21
<211>  30
<212>  RNA
<213>  Artificial Sequence

<220>
<223>  Synthetic oligonucleotide

<400>  21
cuaaaaauuc uucuuucuuu uugugugccc                                        30


<210>  22
<211>  20
<212>  RNA
<213>  Artificial Sequence

<220>
<223>  Synthetic oligonucleotide

<400>  22
uucuuucuuu uugugugccc                                                   20


<210>  23
<211>  15
<212>  RNA
<213>  Artificial Sequence

<220>
<223>  Synthetic oligonucleotide

<400>  23
ucuuuuugug ugccc                                                        15


<210>  24
<211>  10
<212>  RNA
<213>  Artificial Sequence

<220>
<223>  Synthetic oligonucleotide

<400>  24
uugugugccc                                                              10


<210>  25
<211>  30
<212>  RNA
<213>  Artificial Sequence

<220>
<223>  Synthetic oligonucleotide

<400>  25
uaaaaaaccu uuuuucuuuu uguguguccg                                        30


<210>  26
<211>  20
<212>  RNA
<213>  Artificial Sequence

<220>
<223>  Synthetic oligonucleotide

<400>  26
   uuuuucuuuu uguguguccg                                                   20


<210>  27
<211>  30
<212>  RNA
<213>  Artificial Sequence

<220>
<223>  Synthetic oligonucleotide

<400>  27
cugagcuuag ucaaguuacu uuucuuauac                                       30


<210>  28
<211>  20
<212>  RNA
<213>  Artificial Sequence

<220>
<223>  Synthetic oligonucleotide

<400>  28
ucaaguuacu uuucuuauac                                                  20


<210>  29
<211>  15
<212>  RNA
<213>  Artificial Sequence

<220>
<223>  Synthetic oligonucleotide

<400>  29
uuacuuuucu uauac                                                       15


<210>  30
<211>  10
<212>  RNA
<213>  Artificial Sequence

<220>
<223>  Synthetic oligonucleotide

<400>  30
uuucuuauac                                                             10


<210>  31
<211>  30
<212>  RNA
<213>  Artificial Sequence

<220>
<223>  Synthetic oligonucleotide

<400>  31

cugagcuuag ucaaguuacu uuuuuuauac                                          30

<210>  32
<211>  20
<212>  RNA
<213>  Artificial Sequence

<220>
<223>  Synthetic oligonucleotide

<400>  32
ucaaguuacu uuuuuuauac                                                     20

<210>  33
<211>  15
<212>  RNA
<213>  Artificial Sequence

<220>
<223>  Synthetic oligonucleotide

<400>  33
uuacuuuuuu uauac                                                          15

<210>  34
<211>  10
<212>  RNA
<213>  Artificial Sequence

<220>
<223>  Synthetic oligonucleotide

<400>  34
uuuuuuauac                                                                10

<210>  35
<211>  30
<212>  RNA
<213>  Artificial Sequence

<220>
<223>  Synthetic oligonucleotide

<400>  35
uguaacauaa cucaucaucu uuuaugauac                                          30

<210>  36
<211>  20
<212>  RNA
<213>  Artificial Sequence

<220>
<223>  Synthetic oligonucleotide

<400>  36
cucaucaucu uuuaugauac                                                     20

<210>  37
<211>  15
<212>  RNA
<213>  Artificial Sequence

<220>
<223>  Synthetic oligonucleotide

<400>  37
caucuuuuau gauac                                                          15

<210>  38
<211>  10

<212>  RNA
<213>  Artificial Sequence

<220>
<223>  Synthetic oligonucleotide

<400>  38
uuuaugauac                                                              10


<210>  39
<211>  30
<212>  DNA
<213>  Artificial Sequence

<220>
<223>  Synthetic oligonucleotide

<400>  39
gtatcaaaaa agatgatgat acttggaaga                                        30


<210>  40
<211>  30
<212>  RNA
<213>  Artificial Sequence

<220>
<223>  Synthetic oligonucleotide

<400>  40
ucuuccaagu aucaucaucu uuuuugauac                                        30


<210>  41
<211>  20
<212>  RNA
<213>  Artificial Sequence

<220>
<223>  Synthetic oligonucleotide

<400>  41
aucaucaucu uuuuugauac                                                   20


<210>  42
<211>  15
<212>  RNA
<213>  Artificial Sequence

<220>
<223>  Synthetic oligonucleotide

<400>  42
caucuuuuuu gauac                                                        15


<210>  43
<211>  10
<212>  RNA
<213>  Artificial Sequence

<220>
<223>  Synthetic oligonucleotide

<400>  43
uuuuugauac                                                              10


<210>  44
<211>  30
<212>  RNA
<213>  Artificial Sequence

<220>
<223>  Synthetic oligonucleotide

<400>  44
ugugucuucu uugaucuggu uguuaagcgu                                        30


<210>  45
<211>  30
<212>  RNA
<213>  Artificial Sequence

<220>
<223>  Synthetic oligonucleotide

<400>  45
uguuuuuucu uugaucuggu uguuaagcgu                                        30


<210>  46
<211>  15
<212>  RNA
<213>  Artificial Sequence

<220>
<223>  Synthetic oligonucleotide

<400>  46
cugguuguua agcgu                                                        15


<210>  47
<211>  10
<212>  RNA
<213>  Artificial Sequence

<220>
<223>  Synthetic oligonucleotide

<400>  47
uguuaagcgu                                                              10


<210>  48
<211>  30
<212>  RNA
<213>  Artificial Sequence

<220>
<223>  Synthetic oligonucleotide

<400>  48
ugcuucuucu uugguuuugu uguuaagcgu                                        30


<210>  49
<211>  20
<212>  RNA
<213>  Artificial Sequence

<220>
<223>  Synthetic oligonucleotide

<400>  49
uugguuuugu uguuaagcgu                                                   20


<210>  50
<211>  15
<212>  RNA
<213>  Artificial Sequence

<220>
<223>  Synthetic oligonucleotide

<400>  50
uuuguuguua agcgu                                                        15

<210>  51
<211>  30
<212>  RNA
<213>  Artificial Sequence

<220>
<223>  Synthetic oligonucleotide

<400>  51
cuuuucuucu cugguuuugu uguuaagcgu                                        30


<210>  52
<211>  20
<212>  RNA
<213>  Artificial Sequence

<220>
<223>  Synthetic oligonucleotide

<400>  52
cugguuuugu uguuaagcgu                                                   20


<210>  53
<211>  30
<212>  RNA
<213>  Artificial Sequence

<220>
<223>  Synthetic oligonucleotide

<400>  53
ugauuuuuau augguuuuuu uguuaagcgu                                        30


<210>  54
<211>  20
<212>  RNA
<213>  Artificial Sequence

<220>
<223>  Synthetic oligonucleotide

<400>  54
augguuuuuu uguuaagcgu                                                   20


<210>  55
<211>  15
<212>  RNA
<213>  Artificial Sequence

<220>
<223>  Synthetic oligonucleotide

<400>  55
uuuuuuguua agcgu                                                        15


<210>  56
<211>  30
<212>  RNA
<213>  Artificial Sequence

<220>
<223>  Synthetic oligonucleotide

<400>  56
uaaugauaau aaugguuugu uugucuucgu                                        30


<210>  57
<211>  15
<212>  RNA
<213>  Artificial Sequence

<220>
<223>  Synthetic oligonucleotide

<400>  57
uuuguuuguc uucgu                                                        15


<210>  58
<211>  10
<212>  RNA
<213>  Artificial Sequence

<220>
<223>  Synthetic oligonucleotide

<400>  58
uugucuucgu                                                              10


<210>  59
<211>  30
<212>  RNA
<213>  Artificial Sequence

<220>
<223>  Synthetic oligonucleotide

<400>  59
uaaugguaau aaugguuugu uugucuucgu                                        30


<210>  60
<211>  30
<212>  RNA
<213>  Artificial Sequence

<220>
<223>  Synthetic oligonucleotide

<400>  60
uaauuauauu aaugguuugu uugucuucgu                                        30


<210>  61
<211>  30
<212>  RNA
<213>  Artificial Sequence

<220>
<223>  Synthetic oligonucleotide

<400>  61
uaauuguaau aaugguuuuu uugucuucgu                                        30


<210>  62
<211>  20
<212>  RNA
<213>  Artificial Sequence

<220>
<223>  Synthetic oligonucleotide

<400>  62
aaugguuuuu uugucuucgu                                                   20


<210>  63
<211>  15
<212>  RNA
<213>  Artificial Sequence

<220>
<223>  Synthetic oligonucleotide

<400>  63
uuuuuuuguc uucgu                                                        15

<210>  64
<211>  30
<212>  RNA
<213>  Artificial Sequence

<220>
<223>  Synthetic oligonucleotide

<400>  64
uaauuguaag aaugguuuuu uugucuucgu                                        30


<210>  65
<211>  30
<212>  RNA
<213>  Artificial Sequence

<220>
<223>  Synthetic oligonucleotide

<400>  65
uuguauuagg aaugguuuuu uugucuucgu                                        30


<210>  66
<211>  30
<212>  RNA
<213>  Artificial Sequence

<220>
<223>  Synthetic oligonucleotide

<400>  66
uaauguuauc aaugguuuau uugucuucgu                                        30


<210>  67
<211>  20
<212>  RNA
<213>  Artificial Sequence

<220>
<223>  Synthetic oligonucleotide

<400>  67
aaugguuuau uugucuucgu                                                   20


<210>  68
<211>  15
<212>  RNA
<213>  Artificial Sequence

<220>
<223>  Synthetic oligonucleotide

<400>  68
uuuauuuguc uucgu                                                        15


<210>  69
<211>  30
<212>  RNA
<213>  Artificial Sequence

<220>
<223>  Synthetic oligonucleotide

<400>  69
uaagaaugcu auugguuugu uuuucuucgu                                        30


<210>  70
<211>  20
<212>  RNA

<213>  Artificial Sequence

<220>
<223>  Synthetic oligonucleotide

<400>  70
auugguuugu uuuucuucgu                                                    20


<210>  71
<211>  15
<212>  RNA
<213>  Artificial Sequence

<220>
<223>  Synthetic oligonucleotide

<400>  71
   uuuguuuuuc uucgu                                                          15


<210>  72
<211>  10
<212>  RNA
<213>  Artificial Sequence

<220>
<223>  Synthetic oligonucleotide

<400>  72
uuuucuucgu                                                               10


<210>  73
<211>  30
<212>  RNA
<213>  Artificial Sequence

<220>
<223>  Synthetic oligonucleotide

<400>  73
uugucauaua auugguuuuu uugucuucgu                                         30


<210>  74
<211>  20
<212>  RNA
<213>  Artificial Sequence

<220>
<223>  Synthetic oligonucleotide

<400>  74
auugguuuuu uugucuucgu                                                    20


<210>  75
<211>  30
<212>  RNA
<213>  Artificial Sequence

<220>
<223>  Synthetic oligonucleotide

<400>  75
auaucaauua guuuuuuugu uuuuucucgu                                          30


<210>  76
<211>  20
<212>  RNA
<213>  Artificial Sequence

<220>
<223>  Synthetic oligonucleotide

<400>  76
guuuuuugu uuuuucucgu                                                    20


<210>  77
<211>  15
<212>  RNA
<213>  Artificial Sequence

<220>
<223>  Synthetic oligonucleotide

<400>  77
uuuguuuuu cucgu                                                         15


<210>  78
<211>  10
<212>  RNA
<213>  Artificial Sequence

<220>
<223>  Synthetic oligonucleotide

<400>  78
uuuuucucgu                                                              10


<210>  79
<211>  30
<212>  RNA
<213>  Artificial Sequence

<220>
<223>  Synthetic oligonucleotide

<400>  79
aaucacuaua guuuuuuugu uuuucuccgu                                         30


<210>  80
<211>  20
<212>  RNA
<213>  Artificial Sequence

<220>
<223>  Synthetic oligonucleotide

<400>  80
   guuuuuuugu uuuucuccgu                                                    20


<210>  81
<211>  15
<212>  RNA
<213>  Artificial Sequence

<220>
<223>  Synthetic oligonucleotide

<400>  81
   uuuguuuuuc uccgu                                                         15


<210>  82
<211>  10
<212>  RNA
<213>  Artificial Sequence

<220>
<223>  Synthetic oligonucleotide

<400>  82
uuuucuccgu                                                              10


<210>  83

<211>  30
<212>  RNA
<213>  Artificial Sequence

<220>
<223>  Synthetic oligonucleotide

<400>  83
aacacguauc cauauuuccc cuuguucggu                                        30


<210>  84
<211>  20
<212>  RNA
<213>  Artificial Sequence

<220>
<223>  Synthetic oligonucleotide

<400>  84
cauauuuccc cuuguucggu                                                   20


<210>  85
<211>  15
<212>  RNA
<213>  Artificial Sequence

<220>
<223>  Synthetic oligonucleotide

<400>  85
uuccccuugu ucggu                                                        15


<210>  86
<211>  10
<212>  RNA
<213>  Artificial Sequence

<220>
<223>  Synthetic oligonucleotide

<400>  86
cuuguucggu                                                              10


<210>  87
<211>  30
<212>  RNA
<213>  Artificial Sequence

<220>
<223>  Synthetic oligonucleotide

<400>  87
uugaacuauc cuuacccaac uuuguuuggu                                        30


<210>  88
<211>  15
<212>  RNA
<213>  Artificial Sequence

<220>
<223>  Synthetic oligonucleotide

<400>  88
ccaacuuugu uuggu                                                        15


<210>  89
<211>  10
<212>  RNA
<213>  Artificial Sequence

<220>

<223>  Synthetic oligonucleotide

<400>  89
uuuguuuggu                                                            10


<210>  90
<211>  30
<212>  RNA
<213>  Artificial Sequence

<220>
<223>  Synthetic oligonucleotide

<400>  90
uugaucuauc cuuacccaac uuuguuuggu                                      30


<210>  91
<211>  30
<212>  RNA
<213>  Artificial Sequence

<220>
<223>  Synthetic oligonucleotide

<400>  91
uagaccgauc cuuacccaac uuuguuuggu                                      30


<210>  92
<211>  30
<212>  RNA
<213>  Artificial Sequence

<220>
<223>  Synthetic oligonucleotide

<400>  92
uauaccuauc cuuacccagc uuuguuuggu                                      30


<210>  93
<211>  20
<212>  RNA
<213>  Artificial Sequence

<220>
<223>  Synthetic oligonucleotide

<400>  93
cuuacccagc uuuguuuggu                                                 20


<210>  94
<211>  15
<212>  RNA
<213>  Artificial Sequence

<220>
<223>  Synthetic oligonucleotide

<400>  94
ccagcuuugu uuggu                                                      15


<210>  95
<211>  30
<212>  RNA
<213>  Artificial Sequence

<220>
<223>  Synthetic oligonucleotide

<400>  95
uagaacgauc cuuacccagc uuugucuggu                                      30

<210>  96
<211>  20
<212>  RNA
<213>  Artificial Sequence

<220>
<223>  Synthetic oligonucleotide

<400>  96
cuuacccagc uuugucuggu                                                    20


<210>  97
<211>  15
<212>  RNA
<213>  Artificial Sequence

<220>
<223>  Synthetic oligonucleotide

<400>  97
ccagcuuugu cuggu                                                         15


<210>  98
<211>  10
<212>  RNA
<213>  Artificial Sequence

<220>
<223>  Synthetic oligonucleotide

<400>  98
uuugucuggu                                                               10


<210>  99
<211>  30
<212>  RNA
<213>  Artificial Sequence

<220>
<223>  Synthetic oligonucleotide

<400>  99
uuuaacuauc cuuagccaac uuugucuggu                                         30


<210>  100
<211>  20
<212>  RNA
<213>  Artificial Sequence

<220>
<223>  Synthetic oligonucleotide

<400>  100
cuuagccaac uuugucuggu                                                    20


<210>  101
<211>  15
<212>  RNA
<213>  Artificial Sequence

<220>
<223>  Synthetic oligonucleotide

<400>  101
ccaacuuugu cuggu                                                         15


<210>  102
<211>  30
<212>  RNA
<213>  Artificial Sequence

<220>
<223>  Synthetic oligonucleotide

<400>  102
uuuaucuauc cuuagccaac uuugucuggu                                        30


<210>  103
<211>  30
<212>  RNA
<213>  Artificial Sequence

<220>
<223>  Synthetic oligonucleotide

<400>  103
uuuaucuauc cauagccaac uuuuucuggu                                        30


<210>  104
<211>  20
<212>  RNA
<213>  Artificial Sequence

<220>
<223>  Synthetic oligonucleotide

<400>  104
cauagccaac uuuuucuggu                                                   20


<210>  105
<211>  15
<212>  RNA
<213>  Artificial Sequence

<220>
<223>  Synthetic oligonucleotide

<400>  105
ccaacuuuuu cuggu                                                        15


<210>  106
<211>  10
<212>  RNA
<213>  Artificial Sequence

<220>
<223>  Synthetic oligonucleotide

<400>  106
uuuuucuggu                                                              10


<210>  107
<211>  30
<212>  RNA
<213>  Artificial Sequence

<220>
<223>  Synthetic oligonucleotide

<400>  107
uuaucguaac ucaccgauuc ucuguuuggu                                        30


<210>  108
<211>  20
<212>  RNA
<213>  Artificial Sequence

<220>
<223>  Synthetic oligonucleotide

<400>  108

ucaccgauuc ucuguuuggu 20

<210> 109
<211> 15
<212> RNA
<213> Artificial Sequence

<220>
<223> Synthetic oligonucleotide

<400> 109
gauucucugu uuggu 15

<210> 110
<211> 10
<212> RNA
<213> Artificial Sequence

<220>
<223> Synthetic oligonucleotide

<400> 110
ucuguuuggu 10

<210> 111
<211> 30
<212> RNA
<213> Artificial Sequence

<220>
<223> Synthetic oligonucleotide

<400> 111
uuaucguaac ucacggauuc ucuguuuggu 30

<210> 112
<211> 20
<212> RNA
<213> Artificial Sequence

<220>
<223> Synthetic oligonucleotide

<400> 112
ucacggauuc ucuguuuggu 20

<210> 113
<211> 30
<212> RNA
<213> Artificial Sequence

<220>
<223> Synthetic oligonucleotide

<400> 113
uuaucguaac uuacggauuc ucuguuuggu 30

<210> 114
<211> 20
<212> RNA
<213> Artificial Sequence

<220>
<223> Synthetic oligonucleotide

<400> 114
uuacggauuc ucuguuuggu 20

<210> 115
<211> 30

<212>  RNA
<213>  Artificial Sequence

<220>
<223>  Synthetic oligonucleotide

<400>  115
uuaucguacc ucacagauuc ucuguuuggu                                        30


<210>  116
<211>  20
<212>  RNA
<213>  Artificial Sequence

<220>
<223>  Synthetic oligonucleotide

<400>  116
ucacagauuc ucuguuuggu                                                   20


<210>  117
<211>  30
<212>  RNA
<213>  Artificial Sequence

<220>
<223>  Synthetic oligonucleotide

<400>  117
uuaucguacc uuacagauuc ucuguuuggu                                        30


<210>  118
<211>  20
<212>  RNA
<213>  Artificial Sequence

<220>
<223>  Synthetic oligonucleotide

<400>  118
uuacagauuc ucuguuuggu                                                   20


<210>  119
<211>  30
<212>  RNA
<213>  Artificial Sequence

<220>
<223>  Synthetic oligonucleotide

<400>  119
ccgauauccc aucuucuuuu uccccuuggu                                        30


<210>  120
<211>  20
<212>  RNA
<213>  Artificial Sequence

<220>
<223>  Synthetic oligonucleotide

<400>  120
aucuucuuuu uccccuuggu                                                   20


<210>  121
<211>  15
<212>  RNA
<213>  Artificial Sequence

<220>
<223>  Synthetic oligonucleotide

<400>  121
cuuuuucccc uuggu                                                        15


<210>  122
<211>  10
<212>  RNA
<213>  Artificial Sequence

<220>
<223>  Synthetic oligonucleotide

<400>  122
uccccuuggu                                                              10


<210>  123
<211>  30
<212>  RNA
<213>  Artificial Sequence

<220>
<223>  Synthetic oligonucleotide

<400>  123
ccaacauccc aucuucuuuu uccccuuggu                                        30


<210>  124
<211>  30
<212>  RNA
<213>  Artificial Sequence

<220>
<223>  Synthetic oligonucleotide

<400>  124
ccaauauccc aucuucauuu uccccuuggu                                        30


<210>  125
<211>  20
<212>  RNA
<213>  Artificial Sequence

<220>
<223>  Synthetic oligonucleotide

<400>  125
aucuucauuu uccccuuggu                                                   20


<210>  126
<211>  15
<212>  RNA
<213>  Artificial Sequence

<220>
<223>  Synthetic oligonucleotide

<400>  126
cauuuucccc uuggu                                                        15


<210>  127
<211>  30
<212>  RNA
<213>  Artificial Sequence

<220>
<223>  Synthetic oligonucleotide

<400>  127
accgauaucc caucuucauu uuccccuugg                                        30

<210>  128
<211>  20
<212>  RNA
<213>  Artificial Sequence

<220>
<223>  Synthetic oligonucleotide

<400>  128
caucuucauu uuccccuugg                                                   20


<210>  129
<211>  15
<212>  RNA
<213>  Artificial Sequence

<220>
<223>  Synthetic oligonucleotide

<400>  129
ucauuuuccc cuugg                                                        15


<210>  130
<211>  10
<212>  RNA
<213>  Artificial Sequence

<220>
<223>  Synthetic oligonucleotide

<400>  130
uuccccuugg                                                              10


<210>  131
<211>  30
<212>  RNA
<213>  Artificial Sequence

<220>
<223>  Synthetic oligonucleotide

<400>  131
ccaauauccc auauucauuc uccccuuggu                                        30


<210>  132
<211>  20
<212>  RNA
<213>  Artificial Sequence

<220>
<223>  Synthetic oligonucleotide

<400>  132
auauucauuc uccccuuggu                                                   20


<210>  133
<211>  15
<212>  RNA
<213>  Artificial Sequence

<220>
<223>  Synthetic oligonucleotide

<400>  133
cauucucccc uuggu                                                        15


<210>  134
<211>  30
<212>  RNA
<213>  Artificial Sequence

<220>
<223>  Synthetic oligonucleotide

<400>  134
aucccauaca uguuuuuucu cuuguuuggu                                          30


<210>  135
<211>  15
<212>  RNA
<213>  Artificial Sequence

<220>
<223>  Synthetic oligonucleotide

<400>  135
uuucucuugu uuggu                                                         15


<210>  136
<211>  10
<212>  RNA
<213>  Artificial Sequence

<220>
<223>  Synthetic oligonucleotide

<400>  136
cuuguuuggu                                                               10


<210>  137
<211>  30
<212>  RNA
<213>  Artificial Sequence

<220>
<223>  Synthetic oligonucleotide

<400>  137
auuccaugca aguuuuuucu cuuguuuggu                                          30


<210>  138
<211>  20
<212>  RNA
<213>  Artificial Sequence

<220>
<223>  Synthetic oligonucleotide

<400>  138
aguuuuucu cuuguuuggu                                                     20


<210>  139
<211>  30
<212>  RNA
<213>  Artificial Sequence

<220>
<223>  Synthetic oligonucleotide

<400>  139
auuccaaaca uguuucuucu cuuguuuggu                                         30


<210>  140
<211>  20
<212>  RNA
<213>  Artificial Sequence

<220>
<223>  Synthetic oligonucleotide

<400>  140
uguuucuucu cuuguuuggu                                                    20

<210>  141
<211>  15
<212>  RNA
<213>  Artificial Sequence

<220>
<223>  Synthetic oligonucleotide

<400>  141
cuucucuugu uuggu                                                        15


<210>  142
<211>  30
<212>  RNA
<213>  Artificial Sequence

<220>
<223>  Synthetic oligonucleotide

<400>  142
auuccaaaca aguuucuucu cuuguuuggu                                        30


<210>  143
<211>  20
<212>  RNA
<213>  Artificial Sequence

<220>
<223>  Synthetic oligonucleotide

<400>  143
aguuucuucu cuuguuuggu                                                   20


<210>  144
<211>  30
<212>  RNA
<213>  Artificial Sequence

<220>
<223>  Synthetic oligonucleotide

<400>  144
auuccauaca uguuucuucu cuuguuuggu                                        30


<210>  145
<211>  30
<212>  RNA
<213>  Artificial Sequence

<220>
<223>  Synthetic oligonucleotide

<400>  145
auuccauaca cguuuuuucu cuugucuggu                                         30


<210>  146
<211>  20
<212>  RNA
<213>  Artificial Sequence

<220>
<223>  Synthetic oligonucleotide

<400>  146
   cguuuuuucu cuugucuggu                                                    20


<210>  147
<211>  15
<212>  RNA

<213>  Artificial Sequence

<220>
<223>  Synthetic oligonucleotide

<400>  147
uuucucuugu cuggu                                                        15


<210>  148
<211>  10
<212>  RNA
<213>  Artificial Sequence

<220>
<223>  Synthetic oligonucleotide

<400>  148
cuugucuggu                                                              10


<210>  149
<211>  30
<212>  RNA
<213>  Artificial Sequence

<220>
<223>  Synthetic oligonucleotide

<400>  149
ggauacauau cucuuaaacu cuugucuggu                                        30


<210>  150
<211>  20
<212>  RNA
<213>  Artificial Sequence

<220>
<223>  Synthetic oligonucleotide

<400>  150
cucuuaaacu cuugucuggu                                                   20


<210>  151
<211>  15
<212>  RNA
<213>  Artificial Sequence

<220>
<223>  Synthetic oligonucleotide

<400>  151
aaacucuugu cuggu                                                        15


<210>  152
<211>  30
<212>  RNA
<213>  Artificial Sequence

<220>
<223>  Synthetic oligonucleotide

<400>  152
uucccagaca aguuucuucu cuuguuuggu                                        30


<210>  153
<211>  30
<212>  RNA
<213>  Artificial Sequence

<220>
<223>  Synthetic oligonucleotide

<400>  153
uuaccaagca aguuucuucu cuuguuuggu                                        30


<210>  154
<211>  30
<212>  RNA
<213>  Artificial Sequence

<220>
<223>  Synthetic oligonucleotide

<400>  154
uucccaagca agucucuucu cuuguuuggu                                        30


<210>  155
<211>  20
<212>  RNA
<213>  Artificial Sequence

<220>
<223>  Synthetic oligonucleotide

<400>  155
agucucuucu cuuguuuggu                                                   20


<210>  156
<211>  30
<212>  RNA
<213>  Artificial Sequence

<220>
<223>  Synthetic oligonucleotide

<400>  156
uuuccaaaca agucucuucu cuuguuuggu                                        30


<210>  157
<211>  30
<212>  RNA
<213>  Artificial Sequence

<220>
<223>  Synthetic oligonucleotide

<400>  157
auuccaaaca aguuuuuccu cuuguuuggu                                        30


<210>  158
<211>  20
<212>  RNA
<213>  Artificial Sequence

<220>
<223>  Synthetic oligonucleotide

<400>  158
aguuuuuccu cuuguuuggu                                                   20


<210>  159
<211>  15
<212>  RNA
<213>  Artificial Sequence

<220>
<223>  Synthetic oligonucleotide

<400>  159
uuccucuugu uuggu                                                        15


<210>  160

<211>  30
<212>  RNA
<213>  Artificial Sequence

<220>
<223>  Synthetic oligonucleotide

<400>  160
cgcaaguuug uuguacgcau uuuuucgcgu                                        30


<210>  161
<211>  15
<212>  RNA
<213>  Artificial Sequence

<220>
<223>  Synthetic oligonucleotide

<400>  161
cgcauuuuuu cgcgu                                                        15


<210>  162
<211>  10
<212>  RNA
<213>  Artificial Sequence

<220>
<223>  Synthetic oligonucleotide

<400>  162
uuuuucgcgu                                                              10


<210>  163
<211>  30
<212>  RNA
<213>  Artificial Sequence

<220>
<223>  Synthetic oligonucleotide

<400>  163
ugcaaguuug uaguacgcau uuuuucgcgu                                        30


<210>  164
<211>  20
<212>  RNA
<213>  Artificial Sequence

<220>
<223>  Synthetic oligonucleotide

<400>  164
uaguacgcau uuuuucgcgu                                                   20


<210>  165
<211>  30
<212>  RNA
<213>  Artificial Sequence

<220>
<223>  Synthetic oligonucleotide

<400>  165
ugcaaguuug uuguacgcau uuuuucccgu                                        30


<210>  166
<211>  20
<212>  RNA
<213>  Artificial Sequence

<220>

<223>  Synthetic oligonucleotide

<400>  166
uuguacgcau uuuuucccgu                                                    20


<210>  167
<211>  15
<212>  RNA
<213>  Artificial Sequence

<220>
<223>  Synthetic oligonucleotide

<400>  167
cgcauuuuuu cccgu                                                         15


<210>  168
<211>  10
<212>  RNA
<213>  Artificial Sequence

<220>
<223>  Synthetic oligonucleotide

<400>  168
uuuuucccgu                                                               10


<210>  169
<211>  30
<212>  RNA
<213>  Artificial Sequence

<220>
<223>  Synthetic oligonucleotide

<400>  169
uaacuuaauu uauacgcguu uuuuucgcgu                                         30


<210>  170
<211>  20
<212>  RNA
<213>  Artificial Sequence

<220>
<223>  Synthetic oligonucleotide

<400>  170
uauacgcguu uuuuucgcgu                                                    20


<210>  171
<211>  15
<212>  RNA
<213>  Artificial Sequence

<220>
<223>  Synthetic oligonucleotide

<400>  171
gcguuuuuuu cgcgu                                                         15


<210>  172
<211>  30
<212>  RNA
<213>  Artificial Sequence

<220>
<223>  Synthetic oligonucleotide

<400>  172
cauauugaau auaauugcgc ugcuuucgcu                                         30

<210>  173
<211>  20
<212>  RNA
<213>  Artificial Sequence

<220>
<223>  Synthetic oligonucleotide

<400>  173
auaauugcgc ugcuuucgcu                                                    20


<210>  174
<211>  15
<212>  RNA
<213>  Artificial Sequence

<220>
<223>  Synthetic oligonucleotide

<400>  174
ugcgcugcuu ucgcu                                                         15


<210>  175
<211>  10
<212>  RNA
<213>  Artificial Sequence

<220>
<223>  Synthetic oligonucleotide

<400>  175
ugcuuucgcu                                                               10


<210>  176
<211>  30
<212>  RNA
<213>  Artificial Sequence

<220>
<223>  Synthetic oligonucleotide

<400>  176
cauauugaau auaauugacc ugcuuucgcu                                         30


<210>  177
<211>  15
<212>  RNA
<213>  Artificial Sequence

<220>
<223>  Synthetic oligonucleotide

<400>  177
ugaccugcuu ucgcu                                                         15


<210>  178
<211>  30
<212>  RNA
<213>  Artificial Sequence

<220>
<223>  Synthetic oligonucleotide

<400>  178
cauauucaau auaauugacc ugcuuuucgu                                         30


<210>  179
<211>  20
<212>  RNA
<213>  Artificial Sequence

<220>
<223>  Synthetic oligonucleotide

<400>  179
auaauugacc ugcuuuucgu                                                    20


<210>  180
<211>  15
<212>  RNA
<213>  Artificial Sequence

<220>
<223>  Synthetic oligonucleotide

<400>  180
ugaccugcuu uucgu                                                         15


<210>  181
<211>  10
<212>  RNA
<213>  Artificial Sequence

<220>
<223>  Synthetic oligonucleotide

<400>  181
ugcuuuucgu                                                               10


<210>  182
<211>  30
<212>  RNA
<213>  Artificial Sequence

<220>
<223>  Synthetic oligonucleotide

<400>  182
auccauucaa augguuugcc ugcuuucgcu                                         30


<210>  183
<211>  20
<212>  RNA
<213>  Artificial Sequence

<220>
<223>  Synthetic oligonucleotide

<400>  183
augguuugcc ugcuuucgcu                                                    20


<210>  184
<211>  15
<212>  RNA
<213>  Artificial Sequence

<220>
<223>  Synthetic oligonucleotide

<400>  184
uugccugcuu ucgcu                                                         15


<210>  185
<211>  30
<212>  RNA
<213>  Artificial Sequence

<220>
<223>  Synthetic oligonucleotide

<400>  185

auccauucaa augguuugcc ugcuuuugcu 30

<210> 186
<211> 20
<212> RNA
<213> Artificial Sequence

<220>
<223> Synthetic oligonucleotide

<400> 186
augguuugcc ugcuuuugcu 20

<210> 187
<211> 15
<212> RNA
<213> Artificial Sequence

<220>
<223> Synthetic oligonucleotide

<400> 187
uugccugcuu uugcu 15

<210> 188
<211> 10
<212> RNA
<213> Artificial Sequence

<220>
<223> Synthetic oligonucleotide

<400> 188
ugcuuuugcu 10

<210> 189
<211> 30
<212> RNA
<213> Artificial Sequence

<220>
<223> Synthetic oligonucleotide

<400> 189
auccauucaa gugguuugcc ugcuuuugcu 30

<210> 190
<211> 20
<212> RNA
<213> Artificial Sequence

<220>
<223> Synthetic oligonucleotide

<400> 190
gugguuugcc ugcuuuugcu 20

<210> 191
<211> 30
<212> RNA
<213> Artificial Sequence

<220>
<223> Synthetic oligonucleotide

<400> 191
auccauucaa augguuucgc ugcuuucgcu 30

<210> 192
<211> 20

<212>  RNA
<213>  Artificial Sequence

<220>
<223>  Synthetic oligonucleotide

<400>  192
augguuucgc ugcuuucgcu                                                   20


<210>  193
<211>  15
<212>  RNA
<213>  Artificial Sequence

<220>
<223>  Synthetic oligonucleotide

<400>  193
uucgcugcuu ucgcu                                                        15


<210>  194
<211>  30
<212>  RNA
<213>  Artificial Sequence

<220>
<223>  Synthetic oligonucleotide

<400>  194
uuccauuuug gaucaguacc ugcuuucgcu                                        30


<210>  195
<211>  20
<212>  RNA
<213>  Artificial Sequence

<220>
<223>  Synthetic oligonucleotide

<400>  195
gaucaguacc ugcuuucgcu                                                   20


<210>  196
<211>  15
<212>  RNA
<213>  Artificial Sequence

<220>
<223>  Synthetic oligonucleotide

<400>  196
guaccugcuu ucgcu                                                        15


<210>  197
<211>  30
<212>  RNA
<213>  Artificial Sequence

<220>
<223>  Synthetic oligonucleotide

<400>  197
uuccauuuug gaucaguacc ugcuuuugcu                                        30


<210>  198
<211>  20
<212>  RNA
<213>  Artificial Sequence

<220>
<223>  Synthetic oligonucleotide

<400>  198
gaucaguacc ugcuuuugcu                                                   20


<210>  199
<211>  15
<212>  RNA
<213>  Artificial Sequence

<220>
<223>  Synthetic oligonucleotide

<400>  199
guaccugcuu uugcu                                                        15


<210>  200
<211>  30
<212>  RNA
<213>  Artificial Sequence

<220>
<223>  Synthetic oligonucleotide

<400>  200
uuccauuuug aaucaguacc ugcuuucgcu                                        30


<210>  201
<211>  20
<212>  RNA
<213>  Artificial Sequence

<220>
<223>  Synthetic oligonucleotide

<400>  201
aaucaguacc ugcuuucgcu                                                   20


<210>  202
<211>  30
<212>  RNA
<213>  Artificial Sequence

<220>
<223>  Synthetic oligonucleotide

<400>  202
uuccauuucg aaucaguacc ugcuuucgcu                                        30


<210>  203
<211>  30
<212>  RNA
<213>  Artificial Sequence

<220>
<223>  Synthetic oligonucleotide

<400>  203
uuccauuucg gaucaguacc ugcuuuugcu                                        30


<210>  204
<211>  30
<212>  RNA
<213>  Artificial Sequence

<220>
<223>  Synthetic oligonucleotide

<400>  204
uuccauucug aaucaguacc ugcuuuugcu                                        30

<210>  205
<211>  20
<212>  RNA
<213>  Artificial Sequence

<220>
<223>  Synthetic oligonucleotide

<400>  205
aaucaguacc ugcuuuugcu                                                    20


<210>  206
<211>  30
<212>  RNA
<213>  Artificial Sequence

<220>
<223>  Synthetic oligonucleotide

<400>  206
ugguuguuuu uauuuucccc ugcuuuugcu                                         30


<210>  207
<211>  20
<212>  RNA
<213>  Artificial Sequence

<220>
<223>  Synthetic oligonucleotide

<400>  207
uauuuucccc ugcuuuugcu                                                    20


<210>  208
<211>  15
<212>  RNA
<213>  Artificial Sequence

<220>
<223>  Synthetic oligonucleotide

<400>  208
uccccugcuu uugcu                                                         15


<210>  209
<211>  30
<212>  RNA
<213>  Artificial Sequence

<220>
<223>  Synthetic oligonucleotide

<400>  209
ugguugcuuu uauuuucccc ugcuuuugcu                                         30


<210>  210
<211>  30
<212>  RNA
<213>  Artificial Sequence

<220>
<223>  Synthetic oligonucleotide

<400>  210
ugguugguuu uauuuucccc ugcuuuugcu                                         30


<210>  211
<211>  30
<212>  RNA
<213>  Artificial Sequence

<220>
<223>  Synthetic oligonucleotide

<400>  211
ugguuguauu uauuuucccc ugcuuuugcu                                        30


<210>  212
<211>  30
<212>  RNA
<213>  Artificial Sequence

<220>
<223>  Synthetic oligonucleotide

<400>  212
ugguugauuu uauuuucccc ugcuuuugcu                                        30


<210>  213
<211>  30
<212>  RNA
<213>  Artificial Sequence

<220>
<223>  Synthetic oligonucleotide

<400>  213
ugguugauuu aauuuucccc ugcuuuugcu                                        30


<210>  214
<211>  20
<212>  RNA
<213>  Artificial Sequence

<220>
<223>  Synthetic oligonucleotide

<400>  214
aauuuucccc ugcuuuugcu                                                   20


<210>  215
<211>  30
<212>  RNA
<213>  Artificial Sequence

<220>
<223>  Synthetic oligonucleotide

<400>  215
ugguugauuu gauuuccccc ugcuuuugcu                                        30


<210>  216
<211>  20
<212>  RNA
<213>  Artificial Sequence

<220>
<223>  Synthetic oligonucleotide

<400>  216
gauuuccccc ugcuuuugcu                                                   20


<210>  217
<211>  30
<212>  RNA
<213>  Artificial Sequence

<220>
<223>  Synthetic oligonucleotide

<400>  217
gguugcuuuu auuuuccccu gcuuuugcua                                        30

<210>  218
<211>  20
<212>  RNA
<213>  Artificial Sequence

<220>
<223>  Synthetic oligonucleotide

<400>  218
   auuuuccccu gcuuuugcua                                                    20


<210>  219
<211>  15
<212>  RNA
<213>  Artificial Sequence

<220>
<223>  Synthetic oligonucleotide

<400>  219
ccccugcuuu ugcua                                                        15


<210>  220
<211>  10
<212>  RNA
<213>  Artificial Sequence

<220>
<223>  Synthetic oligonucleotide

<400>  220
gcuuuugcua                                                              10


<210>  221
<211>  30
<212>  RNA
<213>  Artificial Sequence

<220>
<223>  Synthetic oligonucleotide

<400>  221
guugcuuuua uuuuccccug cuuuugcuaa                                         30


<210>  222
<211>  20
<212>  RNA
<213>  Artificial Sequence

<220>
<223>  Synthetic oligonucleotide

<400>  222
   uuuuccccug cuuuugcuaa                                                    20


<210>  223
<211>  15
<212>  RNA
<213>  Artificial Sequence

<220>
<223>  Synthetic oligonucleotide

<400>  223
cccugcuuuu gcuaa                                                        15


<210>  224
<211>  10
<212>  RNA

<213>  Artificial Sequence

<220>
<223>  Synthetic oligonucleotide

<400>  224
cuuuugcuaa                                                              10


<210>  225
<211>  30
<212>  RNA
<213>  Artificial Sequence

<220>
<223>  Synthetic oligonucleotide

<400>  225
cauugaguga uuaucuaccc ugcuuuugcu                                        30


<210>  226
<211>  20
<212>  RNA
<213>  Artificial Sequence

<220>
<223>  Synthetic oligonucleotide

<400>  226
uuaucuaccc ugcuuuugcu                                                   20


<210>  227
<211>  15
<212>  RNA
<213>  Artificial Sequence

<220>
<223>  Synthetic oligonucleotide

<400>  227
uacccugcuu uugcu                                                        15


<210>  228
<211>  30
<212>  RNA
<213>  Artificial Sequence

<220>
<223>  Synthetic oligonucleotide

<400>  228
cggugaguga uuaucuaccc ugcuuuugcu                                        30


<210>  229
<211>  30
<212>  RNA
<213>  Artificial Sequence

<220>
<223>  Synthetic oligonucleotide

<400>  229
cggugagaga uuaucuaccc ugcuuuugcu                                        30


<210>  230
<211>  30
<212>  RNA
<213>  Artificial Sequence

<220>
<223>  Synthetic oligonucleotide

<400>  230
cagugaguga uuaucaaccc ugcuuuugcu                                        30


<210>  231
<211>  20
<212>  RNA
<213>  Artificial Sequence

<220>
<223>  Synthetic oligonucleotide

<400>  231
uuaucaaccc ugcuuuugcu                                                   20


<210>  232
<211>  15
<212>  RNA
<213>  Artificial Sequence

<220>
<223>  Synthetic oligonucleotide

<400>  232
aacccugcuu uugcu                                                        15


<210>  233
<211>  30
<212>  RNA
<213>  Artificial Sequence

<220>
<223>  Synthetic oligonucleotide

<400>  233
cagugaguga uuauuaaccc ugcuuuugcu                                        30


<210>  234
<211>  20
<212>  RNA
<213>  Artificial Sequence

<220>
<223>  Synthetic oligonucleotide

<400>  234
uuauuaaccc ugcuuuugcu                                                   20


<210>  235
<211>  30
<212>  RNA
<213>  Artificial Sequence

<220>
<223>  Synthetic oligonucleotide

<400>  235
uuguauucau uuuaaacccc ugcuuuugcu                                        30


<210>  236
<211>  20
<212>  RNA
<213>  Artificial Sequence

<220>
<223>  Synthetic oligonucleotide

<400>  236
uuuaaacccc ugcuuuugcu                                                   20


<210>  237

<211>  15
<212>  RNA
<213>  Artificial Sequence

<220>
<223>  Synthetic oligonucleotide

<400>  237
accccugcuu uugcu                                                        15


<210>  238
<211>  30
<212>  RNA
<213>  Artificial Sequence

<220>
<223>  Synthetic oligonucleotide

<400>  238
uuguauucau uuuaaacucc ugcuuuugcu                                        30


<210>  239
<211>  20
<212>  RNA
<213>  Artificial Sequence

<220>
<223>  Synthetic oligonucleotide

<400>  239
uuuaaacucc ugcuuuugcu                                                   20


<210>  240
<211>  15
<212>  RNA
<213>  Artificial Sequence

<220>
<223>  Synthetic oligonucleotide

<400>  240
acuccugcuu uugcu                                                        15


<210>  241
<211>  30
<212>  RNA
<213>  Artificial Sequence

<220>
<223>  Synthetic oligonucleotide

<400>  241
ggauucauuu ugaacuccug cuuuugcuaa                                        30


<210>  242
<211>  20
<212>  RNA
<213>  Artificial Sequence

<220>
<223>  Synthetic oligonucleotide

<400>  242
ugaacuccug cuuuugcuaa                                                   20


<210>  243
<211>  15
<212>  RNA
<213>  Artificial Sequence

<220>

<223>  Synthetic oligonucleotide

<400>  243
uccugcuuuu gcuaa                                                        15


<210>  244
<211>  30
<212>  RNA
<213>  Artificial Sequence

<220>
<223>  Synthetic oligonucleotide

<400>  244
uuggauucau uuuaaucucc ugcuuuugcu                                        30


<210>  245
<211>  20
<212>  RNA
<213>  Artificial Sequence

<220>
<223>  Synthetic oligonucleotide

<400>  245
uuuaaucucc ugcuuuugcu                                                   20


<210>  246
<211>  15
<212>  RNA
<213>  Artificial Sequence

<220>
<223>  Synthetic oligonucleotide

<400>  246
ucuccugcuu uugcu                                                        15


<210>  247
<211>  30
<212>  RNA
<213>  Artificial Sequence

<220>
<223>  Synthetic oligonucleotide

<400>  247
auuuggauuc auuuuaaucu ccugcuuuug                                        30


<210>  248
<211>  20
<212>  RNA
<213>  Artificial Sequence

<220>
<223>  Synthetic oligonucleotide

<400>  248
auuuuaaucu ccugcuuuug                                                   20


<210>  249
<211>  15
<212>  RNA
<213>  Artificial Sequence

<220>
<223>  Synthetic oligonucleotide

<400>  249
aaucuccugc uuuug                                                        15

<210>  250
<211>  10
<212>  RNA
<213>  Artificial Sequence

<220>
<223>  Synthetic oligonucleotide

<400>  250
ccugcuuuug                                                              10


<210>  251
<211>  30
<212>  RNA
<213>  Artificial Sequence

<220>
<223>  Synthetic oligonucleotide

<400>  251
cucaucuuuc aauaucuacc ugcuuuugcu                                        30


<210>  252
<211>  20
<212>  RNA
<213>  Artificial Sequence

<220>
<223>  Synthetic oligonucleotide

<400>  252
aauaucuacc ugcuuuugcu                                                   20


<210>  253
<211>  15
<212>  RNA
<213>  Artificial Sequence

<220>
<223>  Synthetic oligonucleotide

<400>  253
cuaccugcuu uugcu                                                        15


<210>  254
<211>  30
<212>  RNA
<213>  Artificial Sequence

<220>
<223>  Synthetic oligonucleotide

<400>  254
cucaucuuuc aacaucuacc ugcuuuugcu                                        30


<210>  255
<211>  20
<212>  RNA
<213>  Artificial Sequence

<220>
<223>  Synthetic oligonucleotide

<400>  255
aacaucuacc ugcuuuugcu                                                   20


<210>  256
<211>  30
<212>  RNA
<213>  Artificial Sequence

<220>
<223>  Synthetic oligonucleotide

<400>  256
cucaucuuuc aauaucuacc ugcuuucgcu                                        30


<210>  257
<211>  20
<212>  RNA
<213>  Artificial Sequence

<220>
<223>  Synthetic oligonucleotide

<400>  257
aauaucuacc ugcuuucgcu                                                   20


<210>  258
<211>  15
<212>  RNA
<213>  Artificial Sequence

<220>
<223>  Synthetic oligonucleotide

<400>  258
cuaccugcuu ucgcu                                                        15


<210>  259
<211>  30
<212>  RNA
<213>  Artificial Sequence

<220>
<223>  Synthetic oligonucleotide

<400>  259
ccauuauguc uuugucaccc ugcuuuugcu                                        30


<210>  260
<211>  15
<212>  RNA
<213>  Artificial Sequence

<220>
<223>  Synthetic oligonucleotide

<400>  260
cacccugcuu uugcu                                                        15


<210>  261
<211>  30
<212>  RNA
<213>  Artificial Sequence

<220>
<223>  Synthetic oligonucleotide

<400>  261
uauauucauc uuaaaggcuc cgcuucugcu                                        30


<210>  262
<211>  20
<212>  RNA
<213>  Artificial Sequence

<220>
<223>  Synthetic oligonucleotide

<400>  262

uuaaaggcuc cgcuucugcu                                                    20

<210>  263
<211>  15
<212>  RNA
<213>  Artificial Sequence

<220>
<223>  Synthetic oligonucleotide

<400>  263
ggcuccgcuu cugcu                                                         15

<210>  264
<211>  10
<212>  RNA
<213>  Artificial Sequence

<220>
<223>  Synthetic oligonucleotide

<400>  264
cgcuucugcu                                                               10

<210>  265
<211>  30
<212>  RNA
<213>  Artificial Sequence

<220>
<223>  Synthetic oligonucleotide

<400>  265
uuauauucau cuuaaagcuc cgcuucugcu                                         30

<210>  266
<211>  20
<212>  RNA
<213>  Artificial Sequence

<220>
<223>  Synthetic oligonucleotide

<400>  266
cuuaaagcuc cgcuucugcu                                                    20

<210>  267
<211>  15
<212>  RNA
<213>  Artificial Sequence

<220>
<223>  Synthetic oligonucleotide

<400>  267
agcuccgcuu cugcu                                                         15

<210>  268
<211>  30
<212>  RNA
<213>  Artificial Sequence

<220>
<223>  Synthetic oligonucleotide

<400>  268
augucaucuu gaaaacgcuc cgcuucugcu                                         30

<210>  269
<211>  20

<212>  RNA
<213>  Artificial Sequence

<220>
<223>  Synthetic oligonucleotide

<400>  269
gaaaacgcuc cgcuucugcu                                                   20


<210>  270
<211>  15
<212>  RNA
<213>  Artificial Sequence

<220>
<223>  Synthetic oligonucleotide

<400>  270
cgcuccgcuu cugcu                                                        15


<210>  271
<211>  30
<212>  RNA
<213>  Artificial Sequence

<220>
<223>  Synthetic oligonucleotide

<400>  271
uuauggcaaa ucaaacgcac cgcuucugcu                                        30


<210>  272
<211>  20
<212>  RNA
<213>  Artificial Sequence

<220>
<223>  Synthetic oligonucleotide

<400>  272
ucaaacgcac cgcuucugcu                                                   20


<210>  273
<211>  15
<212>  RNA
<213>  Artificial Sequence

<220>
<223>  Synthetic oligonucleotide

<400>  273
cgcaccgcuu cugcu                                                        15


<210>  274
<211>  30
<212>  RNA
<213>  Artificial Sequence

<220>
<223>  Synthetic oligonucleotide

<400>  274
uuauggcaaa ucaaacguau cgcuucugcu                                        30


<210>  275
<211>  20
<212>  RNA
<213>  Artificial Sequence

<220>
<223>  Synthetic oligonucleotide

<400>  275
ucaaacguau cgcuucugcu                                                    20


<210>  276
<211>  15
<212>  RNA
<213>  Artificial Sequence

<220>
<223>  Synthetic oligonucleotide

<400>  276
cguaucgcuu cugcu                                                         15


<210>  277
<211>  30
<212>  RNA
<213>  Artificial Sequence

<220>
<223>  Synthetic oligonucleotide

<400>  277
guggauauua gaaaaugcuc ugcuucugcu                                         30


<210>  278
<211>  20
<212>  RNA
<213>  Artificial Sequence

<220>
<223>  Synthetic oligonucleotide

<400>  278
gaaaaugcuc ugcuucugcu                                                    20


<210>  279
<211>  15
<212>  RNA
<213>  Artificial Sequence

<220>
<223>  Synthetic oligonucleotide

<400>  279
ugcucugcuu cugcu                                                         15


<210>  280
<211>  10
<212>  RNA
<213>  Artificial Sequence

<220>
<223>  Synthetic oligonucleotide

<400>  280
ugcuucugcu                                                               10


<210>  281
<211>  30
<212>  RNA
<213>  Artificial Sequence

<220>
<223>  Synthetic oligonucleotide

<400>  281
uauccaucuu gaaaauagcc aaucuuagcu                                         30

<210>  282
<211>  20
<212>  RNA
<213>  Artificial Sequence

<220>
<223>  Synthetic oligonucleotide

<400>  282
gaaaauagcc aaucuuagcu                                                    20


<210>  283
<211>  15
<212>  RNA
<213>  Artificial Sequence

<220>
<223>  Synthetic oligonucleotide

<400>  283
uagccaaucu uagcu                                                         15


<210>  284
<211>  10
<212>  RNA
<213>  Artificial Sequence

<220>
<223>  Synthetic oligonucleotide

<400>  284
aaucuuagcu                                                               10


<210>  285
<211>  24
<212>  DNA
<213>  Artificial Sequence

<220>
<223>  Synthetic oligonucleotide

<400>  285
tcgtcgtttt gtcgttttgt cgtt                                               24


<210>  286
<211>  24
<212>  RNA
<213>  Artificial Sequence

<220>
<223>  Synthetic oligonucleotide

<400>  286
uuguuguuuu guuguuuugu uguu                                               24


<210>  287
<211>  24
<212>  RNA
<213>  Artificial Sequence

<220>
<223>  Synthetic oligonucleotide


<220>
<221>  misc_feature
<222>  (1)..(1)
<223>  where n is thymidine

<220>
<221>  misc_feature
<222>  (4)..(4)

<223>  where n is thymidine

<220>
<221>  misc_feature
<222>  (7)..(10)
<223>  where n is thymidine

<220>
<221>  misc_feature
<222>  (12)..(12)
<223>  where n is thymidine

<220>
<221>  misc_feature
<222>  (15)..(18)
<223>  where n is thymidine

<220>
<221>  misc_feature
<222>  (20)..(20)
<223>  where n is thymidine

<220>
<221>  misc_feature
<222>  (23)..(24)
<223>  where n is thymidine

<400>  287
nugnugnnnn gnugnnnngn ugnn                                              24


<210>  288
<211>  22
<212>  DNA
<213>  Artificial Sequence

<220>
<223>  Synthetic oligonucleotide


<220>
<221>  misc_feature
<222>  (2)..(2)
<223>  n is a, c, g, or t

<400>  288
tncgtcgttt cggcggccgc cg                                                22


<210>  289
<211>  22
<212>  RNA
<213>  Artificial Sequence

<220>
<223>  Synthetic oligonucleotide

<400>  289
uuguuguuuu uggugguugu ug                                                22


<210>  290
<211>  22
<212>  RNA
<213>  Artificial Sequence

<220>
<223>  Synthetic oligonucleotide


<220>
<221>  misc_feature
<222>  (1)..(1)
<223>  where n is thymidine

<220>
<221>  misc_feature

<222>  (4)..(4)
<223>  where n is thymidine

<220>
<221>  misc_feature
<222>  (7)..(10)
<223>  where n is thymidine

<400>  290
nugnugnnnn uggugguugu ug                                                22


<210>  291
<211>  24
<212>  DNA
<213>  Artificial Sequence

<220>
<223>  synthetic DNA/RNA hybrid oligonucleotide


<220>
<221>  misc_feature
<222>  (12)..(13)
<223>  where n is uracil

<220>
<221>  misc_feature
<222>  (15)..(18)
<223>  where n is uracil

<220>
<221>  misc_feature
<222>  (20)..(21)
<223>  where n is uracil

<220>
<221>  misc_feature
<222>  (23)..(24)
<223>  where n is uracil

<400>  291
tcgtcgtttt gnngnnnngn ngnn                                              24


<210>  292
<211>  10
<212>  DNA
<213>  Artificial Sequence

<220>
<223>  Synthetic oligonucleotide

<400>  292
tcgtcgtttt                                                              10


<210>  293
<211>  14
<212>  RNA
<213>  Artificial Sequence

<220>
<223>  Synthetic oligonucleotide

<400>  293
guuguuuugu uguu                                                         14


<210>  294
<211>  22
<212>  DNA
<213>  Artificial Sequence

<220>
<223>  synthetic DNA/RNA hybrid oligonucleotide

<220>
<221>  misc_feature
<222>  (11)..(11)
<223>  where n is uracil

<220>
<221>  misc_feature
<222>  (14)..(14)
<223>  where n is uracil

<220>
<221>  misc_feature
<222>  (17)..(18)
<223>  where n is uracil

<220>
<221>  misc_feature
<222>  (20)..(21)
<223>  where n is uracil

<400>  294
tcgtcgtttt nggnggnngn ng                                                22


<210>  295
<211>  12
<212>  RNA
<213>  Artificial Sequence

<220>
<223>  Synthetic oligonucleotide

<400>  295
uggugguugu ug                                                           12


<210>  296
<211>  19
<212>  DNA
<213>  Artificial Sequence

<220>
<223>  synthetic DNA/RNA hybrid oligonucleotide


<220>
<221>  misc_feature
<222>  (11)..(12)
<223>  where n is uracil

<220>
<221>  misc_feature
<222>  (14)..(14)
<223>  where n is uracil

<220>
<221>  misc_feature
<222>  (16)..(16)
<223>  where n is uracil

<400>  296
tcgtcgtttg nngngnaat                                                    19


<210>  297
<211>  26
<212>  RNA
<213>  Artificial Sequence

<220>
<223>  Synthetic oligonucleotide

<400>  297
uugaauauaa uugaccugcu uucgcu                                            26

<210>  298
<211>  26
<212>  RNA
<213>  Artificial Sequence

<220>
<223>  Synthetic oligonucleotide

<400>  298
auucaaaugg uuugccugcu uucgcu                                            26


<210>  299
<211>  26
<212>  RNA
<213>  Artificial Sequence

<220>
<223>  Synthetic oligonucleotide

<400>  299
auuuuggauc aguaccugcu uucgcu                                            26


<210>  300
<211>  26
<212>  RNA
<213>  Artificial Sequence

<220>
<223>  Synthetic oligonucleotide

<400>  300
uguuuuuauu uuccccugcu uuugcu                                            26


<210>  301
<211>  26
<212>  RNA
<213>  Artificial Sequence

<220>
<223>  Synthetic oligonucleotide

<400>  301
gagugauuau cuacccugcu uuugcu                                            26


<210>  302
<211>  26
<212>  RNA
<213>  Artificial Sequence

<220>
<223>  Synthetic oligonucleotide

<400>  302
auucauuuua aaccccugcu uucgcu                                            26


<210>  303
<211>  26
<212>  RNA
<213>  Artificial Sequence

<220>
<223>  Synthetic oligonucleotide

<400>  303
ucuuucaaua ucuaccugcu uucgcu                                            26


<210>  304
<211>  26
<212>  RNA
<213>  Artificial Sequence

<220>
<223>  Synthetic oligonucleotide

<400>  304
uaugucuuug ucacccugcu uuugcu                                          26


<210>  305
<211>  26
<212>  RNA
<213>  Artificial Sequence

<220>
<223>  Synthetic oligonucleotide

<400>  305
uucauuuugu auccccugcu uuugcu                                          26


<210>  306
<211>  26
<212>  RNA
<213>  Artificial Sequence

<220>
<223>  Synthetic oligonucleotide

<400>  306
uucaucuuaa aggcuccgcu ucugcu                                          26


<210>  307
<211>  26
<212>  RNA
<213>  Artificial Sequence

<220>
<223>  Synthetic oligonucleotide

<400>  307
caucuugaaa acgcuccgcu ucugcu                                          26


<210>  308
<211>  26
<212>  RNA
<213>  Artificial Sequence

<220>
<223>  Synthetic oligonucleotide

<400>  308
ggcaaaucaa acgcaccgcu ucugcu                                          26


<210>  309
<211>  26
<212>  RNA
<213>  Artificial Sequence

<220>
<223>  Synthetic oligonucleotide

<400>  309
auauuagaaa augcaacgcu ucugcu                                          26


<210>  310
<211>  26
<212>  RNA
<213>  Artificial Sequence

<220>
<223>  Synthetic oligonucleotide

<400>  310
ugagaagaaa augcugugcu ucugcu                                          26

<210>  311
<211>  26
<212>  RNA
<213>  Artificial Sequence

<220>
<223>  Synthetic oligonucleotide

<400>  311
uucuaagaau augcucugcu ucugcu                                            26


<210>  312
<211>  26
<212>  RNA
<213>  Artificial Sequence

<220>
<223>  Synthetic oligonucleotide

<400>  312
auuuuaagaa agugcgugcu ucugcu                                            26


<210>  313
<211>  26
<212>  RNA
<213>  Artificial Sequence

<220>
<223>  Synthetic oligonucleotide

<400>  313
gacuaaacaa augcucugcu ucugcu                                            26


<210>  314
<211>  26
<212>  RNA
<213>  Artificial Sequence

<220>
<223>  Synthetic oligonucleotide

<400>  314
gacauuucca auccccugcu ucugcu                                            26


<210>  315
<211>  26
<212>  RNA
<213>  Artificial Sequence

<220>
<223>  Synthetic oligonucleotide

<400>  315
gauuuccaua auccccugcu ucugcu                                            26


<210>  316
<211>  26
<212>  RNA
<213>  Artificial Sequence

<220>
<223>  Synthetic oligonucleotide

<400>  316
gacauuucgg auccccugcu ucugcu                                            26


<210>  317
<211>  26
<212>  RNA

<213>  Artificial Sequence

<220>
<223>  Synthetic oligonucleotide

<400>  317
aacauuauua acccccugcu ucugcu                                          26


<210>  318
<211>  26
<212>  RNA
<213>  Artificial Sequence

<220>
<223>  Synthetic oligonucleotide

<400>  318
uuguuuagaa auccccugcu ucugcu                                          26


<210>  319
<211>  26
<212>  RNA
<213>  Artificial Sequence

<220>
<223>  Synthetic oligonucleotide

<400>  319
uuuggaaaag uaccccugcu ucugcu                                          26


<210>  320
<211>  26
<212>  RNA
<213>  Artificial Sequence

<220>
<223>  Synthetic oligonucleotide

<400>  320
uucucugucc aucgcuuguu uuugcu                                          26


<210>  321
<211>  26
<212>  RNA
<213>  Artificial Sequence

<220>
<223>  Synthetic oligonucleotide

<400>  321
uuguguuugg agcgccuguu uuugcu                                          26


<210>  322
<211>  26
<212>  RNA
<213>  Artificial Sequence

<220>
<223>  Synthetic oligonucleotide

<400>  322
ugucauguca agugcuuguu uuugcu                                          26


<210>  323
<211>  26
<212>  RNA
<213>  Artificial Sequence

<220>
<223>  Synthetic oligonucleotide

<400>  323
ugaaggaaca ucugcuuguu uuugcu                                          26


<210>  324
<211>  26
<212>  RNA
<213>  Artificial Sequence

<220>
<223>  Synthetic oligonucleotide

<400>  324
agccauuuug acugccuguu uuugcu                                          26


<210>  325
<211>  24
<212>  RNA
<213>  Artificial Sequence

<220>
<223>  Synthetic oligonucleotide

<400>  325
aucaucucuu guuuuugugu gucu                                            24


<210>  326
<211>  25
<212>  RNA
<213>  Artificial Sequence

<220>
<223>  Synthetic oligonucleotide

<400>  326
   auucuucuuu cuuuuugugu guccg                                            25


<210>  327
<211>  20
<212>  RNA
<213>  Artificial Sequence

<220>
<223>  Synthetic oligonucleotide

<400>  327
agcgaaagca ggucaauuau                                                 20


<210>  328
<211>  18
<212>  DNA
<213>  Artificial Sequence

<220>
<223>  synthetic DNA/RNA hybrid oligonucleotide


<220>
<221>  misc_feature
<222>  (1)..(5)
<223>  each n independently can be any deoxyribonucleotide

<220>
<221>  misc_feature
<222>  (6)..(6)
<223>  n is ribonucleotide

<220>
<221>  misc_feature
<222>  (7)..(10)
<223>  where n is uracil

<220>

<221>  misc_feature
<222>  (11)..(12)
<223>  each n independently can be any ribonucleotide

<220>
<221>  misc_feature
<222>  (13)..(18)
<223>  each n independently can be any deoxyribonucleotide

<400>  328
nnnnnnnnnn nnnnnnnn                                                    18


<210>  329
<211>  18
<212>  DNA
<213>  Artificial Sequence

<220>
<223>  synthetic DNA/RNA hybrid oligonucleotide


<220>
<221>  misc_feature
<222>  (1)..(5)
<223>  each n independently can be any deoxyribonucleotide

<220>
<221>  misc_feature
<222>  (6)..(6)
<223>  n is any ribonucleotide

<220>
<221>  misc_feature
<222>  (7)..(8)
<223>  where n is uracil

<220>
<221>  misc_feature
<222>  (10)..(10)
<223>  where n is uracil

<220>
<221>  misc_feature
<222>  (11)..(12)
<223>  each n independently can be any ribonucleotide

<220>
<221>  misc_feature
<222>  (13)..(18)
<223>  each n independently can be any deoxyribonucleotide

<400>  329
nnnnnnnngn nnnnnnnn                                                    18


<210>  330
<211>  18
<212>  RNA
<213>  Artificial Sequence

<220>
<223>  Synthetic oligonucleotide


<220>
<221>  misc_feature
<222>  (1)..(6)
<223>  each n independently can be any ribonucleotide

<220>
<221>  misc_feature
<222>  (11)..(18)
<223>  each n independently can be any ribonucleotide

<400>  330
nnnnnnuuuu nnnnnnnn                                                    18

<210>  331
<211>  18
<212>  RNA
<213>  Artificial Sequence

<220>
<223>  Synthetic oligonucleotide


<220>
<221>  misc_feature
<222>  (1)..(6)
<223>  each n independently can be any ribonucleotide

<220>
<221>  misc_feature
<222>  (11)..(18)
<223>  each n independently can be any ribonucleotide

<400>  331
nnnnnnuugu nnnnnnnn                                                    18


<210>  332
<211>  19
<212>  DNA
<213>  Artificial Sequence

<220>
<223>  synthetic DNA/RNA hybrid oligonucleotide


<220>
<221>  misc_feature
<222>  (10)..(16)
<223>  residues are ribonucleotides

<220>
<221>  misc_feature
<222>  (11)..(12)
<223>  n is uracil

<220>
<221>  misc_feature
<222>  (14)..(14)
<223>  n is uracil

<220>
<221>  misc_feature
<222>  (16)..(16)
<223>  n is uracil

<400>  332
tcgtcgtttg nngngnaat                                                   19


<210>  333
<211>  19
<212>  DNA
<213>  Artificial Sequence

<220>
<223>  synthetic DNA/RNA hybrid oligonucleotide


<220>
<221>  misc_feature
<222>  (9)..(17)
<223>  2' to 5' interconnecting linkages

<220>
<221>  misc_feature
<222>  (10)..(16)
<223>  residues are ribonucleotides

<220>
<221>  misc_feature
<222>  (11)..(12)
<223>  n is uracil

<220>
<221>  misc_feature
<222>  (14)..(14)
<223>  n is uracil

<220>
<221>  misc_feature
<222>  (16)..(16)
<223>  n is uracil

<400>  333
tcgtcgtttg nngngnaat                                                    19


<210>  334
<211>  24
<212>  RNA
<213>  Artificial Sequence

<220>
<223>  Synthetic oligonucleotide

<400>  334
ucgucguuuu gucguuuugu cguu                                              24


<210>  335
<211>  21
<212>  RNA
<213>  Artificial Sequence

<220>
<223>  Synthetic oligonucleotide

<400>  335
ggggaugaug uugugggggg g                                                 21


<210>  336
<211>  21
<212>  RNA
<213>  Artificial Sequence

<220>
<223>  Synthetic oligonucleotide


<220>
<221>  misc_feature
<222>  (11)..(11)
<223>  n is thymidine

<220>
<221>  misc_feature
<222>  (14)..(14)
<223>  n is thymidine

<400>  336
ggggaugaug nugngggggg g                                                 21


<210>  337
<211>  20
<212>  RNA
<213>  Artificial Sequence

<220>
<223>  Synthetic oligonucleotide

<400>  337
gccaccgagc cgaaggcacc                                                   20

<210>  338
<211>  20
<212>  RNA
<213>  Artificial Sequence

<220>
<223>  Synthetic oligonucleotide

<400>  338
gccaccgagc cgauuguacc                                                    20


<210>  339
<211>  20
<212>  RNA
<213>  Artificial Sequence

<220>
<223>  Synthetic oligonucleotide

<400>  339
gccaccgagc cgauuuuacc                                                    20


<210>  340
<211>  18
<212>  DNA
<213>  Artificial Sequence

<220>
<223>  synthetic DNA/RNA hybrid oligonucleotide


<220>
<221>  misc_feature
<222>  (1)..(5)
<223>  each n independently can be any deoxyribonucleotide

<220>
<221>  misc_feature
<222>  (7)..(8)
<223>  n is uracil

<220>
<221>  misc_feature
<222>  (10)..(10)
<223>  n is uracil

<220>
<221>  misc_feature
<222>  (12)..(12)
<223>  n is uracil

<220>
<221>  misc_feature
<222>  (13)..(18)
<223>  each n independently can be any deoxyribonucleotide

<400>  340
nnnnngnngn gnnnnnnn                                                      18


<210>  341
<211>  18
<212>  DNA
<213>  Artificial Sequence

<220>
<223>  synthetic DNA/RNA hybrid oligonucleotide


<220>
<221>  misc_feature
<222>  (1)..(6)
<223>  each n independently can be any deoxyribonucleotide

<220>
<221> misc_feature
<222> (7)..(8)
<223> n is uracil

<220>
<221> misc_feature
<222> (10)..(10)
<223> n is uracil

<220>
<221> misc_feature
<222> (12)..(18)
<223> each n independently can be any deoxyribonucleotide

<400> 341
nnnnnnnngn gnnnnnnn 18


<210> 342
<211> 18
<212> DNA
<213> Artificial Sequence

<220>
<223> synthetic DNA/RNA hybrid oligonucleotide


<220>
<221> misc_feature
<222> (1)..(6)
<223> each n independently can be any deoxyribonucleotide

<220>
<221> misc_feature
<222> (7)..(8)
<223> n is uracil

<220>
<221> misc_feature
<222> (10)..(10)
<223> n is uracil

<220>
<221> misc_feature
<222> (11)..(18)
<223> each n independently can be any deoxyribonucleotide

<400> 342
nnnnnnnngn nnnnnnnn 18


<210> 343
<211> 22
<212> DNA
<213> Artificial Sequence

<220>
<223> Synthetic oligonucleotide

<400> 343
tcgtcgtttt cggcgcgcgc cg 22


<210> 344
<211> 18
<212> RNA
<213> Artificial Sequence

<220>
<223> Synthetic oligonucleotide

<400> 344
ccgucuguug ugugacuc 18